# EUROPEAN PATENT SPECIFICATION

(11) **EP 1 140 818 B1**
(45) Date of publication and mention of the grant of the patent: **10.09.2003**
(21) Application number: 99961876.2
(22) Date of filing: 30.11.1999
(51) Int. Cl.: C07C 323/60, C07D 213/40, C07D 209/48, A61K 31/16, A61K 31/44, A61K 31/40

(54) **AMIDOMALONAMIDES AND THEIR USE AS INHIBITORS OF MATRIX METALLOPROTEINASE**
AMIDOMALONIMIDE UND IHRE ANWENDUNG ALS MATRIX METALLOPROTEINASE-INHIBITOREN
AMIDOMALONAMIDES ET LEURS UTILISATIONS COMME INHIBITEURS DES METALLOPROTEINASES MATRICIELLES

(30) Priority: 31.12.1998 US 224459
(43) Date of publication of application: 10.10.2001
(73) Proprietor: Aventis Pharmaceuticals Inc., Bridgewater, NJ 08807-0800 (US)
(72) Inventor: WARSHAWSKY, Alan, Carmel, IN 46032 (US); JANUSZ, Michael, J., Oregonia, OH 45054 (US)
(74) Representative: Then, Johann, Dr.
(86) International application number: US9928338
(87) International publication number: WO00040552

(56) References cited:
- WO-A-95/13289
- WO-A-96/35712

## Description

### BACKGROUND OF THE INVENTION

The matrix metalloproteinases (MMPs) are a family of zinc containing endopeptidases which are capable of cleaving large biomolecules such as the collagens, proteoglycans and gelatins. Expression is upregulated by pro-inflammatory cytokines and/or growth factors. The MMP's are secreted as inactive zymogens which, upon activation, are subject to control by endogenous inhibitors, for example, tissue inhibitor of metalloproteinases (TIMP) and α₂-macroglobulin. Chapman, K.T. et al., J. Med. Chem. 36, 4293-4301 (1993); Beckett, R.P. et al., DDT 1, 16-26 (1996). The characterizing feature of diseases involving the enzymes appears to be a stoichiometric imbalance between active enzymes and endogenous inhibitors, leading to excessive tissue disruption, and often degradation. McCachren, S.S., Arthritis Rheum. 34, 1085-1093 (1991).

The discovery of different families of matrix metalloproteinase, their relationships, and their individual characteristics have been categorized in several reports. Emonard, H. et al., Cell Molec. Biol. 36, 131-153 (1990); Birkedal-Hansen, H., J. Oral Pathol. 17, 445-451 (1988); Matrisian, L.M., Trends Genet. 6, 121-125 (1990); Murphy. G.J.P. et al., FEBS Lett. 289, 4-7 (1991); Matrisian, L.M.. Bioessays 14, 455-463 (1992). Three groups of MMPs have been delineated: the collagenases which have triple helical interstitial collagen as substrate, the gelatinases which are proteinases of denatured collagen and Type IV collagen, and the stromelysins which were originally
characterized as proteoglycanases but have now been identified to have a broader proteolytic spectrum. Examples of specific collagenases include fibroblast collagenase (MMP-1), neutrophil collagenase (MMP-8), and collagenase 3 (MMP-13). Examples of gelatinases include 72 kDa gelatinase (gelatinase A; MMP-2) and 92 kDa gelatinase (gelatinase B; MMP-9). Examples of stromelysins include stromelysin I (MMP-3), stromelysin 2 (MMP-10) and matrilysin (MMP-7). Other MMPs which do not fit neatly into the above groups include metalloelastase (MMP-12), membrane-type MMP (MT-MMP or MMP-14) and stromelysin 3 (MMP-11). Beckett, R.P. et al., supra.

Over-expression and activation of MMPs have been linked with a wide range of diseases such as cancer; rheumatoid arthritis; osteoarthritis; chronic inflammatory disorders, such as emphysema and smoking-induced emphysema; cardiovascular disorders, such as atherosclerosis; corneal ulceration; dental diseases such as gingivitis and periodontal disease; and neurological disorders, such as multiple sclerosis. For example, in adenocarcinoma, invasive proximal gastric cells express the 72 kDa form of collagenase Type IV, whereas the noninvasive cells do not. Schwartz, G.K. et al., Cancer 73, 22-27 (1994). Rat embryo cells transformed by the Ha-*ras* and *v-myc* oncogenes or by Ha-*ras* alone are metastatic in nude mice and release the 92 kDa gelatinase/collagenase (MMP-9). Bernhard, E.J. et al., Proc. Natl. Acad. Sci. 91, 4293-4597 (1994). The plasma concentration of MMP-9 was significantly increased (P < 0.01) in 122 patients with gastrointestinal tract cancer and breast cancer. Zucker, S. et al., Cancer Res. 53, 140-146 (1993). Moreover, intraperitoneal administration of batimastat, a synthetic MMP inhibitor, gave significant inhibition in the growth and metastatic spread and number of lung colonies which were produced by intravenous injection of the B16-BL6 murine melanoma in C57BL/6N mice. Chirivi, R.G.S. et al., Int. J. Cancer 58, 460-464 (1994). Over-expression of TIMP-2, the endogenous tissue inhibitor of MMP-2, markedly reduced melanoma growth in the skin of immunodeficient mice. Montgomery, A.M.P. et al., Cancer Res. 54, 5467-5473 (1994).

Accelerated breakdown of the extracellular matrix of articular cartilage is a key feature in the pathology of both rheumatoid arthritis and osteoarthritis. Current evidence suggests that the inappropriate synthesis of MMPs is the key event. Beeley, N.R.A. et al., Curr. Opin. Ther. Patents, 4(1), 7-16 (1994). The advent of reliable diagnostic tools have allowed a number of research groups to recognize that stromelysin is a key enzyme in both arthritis and joint trauma. Beeley, N.R.A. et al., Id.; Hasty, K.A. et al., Arthr. Rheum. 33, 388-397 (1990). It has also been shown that stromelysin is important for the conversion of procollagenase to active collagenase. Murphy, G. et al., Biochem. J. 248, 265-268 (1987).

Furthermore, a range of MMPs can hydrolyse the membrane-bound precursor of the pro-inflammatory cytokine tumor necrosis factor α (TNF-α). Gearing, A.J.H. et al., Nature 370, 555-557 (1994). This cleavage yields mature soluble TNF-α and the inhibitors of MMPs can block production of TNF-α both *in vitro* and *in vivo*. Gearing, A.J.H. et al., Id.; Mohler, K.M. et al., Nature 370, 218-220 (1994); McGeehan, G.M. et al., Nature 370, 558-561 (1994). This pharmacological action is a probable contributor to the antiarthritic action of this class of compounds seen in animal models. Beckett, R.P. et al., supra.

Stromelysin has been observed to degrade the α₁-proteinase inhibitor which regulates the activity of enzymes such as elastase, excesses of which have been linked to chronic inflammatory disorders such as emphysema and chronic bronchitis. Beeley, N.R.A. et al., supra.; Wahl, R.C. et al., Annual Reports in Medicinal Chemistry 25, 177-184 (1990). In addition, a recent study indicates that MMP-12 is required for the development of smoking-induced emphysema in mice. Science, 277, 2002 (1997). Inhibition of the appropriate MMP may thus potentiate the inhibitory activity of endogenous inhibitors of this type.

High levels of mRNA corresponding to stromelysin have been observed in atherosclerotic plaques removed from heart transplant patients. Henney, A.M., et al., Proc. Natl. Acad. Sci. 88, 8154-8158 (1991). It is submitted that the role of stromelysin in such plaques is to encourage rupture of the connective tissue matrix which encloses the plaque. This rupture is in turn thought to be a key event in the cascade which leads to clot formation of the type seen in coronary thrombosis. MMP inhibition is thus a preventive measure for such thromboses.

Collagenase. stromelysin and gelatinase have been implicated in the destruction of the extracellular matrix of the cornea. This is thought to be an important mechanism of morbidity and visual loss in a number of ulcerative ocular diseases, particularly those following infection or chemical damage. Bums, F.R. et al., Invest. Opthalmol. and Visual Sci. 32, 1569-1575 (1989). The MMPs present in the eye during ulceration are derived either endogenously from infiltrating leucocytes or fibroblasts. or exogenously from microbes.

Collagenase and stromelysin activities have been identified in fibroblasts isolated from inflamed gingiva and the levels of enzyme have been correlated with the severity of the gingivitis observed. Beeley, N.R.A. et al., supra.; Overall, C.M. et al., J. Periodontal Res. 22, 81-88 (1987).

Excessive levels of gelatinase-B in cerebrospinal fluid has been linked with incidence of multiple sclerosis and other neurological disorders. Beeley, N.R.A. et al., supra.; Miyazaki, K. et al., Nature 362, 839-841 (1993). The enzyme may play a key role in the demyelination of neurones and the breakdown of the blood brain barrier which occurs in such disorders.

### SUMMARY OF THE INVENTION

The present invention provides novel aminomalonamides of the formula 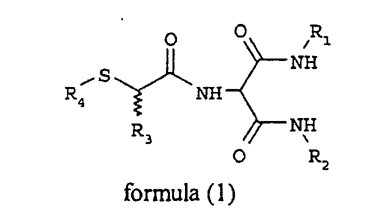 wherein
R₁ and R₂ are each independently selected from the group consisting of hydrogen, C₁-C₁₀ alkyl, -(CH₂)ₐ-Ar₁, and -(CH₂)_{b}-Ar₂;
wherein
a is an integer from 1 to 6;
b is an integer from 2 to 6;
Ar₁ is a radical selected from the group consisting of 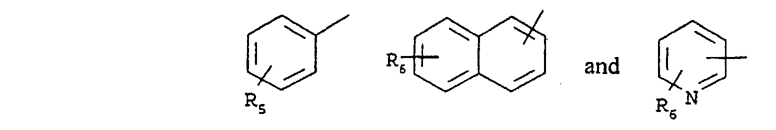 wherein
R₅ is 1 or 2 substituents independently selected from the group consisting of hydrogen, halogen, C₁₋C₄ alkyl, hydroxy, and C₁₋C₄ alkoxy;
R₆ is selected from the group consisting of hydrogen, halogen, C₁₋C₄ alkyl, and C₁₋C₄ alkoxy;
Ar₂ is the radical 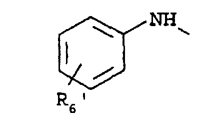 wherein
R_{6'} is selected from the group consisting of hydrogen, halogen, C₁₋C₄ alkyl, and C₁₋C₄ alkoxy;
R₃ is selected from the group consisting of C₁-C₆ alkyl, -(CH₂)ₘ-W, -(CH₂)ₚ-Ar₃, -(CH₂)ₖ-CO₂R₉, -(CH₂)ₘ-NR₈-SO₂-Y₁, and -(CH₂)ₘ-Z-Q
wherein
m is an integer from 2 to 8;
p is an integer from 0-10;
k is an integer from 1 to 9;
W is phthalimido;
Ar₃ is selected from the group consisting of 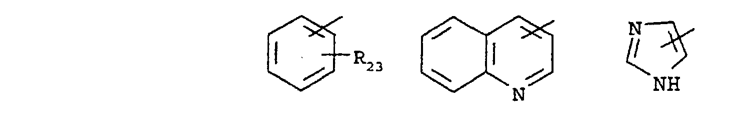 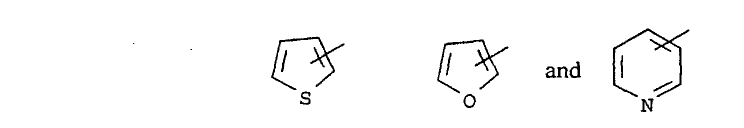 wherein
R₂₃ is from 1 to 2 substituents independently selected from the group consisting of hydrogen, halogen, C₁-C₄ alkyl, and C₁-C₄ alkoxy;
R₈₋ is hydrogen or C₁-C₆ alkyl;
R₉ is hydrogen or C₁-C₆ alkyl;
Y₁ is selected from the group consisting of hydrogen, -(CH₂)ⱼ-Ar₄, and -N(R₂₄)₂
wherein
j is 0 or 1;
R₂₄ each time selected is independently hydrogen or C₁-C₆ alkyl or are taken together with the nitrogen to which they are attached to form N-morpholino, N-piperidino, N-pyrrolidino, or N-isoindolyl;
Ar₄ is the radical 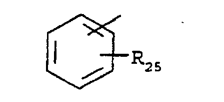 wherein
R₂₅ is from 1 to 3 substituents independently selected from the group consisting of hydrogen, halogen, C₁-C₄ alkyl, and C₁-C₄ alkoxy;
Z is selected from the group consisting of -O-, -NR₈-, -C(O)NR₈-, -NR₈C(O)-, -NR₈C(O)NH-, -NR₈C(O)O-, and -OC(O)NH-;
wherein
R₈ is hydrogen or C₁-C₆ alkyl;
Q is selected from the group consisting of hydrogen, -(CH₂)ₙ-Y₂, and -(CH₂)ₓY₃;
wherein
n is an integer from 0 to 4;
Y₂ is selected from the group consisting of hydrogen, -(CH₂)ₕ-Ar₅ and -(CH₂)ₜ-C(O)OR₂₇
wherein
Ar₅ is selected from the group consisting of 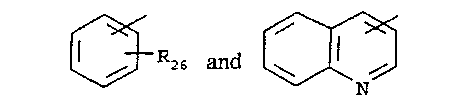 wherein
R₂₆ is from 1 to 3 substituents independently selected from the group consisting of hydrogen, halogen, C₁-C₄ alkyl, and C₁-C₄ alkoxy;
h is an integer from 0 to 6;
t is an integer from 1 to 6;
R₂₇ is hydrogen or C₁-C₆ alkyl;
x is an integer from 2 to 4;
Y₃ is selected from the group consisting of -N(R₂₈)₂, N-morpholino, N-piperidino, N-pyrrolidino, and N-isoindolyl;
wherein
R₂₈ each time taken is independently hydrogen or C₁-C₆ alkyl;
R₄ is selected from the group consisting of hydrogen, -C(O)R₁₀, -C(O)-(CH₂)_{q}-K and - S-G
wherein
R₁₀ is selected from the group consisting of hydrogen. C₁-C₄ alkyl, phenyl, and benzyl;
q is 0, 1, or 2;
K is selected from the group consisting of 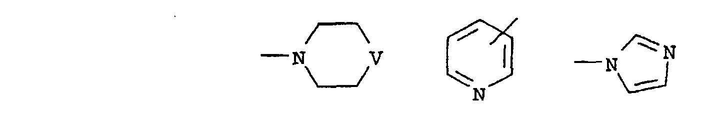 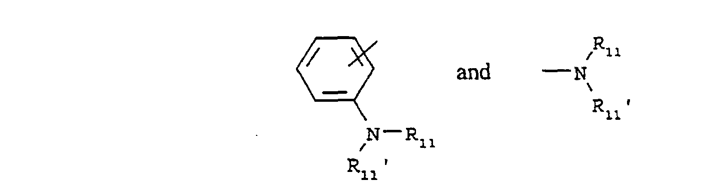 wherein
V is selected from the group consisting of a bond. -CH₂-, -O-, -S(O)ᵣ-, -NR-, and -NC(O)R'-;
wherein
r is 0, 1, or 2;
R is selected from the group consisting of hydrogen, C₁₋C₄ alkyl, and benzyl;
R' is selected from the group consisting of hydrogen, -CF₃, C₁-C₁₀ alkyl, phenyl, and benzyl;
R₁₁ is selected from the group consisting of hydrogen, C₁₋C₄ alkyl, and benzyl;
R₁₁⁻ is selected from the group consisting of hydrogen, C₁₋C₄ alkyl, and benzyl;
G is selected from the group consisting of 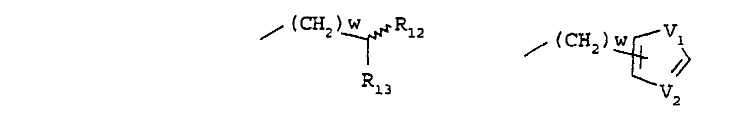 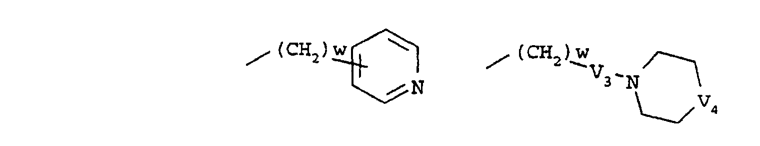 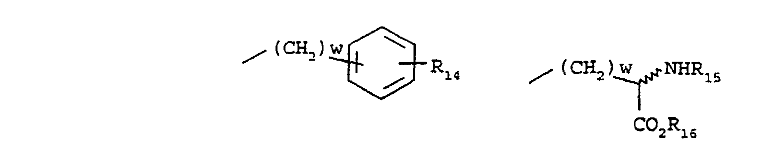 and 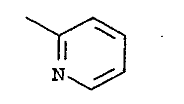 wherein
w is an integer from 1 to 3;
R₁₂ is selected from the group consisting of hydrogen. C₁-C₆ alkyl, -CH₂CH₂S(O)ₑCH₃, and benzyl;
wherein e is 0, 1, or 2;
R₁₃ is selected from the group consisting of hydrogen, hydroxy, amino, C₁-C₆ alkyl, N-methylamino, N,N-dimethylamino, -CO₂R₁₇, and-OC(O)R₁₈;
wherein
R₁₇ is hydrogen, -CH₂O-C(O)C(CH₃)₃, C₁₋C₄ alkyl, benzyl, or diphenylmethyl;
R₁₈ is hydrogen, C₁-C₆ alkyl or phenyl;
R₁₄ is 1 or 2 substituents independently selected from the group consisting of hydrogen, C₁₋C₄ alkyl, C₁₋C₄ alkoxy, or halogen;
V₁ is selected from the group consisting of -O-, -S-. and -NH-;
V₂ is selected from the group consisting of -N- and -CH-;
V₃ is selected from the group consisting of a bond and -C(O)-;
V₄ is selected from the group consisting of -O-, -S-. -NR₁₉-, and -NC(O)R₂₀-;
wherein
R₁₉ is hydrogen, C₁₋C₄ alkyl, or benzyl;
R₂₀ is hydrogen, -CF₃, C₁-C₁₀ alkyl, or benzyl;
R₁₅ is selected from the group consisting of hydrogen, C₁-C₆ alkyl and benzyl;
R₁₆ is selected from the group consisting of hydrogen and C₁₋C₄ alkyl;
and stereoisomers, pharmaceutically acceptable salt, and hydrate thereof.

The present invention further provides a method of inhibiting matrix metalloproteinases (MMPs) in a patient in need thereof comprising administering to the patient an effective matrix metalloproteinase inhibiting amount of a compound of formula (1). As such the present invention provides a method of treating a neoplastic disease state or cancer; rheumatoid arthritis; osteoarthritis; osteoporosis; cardiovascular disorders, such as atherosclerosis; comeal ulceration; dental diseases, such as gingivitis or periodontal disease; and neurological disorders, such as multiple sclerosis; chronic inflammatory disorders, such as emphysema and especially smoking-induced emphysema.

In addition, the present invention provides a composition comprising an assayable amount of a compound of formula (1) in admixture or otherwise in association with an inert carrier. The present invention also provides a pharmaceutical composition comprising an effective MMP inhibitory amount of a compound of formula (1) in admixture or otherwise in association with one or more pharmaceutically acceptable carriers or excipients.

As used in this application:
a) the term "halogen" refers to a fluorine atom, chlorine atom, bromine atom, or iodine atom;
b) the term "C₁-C₆ alkyl" refers to a branched or straight chained alkyl radical containing from I to 6 carbon atoms, such as methyl, ethyl, n-propyl, isopropyl, n-butyl, isobutyl, t-butyl, pentyl, hexyl, etc.;
c) the term "C₁-C₄ alkyl" refers to a saturated straight or branched chain alkyl group containing from 1 to 4 carbon atoms and includes methyl, ethyl, propyl, isopropyl, n-butyl, s-butyl, isobutyl, and t-butyl;
d) the term "C₁-C₄ alkoxy" refers to a straight or branched alkoxy group containing from I to 4 carbon atoms, such as methoxy, ethoxy, n-propoxy, isopropoxy, n-butoxy, isobutoxy, t-butoxy, etc.;
e) the term "C₁-C₁₀ alkyl" refers to a saturated straight or branched chained alkyl group containing from 1 to 10 carbon atoms and includes methyl, ethyl, n-propyl, isopropyl, n-butyl, isobutyl, t-butyl, pentyl, hexyl, heptyl, octyl, nonyl, and decyl, etc.;
f) as used in the examples and preparations, the following terms have the meanings indicated: "g" refers to grams, "mg" refers to milligrams. "µg" refers to micrograms, "mol" refers to moles, "mmol" refers to millimoles. "nmole" refers to nanomoles, "L" refers to liters, "mL" or "ml" refers to milliliters, "µL" refers to microliters, "°C" refers to degrees Celsius, "R_{f}" refers to retention factor, "mp" refers to melting point, "dec" refers to decomposition, "bp" refers to boiling point, "mm of Hg" refers to pressure in millimeters of mercury, "cm" refers to centimeters, "nm" refers to nanometers, "brine" refers to a saturated aqueous sodium chloride solution, "M" refers to molar, "mM" refers to millimolar, "µM" refers to micromolar, "nM" refers to nanomolar, "HPLC" refers to high performance liquid chromatography, "HRMS" refers to high resolution mass spectrum, "DMF" refers to dimethylformamide, "µCi" refers to microcuries, "i.p." refers to intraperitoneally, "i.v." refers to intravenously, and "DPM" refers to disintegrations per minute;
g) the term "pharmaceutically acceptable salts" refers to either an acid addition salt or a basic addition salt.

The expression "pharmaceutically acceptable acid addition salts" is intended to apply to any non-toxic organic or inorganic acid addition salt of the base compounds represented by formula (1) or any of its intermediates. Illustrative inorganic acids which form suitable salts include hydrochloric, hydrobromic, sulphuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic, p-toluenesulfonic acid, and sulfonic acids such as methane sulfonic acid and 2-hydroxyethane sulfonic acid. Such salts can exist in either a hydrated or substantially anhydrous form. In general, the acid addition salts of these compounds are soluble in water and various hydrophilic organic solvents, and which in comparison to their free base forms, generally demonstrate higher melting points.

The expression "pharmaceutically acceptable basic addition salts" is intended to apply to any non-toxic organic or inorganic basic addition salts of the compounds represented by formula (1) or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine. dimethylamine, trimethylamine, and picoline.

As is appreciated by one of ordinary skill in the art the compounds of formula (1) exist as stereoisomers. Specifically, it is recognized that they exist as stereoisomers at the point of attachment of the substituents R₃, R₁₂, and -NHR₁₅. Where indicated the compounds of this application, whether of formula (1), starting materials, or intermediates, follow either the (+)- and (-)- designation for optical rotation, the (D)-and (L)- designation of relative stereochemistry, or the Cahn-Ingold-Prelog designation of (R)-and (S)- for the stereochemistry. Any reference in this application to one of the compounds of the formula (1) is meant to encompass either specific stereoisomers or a mixture of stereoisomers.

The specific stereoisomers can be prepared by stereospecific synthesis using enantiomerically pure or enantiomerically enriched starting materials which are well known in the art. The specific stereoisomers of amino acid starting materials are commercially available or can be prepared by stereospecific synthesis as is well known in the art or analogously known in the art, such as D. A. Evans, et al. J. Am. Chem. Soc., 112, 4011-4030 (1990); S. Ikegami et al. Tetrahedron, 44, 5333-5342 (1988); W. Oppolzer et al. Tet. Lets. 30. 6009-6010 (1989); Synthesis of Optically Active α-Amino-Acids. R. M. Williams (Pergamon Press, Oxford 1989); M. J. O'Donnell ed.: α-Amino-Acid Synthesis, Tetrahedron Symposia in print. No. 33, Tetrahedron 44, No. 17 (1988); U. Schöllkopf, PureAppl. Chem. 55, 1799 (1983); U. Hengartner et al. J. Org. Chem., 44, 3748-3752 (1979); M. J. O'Donnell et al. Tet. Lets., 2641-2644 (1978); M. J. O'Donnell et al. Tet. Lets. 23, 4255-4258 (1982); M. J. O'Donnell et al. J. Am. Chem. Soc., 110, 8520-8525 (1988).

The specific stereoisomers of either starting materials or products can be resolved and recovered by techniques known in the art, such as chromatography on chiral stationary phases, enzymatic resolution, or fractional recrystallization of addition salts formed by reagents used for that purpose. Useful methods of resolving and recovering specific stereoisomers are known in the art and described in Stereochemistry of Organic Compounds, E. L. Eliel and S. H. Wilen, Wiley (1994) and Enantiomers, Racemates, and Resolutions. J. Jacques, A. Collet, and S. H. Wilen. Wiley (1981).

As with any group of structurally related compounds which possess a particular utility, certain groups and configurations of substituents are preferred for the compounds of formula (1). Preferred embodiments are given below:
The compounds in which R₁ and R₂ are selected from the group consisting of C₁-C₆ alkyl and -(CH₂)ₐ-Ar₁ are preferred;
The compounds in which R₁ and R₂ are -(CH₂)ₐ-Ar₁ are more preferred;
The compounds in which R₁ and R₂ are -(CH₂)ₐ-Ar₁ in which a is 1 or 2 and Ar₁ is phenyl or substituted phenyl are most preferred;
The compounds in which R₃ is selected from the group consisting of C₁-C₆ alkyl and -(CH₂)ₚ-Ar₃ are preferred;
Compounds in which R₄ is selected from the group consisting of hydrogen, -C(O)R₁₀ and -S-G are preferred;
Compounds in which R₄ is hydrogen are more preferred; and
Compounds in which R₄ is selected from the group consisting of -C(O)R₁₀ and R₁₀ is C₁₋C₄ alkyl more preferred.

Examples of compounds encompassed by the present invention include the following. It is understood that the examples encompass all of the isomers of the compound and mixtures thereof. This list is meant to be representative only and is not intended to limit the scope of the invention in any way:
N.N'-di-(3-diphenylpropyl)-2-((S)-2-mercapto-4-phenylbutryrylamino)malonamide;
N,N'-di-(4-diphenylbutyl)-2-((S)-2-mercapto-4-phenylpropionylamino)malonamide; and
N,N'-di-(3-diphenylpropyl)-2-((S)-2-mercapto-4-phenylbutyrylamino)malonamide.

The compounds of formula (1) can be prepared by utilizing techniques and procedures well known and appreciated by one of ordinary skill in the art. To illustrate, general synthetic schemes for preparing intermediates and the compounds of formula (1) are set forth below. In the reaction schemes below, the reagents and starting materials are readily available to one of ordinary skill in the art and all substituents are as 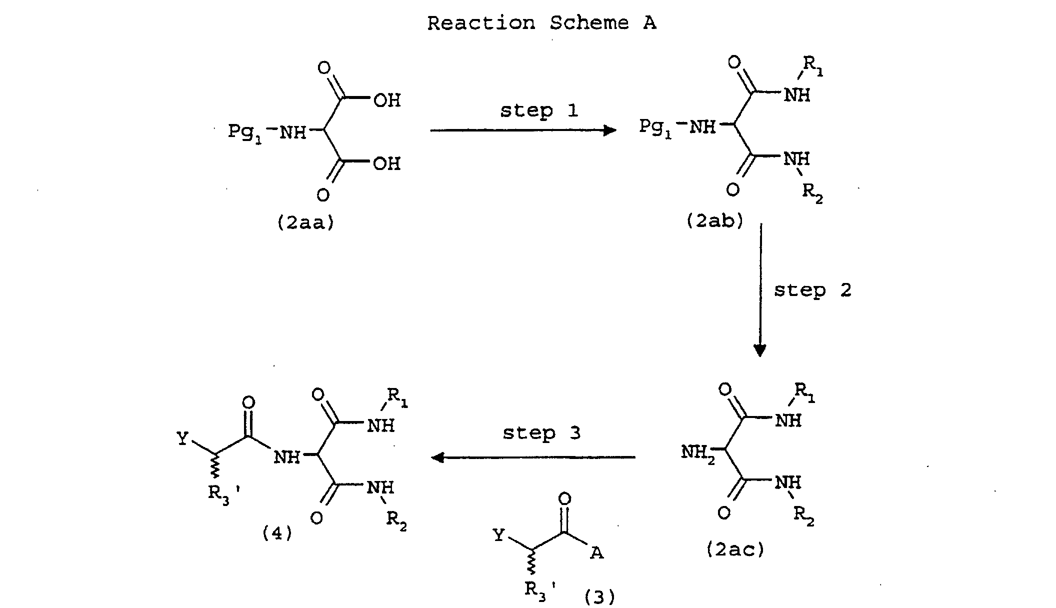 previously defined unless otherwise indicated.

In Scheme A, step 1, an appropriate protected aminomalonic acid derivative of the formula (2aa) is coupled with an appropriate amine to give a compound of formula (2ab). An appropriate protected aminomalonic acid derivative of the formula (2aa) is one in which the protecting group, Pg₁, can be removed in the presence of the amide formed in this step. The use of t-Boc for Pg₁ is preferred.

Such an appropriate amino protected aminomalonic acid derivatives are readily prepared by amine protecting diethyl aminomalonate followed by ester hydrolysis. An appropriate amine is one which gives rise to R₁ and R₂ as desired in the final product of formula (1). As is appreciated by the person skilled in the art, compounds of formula (1) in which R₁ and R₂ are different can be prepared by using mono-carboxy protected aminomalonic acid derivatives using this step, followed by selective removal of the carboxy protecting group and repeated amide formation to give compounds o formula (2ab)in which R₁ and R₂ are different.

Such coupling reactions to form amides are carried out in suitable solvents, such as dichloromethane, tetrahydrofuran, diethyl ether, chloroform, and other suitable solvents intended to have the same effect, and using suitable bases, such as triethylamine, N-methylmorpholine, N,N-disopropylethylamine, pyridine, and other suitable bases intended to have the same effect, and coupling reagents, as required, and are well known and appreciated in the art. The reactions are generally carried out at -10°C to the refluxing temperature of the solvent and generally require form 1 hour to 2 days. The product can be isolated and purified by techniques well known in the art, such as extraction, evaporation, trituration, lyophilization, chromatography, and recrystallization.

The selection of an appropriate coupling reagent is within the skill of the art. Particularly suitable coupling reagents include 1-ethyl-3-(3-(dimethylamino) propyl)carbodiimide and 1-hydroxy-benzotriazole or N,N'-diisopropylcarbodiimide and 1-hydroxy-benzotriazole. Other coupling agents are benzotriazol-1-yloxytris(pyrrolidino)phosphonium hexafluorophosphate complex pyridine benzotriazol-1-yloxytris(dimethylamino)phosphonium hexafluorophosphate complex , carbodiimides (e.g., N,N'-dicyclohexylcarbodiimide); cyanamides (e.g., N,N-dibenzylcyanamide); (3) ketenimines; isoxazolium salts (e.g., N-ethyl-5-phenyl-isoxazolium-3'-sulfonate; monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl- di-1,2,4-triazole; alkoxylated acetylene (e.g., ethoxyacetylene); reagents which form a mixed anhydride with the carboxyl moiety of the amino acid (e.g., ethylchloroformate and isobutylchloroformate). Other activating reagents and their use in peptide coupling are described by Kapoor, J. Pharm. Sci., 59, 1-27(1970).

In Reaction Scheme A, step 2, the amino protecting group, Pg₁, of the compound of formula (2ab) is selectively removed to give the compound of formula (2ac). Such selective amino deprotection reactions are well known and appreciated in the art. The product can be isolated and purified by techniques well known in the art, such as extraction, evaporation, salt formation, trituration, lyophilization, chromatography, and recrystallization

In Reaction Scheme A, step 3, a compound of formula (2ac) coupled with an appropriate acid derivative bearing R₃' and Y (compound of formula (3)) to give a compound of formula (4). Such coupling reactions are well known and appreciated in the art and discussed above. The product o can be isolated and purified by techniques well known in the art such as extraction, evaporation, salt formation, trituration, lyophilization, chromatography, and recrystallization.

An appropriate compound of formula (3) is one in which R₃, is R₃ as desired in the final product of formula (1) or gives rise after deprotection to R₃ as desired in the final product of formula (1) and Y is a protected thio substituent or Y may be a protected hydroxy substituent or bromo which gives rise upon selective deprotection and displacement or displacement and further deprotection and/or elaboration, if required, to -SR₄ as desired in the final product of formula (1). Alternately, an appropriate compound of formula (3) may also be one in which R_{3'} gives rise to R_{3"} which, upon derivatization, gives rise R₃ as desired in the final product of formula (1) and Y is a protected thio substituent. In addition, an appropriate compound of formula (3) may also be one in which the stereochemistry at the R_{3'} and Y bearing carbon is as desired in the final product of formula (1) or gives rise after displacement to the stereochemistry as desired at that carbon in the final product of formula (1). The activating group (A) is one which undergoes an amidation reaction. As is well known in the art an amidation reaction may proceed through an acid, A is -OH; or an acid may be first converted to an acid chloride, A is -Cl; or an activated intermediate; such as an anhydride; a mixed anhydride of aliphatic carboxylic acid, such as formic acid, acetic acid, propionic acid, butyric acid, isobutyric acid, pivalic acid, 2-ethylbutyric acid, trichloroacetic acid, trifluoroacetic acid, and other mixed anhydrides of aliphatic carboxylic acids intended to have the same effect; of aromatic carboxylic acids, such as benzoic acid and other mixed anhydrides of aromatic carboxylic acids intended to have the same effect ; of an activated ester, such as phenol ester, p-nitrophenol ester, 2,4-dinitrophenol ester, pentafluorophenol ester, pentachlorophenol ester, N-hydroxysuccinimide ester, N-hydroxyphthalimide ester, 1-hydroxy-1H-benztriazole ester, and other mixed anhydrides of activated esters intended to have the same effect; activated amide, such as imidazole, dimethylpyrazole, triazole, or tetrazole; or an intermediate formed in the presence of coupling agents, such as dicyclohexylcarbodiimide or 1-(3-dimethyaminopropyl)-3- ethylcarbodiimide. Acid chlorides and activated intermediates may be prepared but is not necessarily isolated before the addition of diethyl aminomalonate.

The use and selection of appropriate protecting groups is within the ability of those skilled in the art and will depend upon compound of formula (3) to be protected, the presence of other protected amino acid residues, other protecting groups, and the nature of the particular R₃ and/or R₄ group(s) ultimately being introduced. Compounds of formula (3) in which Y is bromo and protected thio are commercially available or can be prepared utilizing materials, techniques, and procedures well known and appreciated by one of ordinary skill in the art or described herein. See PCT Application WO 96/11209, published 18 April 1996. Examples commercially available compounds of formula (3) in which Y is bromo include 2-bromopropionic acid, 2-bromobutyric acid, 2-bromovaleric acid, 2-bromohexanoic acid, 6-(benzoylamino)-2-bromohexanoic acid, 2-bromoheptanoic acid, 2-bromooctanoic acid, 2-bromo-3-methylbutyric acid, 2-bromoisocaproic acid, 2-bromo-3-(5-imidazoyl)proionic acid, (R)-(+)-2-bromopropionic acid, (S)-(-)-2-bromopropionic acid.

The compound of formula (4) can also be prepared as set forth below in Reaction Scheme B.

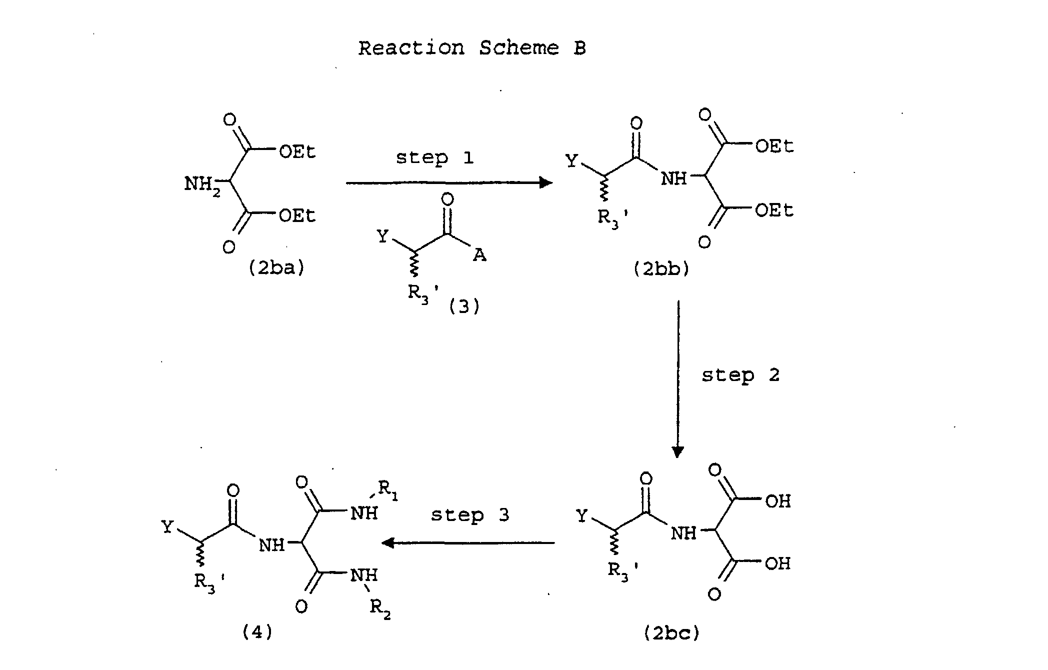

In Scheme B, step 1, diethyl aminomalonate (formula (2ba) is coupled with an appropriate acid derivative of formula (3) to give a compound of formula (2bb). In Reaction Scheme B, an appropriate acid derivative of formula (3) is one in which R_{3'} is as describe above in Reaciton Scheme A and Y is a protected thio group which is stable to the hydrolysis reaction of step 2. In Reaction Scheme B, the use of compoudns in which Y is p-methoxybenzylmercapto is preferred. Such coupling reactions are carried out in suitable solvents and using suitable bases and coupling agents, as required, and are well known and appreciated in the art and are discussed above.

In Reaction Scheme B, step 2, the compound of formula (2bb) is hydrolyzed to give a the diacid of formula (2bc). The hydrolysis of esters can be carried out under acidic or basic conditions as is well known in the art.

In Reaction Scheme B, step 3, a compound of formula (2bc) is coupled with a appropriate amine as described in Reaction Scheme A, step 1. above, to give a compound of formula (4).

In Reaction Scheme C a compound of formula (4) in which R_{3'} is R₃ as desired in the final product of formula (1) or gives rise after deprotection to R₃ as desired in the final product of formula (1) and Y is a protected thio substituent or hydroxy or bromo gives rise to a final product of formula (1).

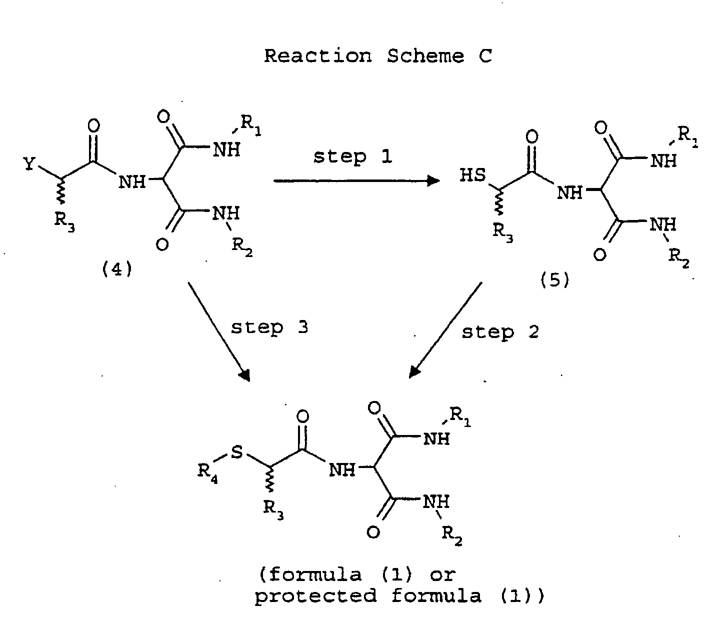

In Reaction Scheme C, step 1, a compound of formula (4) in which Y is protected thio gives rise upon selective deprotection to give a compound of formula (5).

For example, compounds of formula (4) in which Y is a protected thio substituents are selectively deprotected to give a thiol of formula (5). Protected thio substituents include thioesters, such as thioacetyl or thiobenzoyl, thioethers, such as thiobenzyl, thio-4-methoxybenzyl, thiotriphenylmethyl, or thio-t-butyl, or unsymmetrical sulfides, such as dithioethyl or dithio-t-butyl. The use and selective removal of such thio protecting groups is well known and appreciated in the art and described in Protective Groups in Organic Synthesis, Theodora W. Greene (Wiley-Interscience, 2nd Edition, 1991).

In Reaction Scheme C, step 2, a compound of formula (5) undergoes modification reaction to give a compound of formula (1). Such modification reactions include, thiol esterification and disulfide formation.

Compounds of formula (1) in which R₄ is -C(O)R₁₀ or -C(O)-(CH₂)_{q}-X group can be synthesized by thiol esterifications according to techniques'well known and appreciated by one of ordinary skill in the art, such as those disclosed in U.S. Pat. Nos. 5.424,425, issued Jun. 13, 1995.

For example, in a thiol esterification a compound of formula (5) is contacted with about an equimolar amount of an appropriate acid, such as HO-C(O)R₁₀ or HO-C(O)-(CH₂)_{q}-X in the presence of a suitable coupling agent to give a compound of formula (1) in which R₄ is -C(O)R₁₀ or -C(O)-(CH₂)_{q}-X. The reaction is carried out in the presence of a coupling agent such as 2-fluoro-1-methylpyridinium p-toluenesulfate, EDC (1-(3-dimethylaminopropyl)-3-ethylcarbodiimide hydrochloride), carbonyldiimidazole, EEDQ (1-ethoxycarbonyl-2-ethoxy-1,2-dihydroquinoline, DCC, or diethylcyanophosphonate in a suitable aprotic solvent such as methylene chloride. The reaction is generally carried out at temperature of between -20°C and the boiling point of the solvent. Generally, the reaction requires 1 to 24 hours. The product can be isolated and purified by techniques well known in the art, such as extraction, evaporation, trituration, lyophilization. chromatography. and recrystallization.

Compounds of formula (1) in which R₄ is -S-G group can be synthesized according to techniques well known and appreciated by one of ordinary skill in the art, as disclosed in PCT Application No. WO 95/21839, published 17 August 1995 and U.S. Patent Nos. 5,491,143, issued February 13, 1996, and 5,731,306, issued March 24, 1998, and Roques. B.P. et al., J. Med. Chem. 33, 2473-2481 (1992).

For example, in a disulfide formation a compound of formula (5) is contacted with an appropriate compound of formula (7).

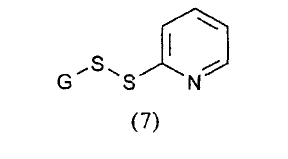

An appropriate compound of formula (7) is one which gives G as desired in the final product of formula (1) or gives rise upon deprotection to G as is desired in the final product of formula (1). In addition, the compound of formula (7) may have stereochemistry as desired in the final product of formula (1). The reaction is carried out in a suitable solvent, such as ethanol, methanol, dichloromethane, or mixtures of ethanol or methanol and dichloromethane. The solvent is degassed by passing a stream of nitrogen gas through it for 15 minutes before the reaction is carried out. The reaction is carried out using from 1.0 to 4.0 molar equivalents of an appropriate compound of formula (7). The reaction is carried out at temperatures of from 0°C to the refluxing temperature of the solvent, with a temperature of 10°C to 30°C being preferred. The reaction generally requires from 1 to 48 hours. The product can be isolated by techniques well known in the art, such as extraction, evaporation, and precipitation and can be purified by chromatography and recrystallization.

In Reaction Scheme C, step 3, a compound of formula (4) in which Y is hydroxy or bromo can be displaced by an appropriate thiol, HSR₄, to give a compound of formula (1) or a protected compound of formula (1). In Reaction Scheme C, step 3, an appropriate thiol HSR₄ is one which gives R₄ as desired in the final product of formula (1) or gives rise upon deprotection to R₄ as desired in the final product of formula (1).

In Reaction Scheme C, step 3, a compound of formula (4) in which Y is hydroxy (obtained from protected hydroxy compounds of formula (4)) undergoes a displacement reaction with an appropriate thio introducing reagent by the method of Mitsunobu to give a compound of formula (4) in which Y is a protected thio substituent or -SR₄ as desired in the final compound of formula (1). For example, a compound of formula (4) in which Y is hydroxy reacts with thioacetic acid or thiobenzoic acid, triphenylphosphine, and diethylazodicarboxylate in a suitable aprotic solvent, such as tetrahydrofuran to give a compound of formula (4) in which Y is thioacetyl or thiobenzoyl. Selective removal of the thioacetic acid or thiobenzoic acid moiety gives the desired compound of formula (5). The product can be isolated and purified by techniques well known in the art, such as extraction, evaporation, trituration, lyophilization, chromatography, and recrystallization.

Also, in Reaction Scheme C, step 3, a compound of formula (4) in which Y is bromo undergo a displacement reaction with an appropriate thio introducing reagent to give a compound of formula (4) in which Y is protected thio substituent which gives rise upon deprotection and subsequent elaboration, if desired, the -SR₄ as desired in the final compound of formula (1). An appropriate thio introducing reagent is also one which introduces a group
-SR₄ as desired in the final compound of formula (1).

For example, a solution of p-methoxybenzylmercaptan in a suitable organic solvent such as dimethylformamide is degassed and treated with a suitable base such as sodium hydride, sodium hydroxide, or cesium carbonate. After about 1 to 2 hours, a solution of a compound of formula (4) in which Y is bromo is added. The reaction may benefit from the addition of a suitable catalyst, such as tetra-n-butylammonium iodide. The reaction mixture is carried out for 1 to 25 hours at temperatures ranging form 0°C to about 100°C. Selective removal of the 4-methoxybenzyl moiety gives the desired compound of formula (1). The product can be isolated and purified by techniques well known in the art, such as extraction, evaporation, trituration, lyophilization, chromatography, and recrystallization.

In addition, in Reaction Scheme C, step 3, a compound of formula (4) in which Y is bromo can be displaced by an appropriate thio ester, Ph₃S-C(O)-(CH₂)_{q}-X by techniques well known and appreciated in the art, as disclosed in U.S. Pat. No. 5,424,425, issued Jun. 13, 1995.

In Reaction Scheme C, in an optional step, a protected compound of formula (1) is deprotected to give a compound of formula (1). Such deprotection reactions are well known appreciated in the art and may include selective deprotections.

In Reaction Scheme D a compound of formula (4a) in which R₃' gives rise to R₃" and Y is -SR₄ as is desired in the final product of formula (1) or a protected thio substituent gives a compound of formula (1).

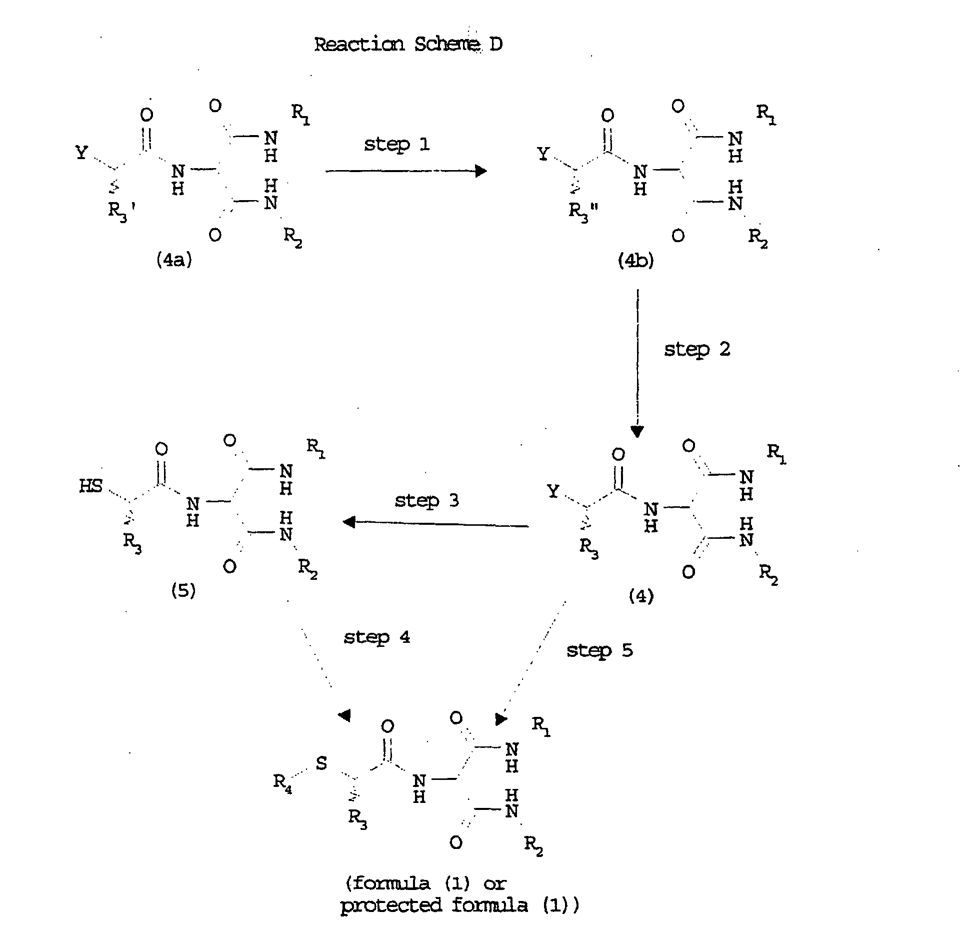

In Reaction Scheme D, step 1, an appropriate compound of formula (4a) is deprotected, hydrolyzed, or reduced to give a compound of formula (4b). In Reaction Scheme D, step 1, an appropriate compound of formula (4a) is one in which R_{3'} gives rise to a compound of formula (4b) in which R_{3"} is R₃ as desired in the final product of formula (1) or R_{3"} undergoes further derivitization (step 2) to give a compound of formula (4a) in which R₃ is a desired in the final product of formula (1). In Reaction Scheme D, step 1, an appropriate compound of formula (4a) is one in which Y is -SR₄ as desired in the final compound of formula (1) or Y is protected thio which gives rise upon deprotection or deprotection and further functionalization to give -SR₄, as desired, in the final product of formula (1) as described in Reactin Scheme C, step 2, above.

For example, in a deprotection a compound of formula (4a) in which R₃' is -(CH₂)ₘ-W (phthalimido group) is contacted with a molar excess of hydrazine monohydrate to give a compound of formula (4b) in which R_{3"} is -(CH₂)ₘ-NHR₈ in which R₈ is hydrogen. The reaction is typically carried out in a protic organic solvent, such as methanol or ethanol. The reaction is generally carried out at room temperature for a period of time ranging from 5-24 hours. The product can be isolated by techniques well known in the art, such as extraction, evaporation, and precipitation and can be purified by chromatography and recrystallization.

Alternately, for example, in a deprotection a compound of formula (4a) in which R_{3'} is -(CH₂)ₘ-NR₈-t-Boc is contacted with a molar excess of a suitable acid to give a compound of formula (4b) in which R_{3"} is -(CH₂)ₘ-NHR₈. The reaction is typically carried out in a organic solvent, such as methanol, ethanol, ethyl acetate, diethyl ether, or dioxane. Suitable acids for this reaction are well known in the art, including hydrochloric acid, hydrobromic acid, trifluoroacetic acid, and methanesulfonic acid. The reaction is generally carried out at room temperature for a period of time ranging from 1-10 hours. The product can be isolated by techniques well known in the art, such as extraction, evaporation, and precipitation and can be purified by chromatography and recrystallization.

For example, in a hydrolysis a compound of formula (4a) in which R_{3'} is -(CH₂)ₘ-C(O)OPg₃ and Pg₃ is methyl or ethyl is contacted with about 1 to 2 molar equivalents of lithium hydroxide, sodium hydroxide, or potassium hydroxide to give a compound of formula (4b) in which R_{3"} is -(CH₂)ₘ-CO₂H. The reaction is carried out in a suitable solvent, such as methanol, ethanol methanol/water mixtures, ethanol/water mixtures, or tetrahydrofuran/water mixtures and generally requires 1 to 24 hours. The reaction is carried out at temperatures of from about 0°C to the refluxing temperature of the solvent. The resulting acid is isolated and purified by techniques well known in the art, such as acidification, extraction, evaporation, and precipitation and can be purified by trituration, precipitation, chromatography, and recrystallization.
For example, in a reduction a compound of formula (4b) in which R_{3'} is -(CH₂)ₘ₋₁-CO₂Pg₃ in which Pg₃ is methyl or ethyl is contacted with a suitable reducing agent, such as lithium borohydride, diisobutylaluminum hydride, 9-borabicyclo[3.3.1]nonane, preferably lithium borohydride to provide a compound of formula (4b) in which R_{3"} is -(CH₂)ₘ₋₁-CH₂OH. The reaction is carried out in a suitable solvent, such as dichloromethane, tetrahydrofuran, or toluene, with tetrahydrofuran being preferred. The reaction is carried out at a temperature of from about -30°C to about 50°C and generally requires from 2 to 12 hours. The product can be isolated by quenching, extraction, evaporation, and precipitation and can be purified by trituration, chromatography, and recrystallization.

In Reaction Scheme D, step 2, a compound of formula (4b) undergoes a derivitization reaction to give a compound of formula (4) in which R₃ is as desired in the final product of formula (1). Such derivitization reactions include hydrolysis of esters and ester formations as are well known in the art, ether formation, amine alkylation, formation of amides, urea formation, carbamate formation, and formation of sulfonamide. In Reaction Scheme D, step 2, the compound of formula (4b) is one in which Y is a protected thio group, such as thioacetyl, thiobenzoyl, 4-methoxybenzyl thiol or t-butylthiol.

For example, in an ether formation a compound of formula (4b) in which R_{3"} is -(CH₂)ₘ₋₁-CH₂OH is contacted with 1 to 10 molar equivalents of a suitable alkylating agent to give a compound of formula (4) in which R₃ is -(CH₂)ₘ-Z-Q in which Z is -O-. A suitable alkylating agent is one which transfers Q or protected Q as desired in the final product of formula (1), such as benzyl bromide, benzyl chloride, substituted benzyl bromide, substituted benzyl chloride, ethyl bromoacetate, t-butyl bromoaceate, ethyl 3-chloropropionate, ethyl 3-bromopropionate, ethyl 5-bromovalerate, ethyl 4-bromobutyrate, 3-chloropropionamide, 2-bromoethylbenzene, substituted 2-bromoethylbenzene, 1-chloro-3-phenylpropane, 1-bromo-4-phenylbutane, and other suitable alkylating agents intended to have the same effect, or nitrogen mustards, including 2-dimethylaminoethyl chloride, 2-diethylaminoethyl chloride, and 3-dimethylaminopropyl chloride. The reaction is carried out in a suitable solvent, such as diethyl ether, tetrahydrofuran, dimethylformamide, dimethyl sulfoxide, or acetonitrile and using a suitable base, such as sodium hydride, potassium hydride, potassium t-butoxide, and lithium diisopropylamide. The reaction is generally carried out at temperatures of -70°C and room temperature and require from about 1-24 hours. The product can be isolated by techniques well known in the art, such as extraction, evaporation, and precipitation and can be purified by chromatography and recrystallization.

Alternately, as appreciated by those skilled in the art, an ether formation can also be carried out by a procedure similar to the one above using a compound of formula (4b) in which R_{3"} is -(CH₂)ₘ₋₁-CH₂OH in which the hydroxy group is first converted to a leaving group, such as chloro, bromo, or mesylate and a suitable alcohol which transfers Q or protected Q as desired in the final product of formula (1), such as benzyl alcohol, substituted benzyl alcohol, phenol, substituted phenol, and other suitable alcohols intended to have the same effect. The conversion of hydroxy to leaving groups, such as chloro, bromo, and mesylate are well known and appreciated in the art

For example, in an amine alkylation a compound of formula (4b) in which R_{3"} is -(CH₂)ₘ-NHR₈ is contacted with 1 to 10 molar equivalents of a suitable akylating agent to give a compound of formula (4) in which R₃ is -(CH₂)ₘ-Z-Q in which Z is -NR₈-. The reaction may be carried out after protection of the amine function of R_{3"} in which R₈ is hydrogen by a suitable protecting group, such as benzyl or t-Boc. For an amine alkylation a suitable alkylating agent is one as described above for the ether formation and also includes alkylhalides, such as methyl iodide, methyl bromide, ethyl bromide, propyl bromide, propyl chloride, butyl bromide, butyl chloride, and other suitable alkylhalides intended to have the same effect. The reaction is carried out in a suitable solvent, such as methanol, ethanol, dimethylformamide, or pyridine and using a suitable base, such as sodium carbonate, triethylamine, N,N-diisopropylethylamine or pyridine. The reaction is generally carried out at temperatures of room temperature to the refluxing temperature of the solvent and require from about 1-24 hours. The product can be isolated by techniques well known in the art, such as extraction, evaporation, and precipitation and can be purified by chromatography and recrystallization.

Alternately, for example, in an amine alkylation a compound of formula (4b) in which R_{3"} is -(CH₂)ₘ-NHR₈ is contacted in a reductive alkylation with a suitable aldehyde to give a compound of formula (4) in which R₃ is -(CH₂)ₘ-Z-Q in which Z is -NR₈-. A suitable aldehyde is one which transfers Q or protected Q as desired in the final product of formula (1), such as benzaldehyde and substituted benzaldehydes. The reaction is carried out in a suitable solvent, such as methanol, ethanol, tetrahydrofuran, or mixtures of methanol or ethanol and tetrahydrofuran. The reaction may be carried out in the presence of a drying agent, such as sodium sulfate or molecular sieves. The reaction is carried out in the presence of from 1.0 to 6.0 molar equivalents of a suitable reducing agent, such as, sodium borohydride or sodium cyanoborohydride with sodium cyanoborohydride being preferred. It may be advantageous to maintain the pH in the range of about 4 to 6. The reaction is generally carried out at temperatures of from 0° C to the refluxing temperature of the solvent. Generally, the reactions require 1 to 72 hours. The product can be isolated by techniques well known in the art, such as extraction, evaporation, and precipitation and can be purified by chromatography and recrystallization.

For example, in an amido formation a compound of formula (4b) in which R_{3"} is is -(CH₂)ₘ-CO₂H is contacted with a suitable amine in an amide formation to give a compound of formula (4) in which R₃ is -(CH₂)ₘ-Z-Q in which Z is amido. Such amide formation reactions using carboxy activation or suitable coupling agents are well known in the art and described above. A suitable amine, HNR₈Q, gives rise to R₈ and Q as desired in the final product of formula (1), such as methylamine, ethylamine, propylamine, butylamine, N-methyl benzylamine, benzyl β-alanine, 4-(3-aminopropyl)morpholine, and other suitable amines intended to have the same effect.

For example, in an amide formation a compound of formula (4b) in which R_{3"} is is -(CH₂)ₘ-NHR₈ is contacted with a suitable carboxylic acid in an amide formation to give a compound of formula (4) in which R₃ is -(CH₂)ₘ-Z-Q in which Z is amide. Such amide formation reactions using carboxy activation or suitable coupling agents are well known in the art and described above. Suitable carboxylic acids, QC(O)-OH, are ones give rise to Q as desired in the final product of formula (1), such as benzoic acid, substituted benzoic acids, phenyl acetic acids, substituted phenylacetic acids, mono-t-butyl malonate, and other suitable carboxylic acids intended to have the same effect.

For example, in a urea formation a compound of formula (4b) in which R_{3"} is is -(CH₂)ₘ-NHR₈ is contacted with an appropriate isocyanate, O=C=N-Q, to give a compound of formula (4) in which R₃ is -(CH₂)ₘ-Z-Q in which Z is urea. An appropriate isocyanate is one which gives rise to Q as desired in the final product, such as phenyl isocyanate, substituted phenyl isocyanate, napthyl isocyanate, ethyl isocyanatoacetate, and other appropriate isocyanates intended to have the same effect. The reaction is carried out by adding an equivalent of, or a slight molar excess of, an appropriate isocyanate is added to a solution of a compound of formula (4b) in which R_{3"} is -(CH₂)ₘ-NHR₈ in a suitable solvent, such as diethyl ether, benzene, or toluene. The reaction is carried out at temperature of from about 0°C to the refluxing temperature of the solvent and require about 1-24 hours. The product can be isolated and purified by techniques well known in the art, such as filtration, extraction, evaporation, trituration, chromatography, and recrystallization.

For example, in an N-carbamoyl formation a compound of formula (4b) in which R_{3"} is -(CH₂)ₘ-NHR₈ is contacted with an appropriate chloroformate to give a compound of formula (4) in which R₃ is -(CH₂)ₘ-Z-Q in which Z is N-carbamoyl. An appropriate chloroformate is one which gives rise to Q as desired in the final product of formula (1). Examples of chloroformates include benzyl chloroformate, naphthyl chloroformate, phenyl chloroformate, and substituted phenyl chloroformates, such as 4-chlorophenyl chloroformate, 4-methylphenyl chloroformate, 4-bromophenyl chloroformate, 4-fluorophenyl chloroformate, 4-methoxyphenyl chloroformate and other substituted phenyl chloroformates intended to have the same effect. The reaction is carried out by adding an equivalent of, or a slight molar excess of, an appropriate chloro formate to a solution of a compound of formula (4b) in which R_{3"} is -(CH₂)ₘ-NHR₈ in a suitable solvent, such as toluene, tetrahydrofuran, dimethylformamide, dichloromethane, pyridine, or chloroform. The reaction is carried out in the presence of an excess of a suitable base, such as triethylamine, sodium carbonate, potassium bicarbonate, pyridine or N,N-diisopropylethylamine. The reaction is carried out at a temperature of from -70°C to the refluxing temperature of the solvent and generally requires from 30 minutes to 24 hours. The product can be isolated and purified by techniques well known in the art, such as extraction, evaporation, chromatography, and recrystallization.

For example, in an O-carbamoyl formation a compound of formula (4b) in which R_{3"} is -(CH₂)ₘ₋₁-CH₂OH is contacted with an appropriate isocyanate, as defined above for urea formation, to give a compound of formula (4) in which R₃ is -(CH₂)ₘ-Z-Q in which Z is O-carbamoyl. The reaction is carried out in a suitable solvent, such as diethyl ether, tetrahydrofuran, dimethylformamide, or acetonitrile. The reaction may be facilitated by the use of catalytic amount of a suitable base, such as sodium hydride, potassium hydride, or potassium t-butoxide. The reaction is generally carried out at temperatures of from -20°C to room temperature and require from about 1-24 hours. The product can be isolated by techniques well known in the art, such as extraction, evaporation, and precipitation and can be purified by chromatography and recrystallization.

For example, in a sulfonamide formation to prepare a compound in which R₃ is -(CH₂)ₘ-SO₂NR₈-Y₁, a compound of formula (4b) in which R₃- is -(CH₂)ₘ-NHR₈ is contacted with an appropriate sulfonamide forming reagent. An appropriate sulfonamide forming reagent, such as a sulfonyl chloride, Y₁S(O)₂Cl, or sulfonyl anhydride, Y₁(O)₂S-O-S(O)₂ Y₁, is one which gives rise to Y₁ as desired in the final product. Examples of appropriate sulfonamide forming reagents are, benzenesulfonyl chloride, 1-napthalenesulfonyl chloride, 2-napthalenesulfonyl chloride, dansyl chloride, N-morpholinylsulfonyl chloride, N-piperidinylsulfonyl chloride, 2,4,5-trichlorobenzenesulfonyl chloride, 2,5-dichlorobenzenesulfonyl chloride, 2,4,6-triisopropylbenzenesulfonyl chloride, 2-mesitylenesulfonyl chloride, 4-bromobenzenesulfonyl chloride, 4-fluorobenzenesulfonyl chloride, 4-chlorobenzenesulfonyl chloride, 4-methoxybenzenesulfonyl chloride, 4-t-butylbenzenesulfonyl chloride, p-toluenesulfonyl chloride, 2,3,4-trichlorobenzenesulfonyl chloride, 2,5-dimethoxybenzenesulfonyl chloride, 4-ethylbenzenesulfonyl chloride, 3,4-dimethoxybenzenesulfonyl chloride, 2,6-dichlorobenzenesulfonyl chloride, 3-bromobenzenesulfonyl chloride, 4-n-butylbenzenesulfonyl chloride, benzenesulfonic anhydride, 4-toluenesulfonic anhydride, and 2-mesitylenesulfonic anhydride. The reaction is carried out in a suitable solvent, such as tetrahydrofuran, dichloromethane, pyridine, or chloroform and in the presence of an excess of a suitable base, such as triethylamine, sodium carbonate, pyridine, or N,N-diisopropylethylamine. The reaction is carried out at a temperature of from -50°C to the refluxing temperature of the solvent. The reaction generally requires from 30 minutes to 24 hours. The product can be isolated and purified by techniques well known in the art, such as extraction, evaporation, chromatography, and recrystallization.

In Reaction Scheme D, step 3, a compound of formula (4) in which R₃ is as desired in the final product of formula (1) undergoes a selective thiol deprotection to give a compound of formula (4). Such selective thiol deprotections using suitable protecting groups are well known and appreciated in the art as discussed in Reaction Scheme C, step 1, above.

In Reaction Scheme D, step 4, a compound of formula (4) undergoes a modification reaction to give a compound of formula (1) or protected compound of formula (1) as described in Reaction Scheme C, step 2, above.

In Reaction Scheme D, step 5, a compound of formula (4a) in which Y is protected thio is deprotected to give a compound of formula (1) or to a protected compound of formula (1).

In Reaction Scheme D, in an optional step, a protected compound of formula (1) is deprotected to give a compound of formula (1). Such deprotection reactions are well known appreciated in the art and may include selective deprotections.

Alternate routes for preparing the compounds of formula (3) in which Y is bromo are presented in Reaction Schemes F.1 and F.2.

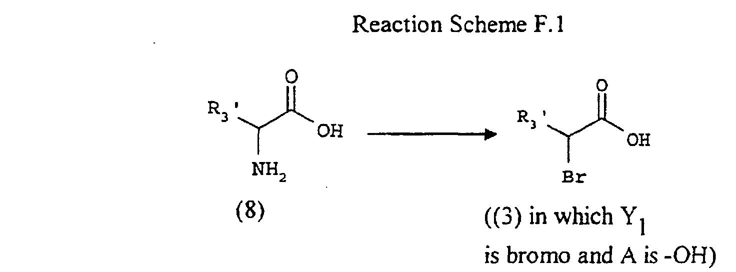

In Reaction Scheme F.1, an appropriate α-amino carboxylic acid of formula (8) is deaminobrominated to give a compound of formula (3) in which Y is bromo and A is -OH. An appropriate α-amino carboxylic acid of formula (8), and protected forms thereof, is one which is one in which R₃' is R₃ as desired in the final product of formula (1) or gives rise after deprotection to R₃ as desired in the final product of formula (1) In addition, α-amino carboxylic acid of formula (8) may also be one in which the stereochemistry at the R₃' bearing carbon gives rise after displacement to the stereochemistry as desired at that carbon in the final product of formula (1). Such appropriate α-amino carboxylic acid of formula (8), are commercially available or may be readily prepared by techniques and procedures well known and appreciated by one of ordinary skill in the art. For example, L-alanine, D-alanine, L-valine, D-valine, D-norvaline, L-leucine, D-leucine, D-isoleucine, D-tert-leucine, glycine, L-glutamic acid, D-glutamic acid, L-glutamine, D-glutamine, L-lysine, D-lysine, L-ornithine, D-ornithine, (D)-(-)-2-aminobutyric acid, D-threonine, D-homoserine, D-allothreonine, D-serine, D-2-aminoadipic acid, D-aspartic acid, D-glutamic acid, D-lysine hydrate, 2,3-diaminopropionic acid monohydrobromide, D-ornithine hydrochloride, D,L-2,4-diaminobutyric acid dihydrochloride, L-meta-tyrosine, D-4-hydroxyphenylglycine, D-tyrosine, L-phenylalanine, D-phenylalanine, D,L-2-fluorophenylalanine, beta-methyl-D,L-phenylalanine hydrochloride, D,L-3-fluorophenylalanine, 4-bromo-D,L-phenylalanine, L-phenylalanine, L-phenylglycine, D-phenylglycine, D,L-4-fluorophenylalanine, 4-iodo-D-phenylalanine, D-homophenylalanine, D,L-2-fluorophenylglycine, D,L-4-chlorophenylalanine, and other appropriate α-amino carboxylic acids intended to have the same effect, are all commercially available and the methods in D. A. Evans, et al. J. Am. Chem. Soc., 112, 4011-4030 (1990); S. Ikegami et al. Tetrahedron, 44, 5333-5342 (1988); W. Oppolzer et al. Tet. Lets. 30, 6009-6010 (1989); Synthesis of Optically Active α-Amino-Acids, R. M. Williams (Pergamon Press, Oxford 1989); M. J. O'Donnell ed.: α-Amino-Acid Synthesis, Tetrahedron Symposia in print, No. 33, Tetrahedron 44, No. 17 (1988); U. Schöllkopf, PureAppl. Chem. 55, 1799 (1983); U. Hengartner et al. J. Org. Chem., 44, 3748-3752 (1979); M. J. O'Donnell et al. Tet. Lets., 2641-2644 (1978); M. J. O'Donnell et al. Tet. Lets. 23, 4255-4258 (1982); M. J. O'Donnell et al. J. Am. Chem. Soc., 110, 8520-8525 (1988).

The deaminobromination described in Reaction Scheme F. 1 can be performed utilizing conditions described in Compagnone, R.S. and Rapoport, H., J. Org. Chem., 51, 1713-1719 (1986); U.S. Pat. No. 5,322,942, issued June 21, 1994; Overberger, C.G. and Cho, I., J. Org. Chem., 33, 3321-3322 (1968); or Pfister, K. et al.. J. Am. Chem. Soc., 71, 1096-1100 (1949).

For example, an α-amino carboxylic acid of formula (8) and a suitable bromide, such as hydrogen bromide or potassium bromide in acidic solution, such as sulfuric acid, is treated with sodium nitrite. The reaction temperature is carried out a temperatures of from about -25°C to about ambient temperature and require about 1 to 5 hours. The product can be isolated and purified by techniques well known in the art, such as acidification, extraction, evaporation, chromatography, and recrystallization to give the compound of formula (3) in which Y is bromo and A is -OH. The product can be isolated and purified by techniques well known and appreciated in the art, such as acidification, basification, filtration, extraction, evaporation, trituration, chromatography, and recrystallization.

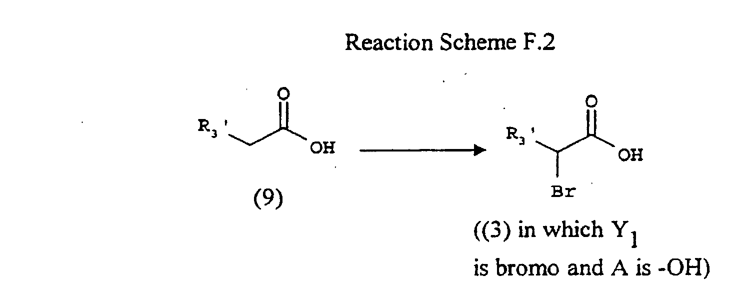

In Reaction Scheme F.2, an appropriate carboxylic acid of formula (9) is brominated to give compound of formula (3) in which Y is bromo and A is -OH. An appropriate carboxylic acid of formula (9), and protected forms thereof, is one which is one in which R_{3'} is R₃ as desired in the final product of formula (1) or gives rise after deprotection to R₃ as desired in the final product of formula (1).

For example, a mixture of a carboxylic acid of formula (9) and dry red phosphorous are treated dropwise with bromine at temperature ranging from about -20° to about 10°C. The reaction mixture is then warmed to room temperature and then heated to about 80°C for about 2-5 hours. The reaction mixture is then cooled to room temperature, poured into water containing sodium bisulfite. and neutralized using solid sodium carbonate. The aqueous layer is extracted and acidified with a suitable acid, such as concentrated hydrochloric acid. The precipitate is collected by filtration and dried to give the compound of formula (3) or formula (3b2)in which Y is bromo and A is -OH. The product can be isolated and purified by techniques well known and appreciated in the art, such as acidification, basification, filtration, extraction, evaporation, trituration, chromatography, and recrystallization.

Compounds of formula (8) and (9) in which R_{3'} is a -(CH₂)ₘ-W for use in Reaction Schemes F. 1 and F.2 are prepared according to Reaction Scheme G.1 and G.2.

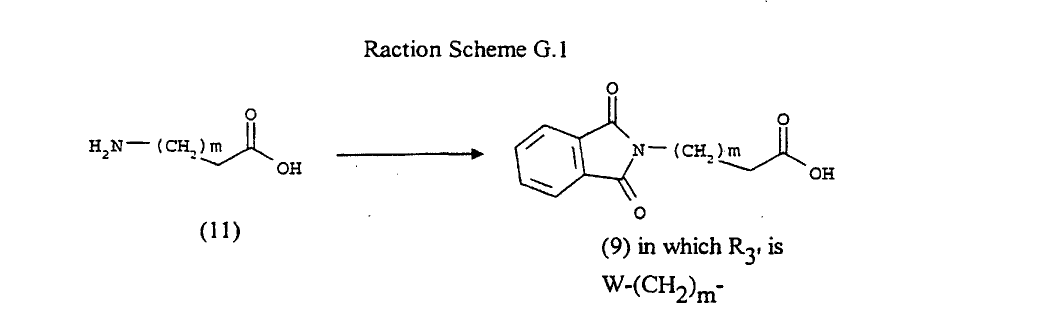

In Reaction Scheme G.1 an appropriate ω-amino carboxylic acid of formula (11) is converted to an compound of formula (9) in which R_{3'} is W-(CH₂)ₘ-. An appropriate ω-amino carboxylic acid of formula (11) is one in which m is as desired in the final product of formula (1) and are readily available in the art. For example, the reaction is carried out in a suitable polar solvent, such as water, ethanol, diethyl ether, tetrahydrofuran, or a water/ethereal solvent mixture using a suitable base, such as sodium carbonate and N-carbethoxyphthalimide. The reaction mixture is typically stirred at about ambient temperature for 1-5 hours. The product can be isolated and purified by techniques well known in the art, such as acidification, extraction, evaporation, chromatography, and recrystallization to give the desired compound of formula (9) in which R_{3'} is W-(CH₂)ₘ-.

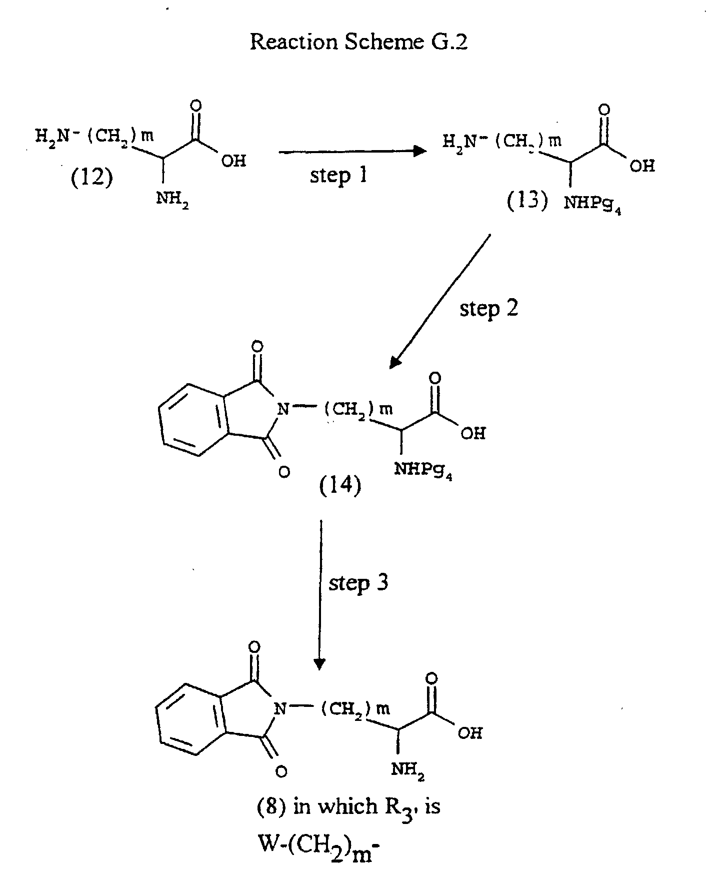

Reaction Scheme G.2, step 1, an appropriate α,ω-diamino acid of formula (12) undergoes a selective N-α-protection to give an N-a-protected-ω-diamino acid of formula (13). An appropriate α,ω-diamino acid of formula (12) is one in which m is as desired in the final product of formula (1).

For example, a selective N-α-protection of a suitable α,ω-diamino acid, such as L-lysine (formula 12 in which m is 4), is accomplished by masking the ω-amino group by formation of a benzylidene imine. The benzylidene imine is formed by dissolving L-lysine monohydrochloride in lithium hydroxide and cooling the solution to a temperature ranging from about 0° to 10°C. Freshly distilled benzaldehyde is then added and the solution is shaken. N-ω-benzylidene-L-lysine is recovered by filtration and evaporation. The α-amino group of the N-ω-benzylidene-L-lysine then undergoes protection, such as the introduction of a Cbz or t-Boc group, followed by hydrolytic cleavage of the imine *in situ* to give N-α-benzyloxy-carbonyl-L-lysine. Accordingly, N-ω-benzylidene-L-lysine is added to a mixture of sodium hydroxide and ethanol, cooled to a temperature of from about -5° to about -25°C. Then, precooled solutions of benzyloxycarbonyl chloride in a solvent, such as ethanol. is added to the reaction mixture. The temperature is maintained in a range of from about -10° to about -25°C during the course of addition, and may allowed to rise afterwards. The reaction mixture is then acidified using a suitable acid, such as precooled hydrochloric acid, and N-α-benzyloxycarbonyl-L-lysine, which corresponds to formula (13) where m is 4, is recovered by filtration evaporate and recrystallization.

In Reaction Scheme G.2, step 2, N-α-benzyloxycarbonyl-L-lysine or other compounds of formula (13) is converted to ω-phthalimido-α-benzyloxycarbonyl-L-lysine or other ω-phthalimido-α-aminoprotected carboxylic acid of formula (14) by the method described in Reaction Scheme G.1, above.

In Reaction Scheme G.2, step 3, the ω-phthalimido-α-aminoprotected carboxylic acid of formula (14) is deprotected to give compound of formula (8) in which R_{3'} is W-(CH₂)ₘ-.

For example, ω-phthalimido-α-benzyloxycarbonyl-L-lysine is contacted with hydrogen in the presence of a hydrogenation catalyst, such as 10% palladium/carbon. The reactants are typically contacted in a suitable solvent mixture such as ethanol, methanol, water, ethanol/water mixtures, or methanol/water mixtures. The reactants are typically shaken under a hydrogen atmosphere of 35-45 psi at room temperature for a period of time ranging from 5-24 hours. The product is typically recovered by filtration and evaporation of the solvent.

A route for preparing the compounds of formula (3) and formula (3b2) in which Y₁ is protected thio is presented in Reaction Scheme H. The reagents and starting materials are readily available to one of ordinary skill in the art. In Reaction Scheme H all substituents, unless otherwise indicated, are as previously defined.

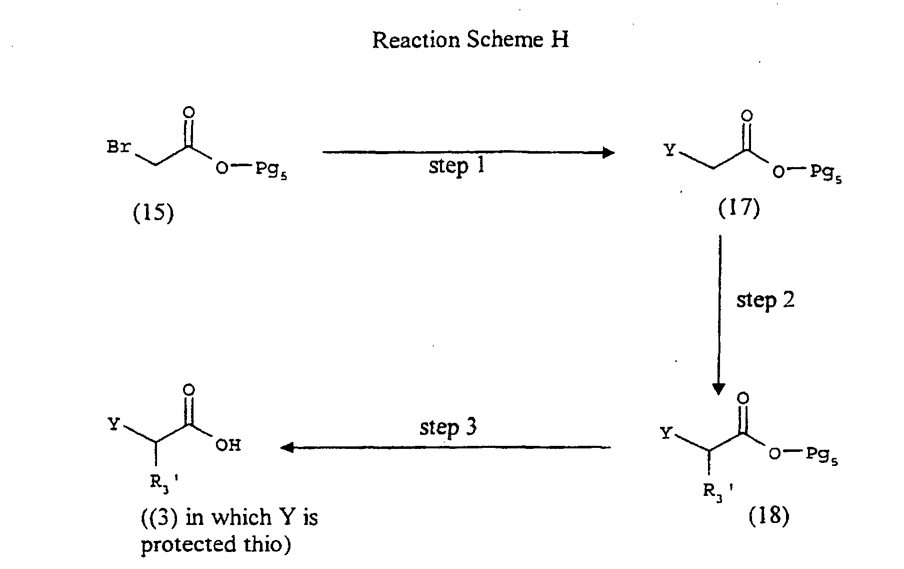

In Reaction Scheme H, step 1, a bromoacetate of formula (15) is contacted with an appropriate thiol to give a protected acetic acid ester of formula (17). In a bromoacetate of formula (15) Pg₅ is a protecting group, such as methyl, ethyl, t-butyl, and benzyl. An appropriate thiol is one which gives rise to a protected thio group, Y, in the product of formula (3b). In Reaction Scheme H, step 1, the use of 4-methoxybenzylmercaptan is preferred.

For example, a bromoacetate of formula (15) is contacted with an appropriate thiol in a suitable organic solvent, such as dimethylformamide. Advantageously, the solvent is degassed. The reaction is carried out using a suitable base, such as sodium hydroxide, triethylamine, or N,N-diisopropylethylamine. The reaction is carried out at temperatures of from about -50° to about ambient temperature and requires about I to 72 hours. The protected acetic acid ester of formula (17) can be isolated and purified by methods well known and appreciated in the art, such as extraction, evaporation, chromatography, and distillation, and recrystallization.

In Reaction Scheme H, step 2. the protected acetic acid ester of formula (17) is alkylated with an appropriate akylating agent to give a compound of formula (18). In Reaction Scheme H, step 2, an appropriate alkylating agent is one which transfers R_{3'} which is R₃ as desired in the final product of formula (1) or gives rise after deprotection to R₃ as desired in the final product of formula (1) or gives rise to R_{3"} as defined in Reaction Scheme D, step 1. Appropriate alkylating agents include alkylhalides, such as methyl iodide, methyl bromide, ethyl bromide, propyl bromide, propyl chloride, butyl bromide, butyl chloride, and the like; benzyl bromide, benzyl chloride, substituted benzyl bromide, substituted benzyl chloride, ethyl bromoacetate, t-butyl bromoaceate, ethyl 3-chloropropionate, ethyl 3-bromopropionate, ethyl 5-bromovalerate, ethyl 4-bromobutyrate, 3-chloropropionamide, 2-bromoethylbenzene, substituted 2-bromoethylbenzene, 1-chloro-3-phenylpropane, 1-bromo-4-phenylbutane, and the like, N-(2-bromoethyl)phthalimide, , N-(3-bromopropyl)phthalimide, N-(4-bromobutyl)phthalimide, and the like; 1-bromo-2-phenylethane, 1-bromo-3-phenylpropane, 1-bromo-4-phenylbutane, and the like.

For example, a protected acetic acid ester of formula (17) is alkylated with an appropriate alkylating agent. The reaction is carried out in a suitable solvent, such as diethyl ether, tetrahydrofuran, dimethylformamide, and toluene using a suitable base, such as sodium hydride, potassium hydride, potassium t-butoxide, lithium bis(trimethylsilyl)amide. sodium bis(trimethylsilyl)amide, potassium bis(trimethylsilyl)amide, or lithium diisopropylamide. The reaction is generally carried out at temperatures of about -70°C to about room temperature and require from about 1-24 hours. The product can be isolated by techniques well known in the art, such as extraction, evaporation, and precipitation and can be purified by chromatography and recrystallization.

In Reaction Scheme H, step 3, the compound of formula (18) the carboxy protecting group Pg₅ is selectively removed to give a compound of formula (3) in which Y is protected thio. Such deprotection of esters to acids in the presence of suitable thio protecting groups are well known and appreciated in the art.

The following examples and preparations present typical syntheses as described in the Reaction Schemes above. These examples and preparations are understood to be illustrative only and are not intended to limit the scope of the invention in any way.

### PREPARATION 1.1

### 2-(t-Butoxycarbonylamino)malonic acid

Combine diethyl aminomalonate hydrochloride (5.0 g, 23.6 mmol), triethylamine (3.30 mL, 23.6 mmol), and dichloromethane (80 mL). Add di-t-butyl dicarbonate (5.4 g, 24.8 mmol). After 18 hours, extract with an aqueous 5% sulfuric acid solution, a saturated aqueous sodium bicarbonate solution, and then brine. Dry the organic layer over Na₂SO₄, filter, and evaporate *in vacuo* to give a residue. Chromatograph the residue on silica gel eluting with 3/2 hexane/ethyl acetate to give diethyl t-butoxycarbonylaminomalonate (87%).
Combine diethyl t-butoxycarbonylaminomalonate (4.55 g, 16.5 mmol), a 6 M aqueous sodium hydroxide solution (6.65 mL, 40 mmol), and ethanol (30 mL). After 18 hours, evaporate *in vacuo* to remove most of the ethanol, dilute with water (50 mL) and extract with diethyl ether. Acidify to a pH of about 3 with aqueous 12M hydrochloric acid and extract twice with ethyl acetate. Dry the combined organic layers over Na₂SO₄, filter, and concentrate *in vacuo* to give the title compound (61%).

### PREPARATION 1.2

### 2-(t-Butoxycarbonylamino)malonic acid

Combine diethyl aminomalonate hydrochloride (13.34 g, 63.0 mmol), triethylamine (8.8 mL, 63.0 mmol), and dichloromethane (220 mL). Add di-t-butyl dicarbonate (14.4 g, 66.2 mmol). After 18 hours, extract with an aqueous 5% sulfuric acid solution, a saturated aquecus sodium bicarbonate solution, and brine. Dry the organic layer over Na₂SO₄, filter, and evaporate in *vacuo* to give diethyl t-butoxycarbonylaminomalonate (103%).
Combine diethyl t-butoxycarbonylaminomalonate (22.72 g, 82.5 mmol), a 6 M aqueous sodium hydroxide solution (55 mL, 330 mmol), and ethanol (150 mL). After 56 hours, evaporate *in vacuo* to remove most of the ethanol, dilute with water (40 mL). Cool and acidify to a pH of about 3 with concentrated hydrochloric acid. Freeze dry to give the title compound and sodium chloride as a powder.

### PREPARATION 2

### 2-(R)-2-Bromo-3-methylbutyric acid

Combine D-valine (4.7 g, 40.0 mmol) and an aqueous 2.5 M sulfuric acid solution. Cool to about -5°C. Add aqueous hydrobromic acid (13.2 g, 48%, 40 mmol). Add a solution of sodium nitrite (2.8 g, 40 mmol) in water (20 mL). After 2 hours, warm to about 5°C. After 18 hours, extract three times with ethyl acetate. Dry the combined organic layers over Na₂SO₄, filter, and evaporate *in vacuo* to give a residue. Chromatograph the residue on silica gel eluting with 1/1/0.1 dichloromethane/ethyl acetate/acetic acid to give a residue. Rechromatograph the residue on silica gel eluting with 1/1/0.5 dichloromethane/ethyl acetate/acetic acid to give the title compound (35%).

EXAMPLE 1

N,N'-Diphenethyl-2-((S)-2-mercapto-3-methylbutyrylamino)malonamide 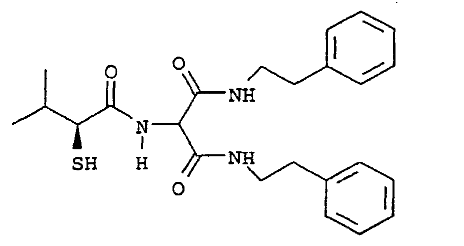 1.1 Synthesis of N,N'-diphenethyl-2-(t-butoxycarbonylamino)malonamide

Combine t-butoxycarbonylaminomalonic acid (0.465 g, 2.12 mmol), phenethylamine (0.715 mL, 5.70 mmol), and dichloromethane (25 mL). Add N-methylmorpholine (0.752 mL, 6.85 mmol). Cool in an ice bath. Add benzotriazol-1-yloxy-tris(pyrrolidino)phosphonium hexafluorophosphate complex (3.15 g, 6.85 mmol). Remove the ice bath and allow to warm to ambient temperature. After 18 hours, dilute the reaction mixture with ethyl acetate, extract with an aqueous 5% sulfuric acid solution, a saturated sodium bicarbonate solution, and then brine. Dry the organic layers over Na₂SO₄, filter, and evaporate *in vacuo* to give a residue. Chromatograph the residue on silica gel eluting with 2/1 ethyl acetate/hexane to give the title compound.

### 1.2 Synthesis of N,N'-diphenethyl-2-aminomalonamide trifluoroacetic acid salt

Combine N,N'-diphenethyl-2-(t-butoxycarbonylamino)malonamide (0.571 g, 1.35 mmol) and dichloromethane (10 mL). Add trifluoroacetic acid (1.8 mL). After 2.5 hours, evaporate *in vacuo* to give a residue. Add hexanes and carbon tetrachloride to co-evaporate residual trifluoroacetic acid and evaporate *in vacuo* to give the title compound (100%).

### 1.3 Synthesis of N,N'-Diphenethyl-2-((R)-2-bromo-3-methylbutyrylamino)malonamide

Combine N,N'-diphenethyl-2-aminomalonamide trifluoroacetic acid salt (0.285 g, 0.655 mmol) and dichloromethane (5 mL). Add (R)-2-bromo-3-methylbutyric acid (0.118 g, 0.655 mmol), N-methylmorpholine (0.10 mL. 0.983 mmol), and 1-ethyl-3-(3-(dimethylamino)propyl)carbodiimide hydrochloric acid salt (0.14 g, 0.725 mmol), and 1-hydroxybenztriazole hydrate (0.10 g, 0.72 mmol). After 18 hours, evaporate the reaction mixture *in vacuo*, dilute the concentrated reaction mixture with ethyl acetate, extract with an aqueous 5% sulfuric acid solution, a saturated sodium bicarbonate solution, and then brine.
Saturate each of the aqueous layers with sodium chloride and extract four times with ethyl acetate. Combine the organic layers, dry over Na₂SO₄, filter, and evaporate *in vacuo* to give a residue. Chromatograph the residue on silica gel eluting with 1/1 ethyl acetate/hexane to give the title compound (67%).

### 1,4 Synthesis of N,N'-diphenethyl-2-((S)-2-thioacetyl-3-methylbutyrylamino)malonamide

Combine N,N'-diphenethyl-2-((R)-2-bromo-3-methylbutyrylamino)malonamide (0.20 g, 0.41 mmol), thioacetic acid (0.08 mL, 1.1 mmol), and dimethylformamide (8 mL). Degas by repeated cycles of vacuum and filling with nitrogen gas. Add cesium carbonate (0.215 g, 0.664 mmol). After 18 hours, dilute with water and extract with ethyl acetate. Extract the organic layer with brine, dry over Na₂SO₄, filter, and evaporate *in vacuo* to give a residue. Chromatograph the residue on silica gel eluting with 1/1 hexane ethyl acetate to give the title compound.

### 1,5 Synthesis of N,N'-diphenethyl-2-((S)-2-mercapto-3-methylbutyrylamino)malonamide

Cool methanol (10 mL) in an ice bath and bubble with ammonia gas for about 15 minutes. Add a cooled (about 0°C) solution of N,N'-diphenethyl-2-((S)-2-thioacetyl-3-methylbutyrylamino)malonamide (0.17 g, 0.35 mmol) in degassed methanol (10 mL). After 1 hour, evaporate *in vacuo* to give a residue. Chromatograph the residue on silica gel eluting sequentially with 3/2 hexane ethyl acetate and then 1/1 hexane ethyl acetate give the title compound (89%).

### EXAMPLE 2

### N,N'-Diphenethyl-2-((S)-2-mercapto-3-phenylpropionylamino)malonamide

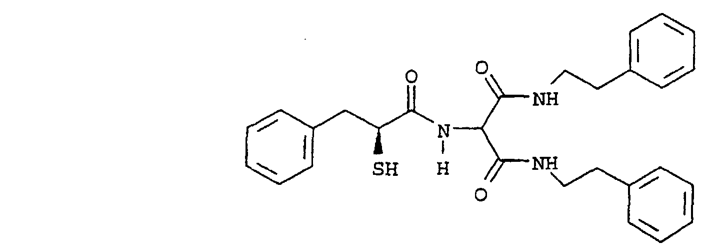

### 2.1 Synthesis of N,N'-Diphenethyl-2-((R)-2-bromo-3-phenylpropionylamino)malonamide

Prepare by the method of Example 1.3 using N,N'-diphenethyl-2-aminomalonamide trifluoroacetic acid salt (0.61 g, 0.600 mmol). (R)-2-bromo-3-phenylpropionic acid (0.137 g, 0.600 mmol), N-methylmorpholine (0.20 mL, 1.8 mmol), 1-ethyl-3-(3-(dimethylamino) propyl)carbodiimide hydrochloric acid salt (0.14 g, 0.72 mmol), and 1-hydroxybenztriazole hydrate (0.097 g, 0.72 mmol) in dichloromethane (10 mL). Purify by chromatography on silica gel eluting with 1/1 ethyl acetate/hexane to give the title compound (60%).

### 2.2 Synthesis of N,N'-diphenethyl-2-((S)-2-thioacetyl-3-phenylpropionylamino)malonamide

Prepare by the method of Example 1.4 using N.N'-diphenethyl-2-((R)-2-bromo-3-phenylpropionylamino)malonamide (0.11 g, 0.21 mmol), thioacetic acid (0.05 mL, 0.62 mmol), and cesium carbonate (0.10 g, 0.31 mmol) in dimethylformamide (8 mL). Purify by chromatography on silica gel eluting with 1/1 hexane ethyl acetate to give the title compound (92%).

### 2.3 Synthesis ofN.N'-diphenethyl-2-((S)-2-mercapto-3-phenylpropionylamino)malonamide

Prepare by the method of Example 1.5 using N,N'-diphenethyl-2-((S)-2-thioacetyl-3-phenylpropionylamino)malonamide (0.10 g, 0.192 mmol). Purify by chromatography on silica gel eluting with 1/1 hexane ethyl acetate give the title compound (83%).

### PREPARATION 3

### 2-(S)-2-Thioacetyl-4-pheynylbutyric acid

Combine D-homophenylalanine (5.0 g, 28.0 mmol) and an aqueous 2.5 M sulfuric acid solution (28 mL). Cool to about -5°C. Add aqueous hydrobromic acid (6.35 mL, 48%, 56 mmol). Add a solution of sodium nitrite (1.95 g, 28 mmol) in water (15 mL) over about 30 minutes. After 2 hours, warm to about 0°C. After 18 hours, extract three times with ethyl acetate. Dry the combined organic layers over Na₂SO₄, filter, and evaporate *in vacuo* to give a residue. Chromatograph the residue on silica gel eluting with 9/1/0.5 dichloromethane/ethyl acetate/acetic acid to give 2-(R)-2-bromo-4-pheynylbutyric acid (54%).
Combine 2-(R)-2-bromo-4-pheynylbutyric acid (0.486 g, 2.05 mmol), thioacetic acid (0.40 mL, 5.5 mmol), and dimethylformamide (40 mL). Degas by repeated cycles of vacuum and filling with nitrogen gas. Add cesium carbonate (1.08 g, .32 mmol). After 18 hours, dilute with water and extract with ethyl acetate. Extract the organic layer with brine, dry over Na₂SO₄, filter, and evaporate *in vacuo* to give the title compound.

### EXAMPLE 3

### N,N'-Diphenethyl-2-((S)-2-mercapto-4-phenylbutyrylamino)malonamide

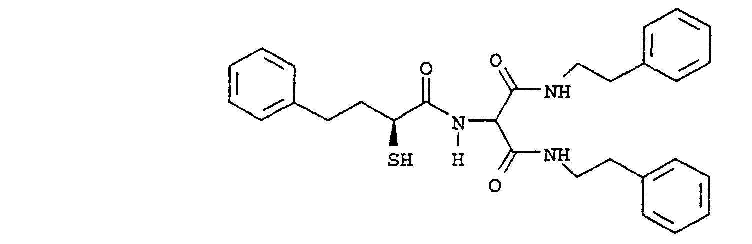

### 3.1 Synthesis of N.N'-diphenethyl-2-(t-butoxycarbonylamino)malonamide

Combine a mixture of t-butoxycarbonylaminomalonic acid and sodium chloride from Preparation 1.2 (5.0 g, 13.7 mmol), phenethylamine (4.32 mL, 34.3 mmol), and tetrahydrofuran (25 mL). Cool in an ice bath. Remove the ice bath. Add N-methylmorpholine (3.8 mL, 34.3 mmol), 1-ethyl-3-(3-(dimethylamino)propyl)carbodiimide hydrochloric acid salt (6.58 g, 34.3 mmol), and 1-hydroxybenztriazole hydrate (4.63 g, 34.3 mmol). Allow to warm to ambient temperature. After 18 hours, dilute the reaction mixture with ethyl acetate, extract with an aqueous 5% sulfuric acid solution, a saturated sodium bicarbonate solution, and then brine. Dry the organic layers over Na₂SO₄, filter, and evaporate *in vacuo* to give a residue. Chromatograph the residue on silica gel eluting with 2/1 ethyl acetate/hexane to give the title compound (84%).

### 3.2 Synthesis of N,N'-diphenethyl-2-aminomalonamide trifluoroacetic acid salt

Combine N,N'-diphenethyl-2-(t-butoxycarbonylamino)malonamide (0.425 g, 1.00 mmol) and dichloromethane (7 mL). Add trifluoroacetic acid (1.5 mL). After 2.5 hours, evaporate *in vacuo* to give a residue. Repeatedly, add carbon tetrachloride to co-evaporate residual trifluoroacetic acid and evaporate *in vacuo* to give the title compound.

### 3,3 Synthesis of N,N'-diphenethyl-2-((S)-2-thioacetyl-4-phenylbutyrylamino)malonamide

Combine N,N'-diphenethyl-2-aminomalonamide trifluoroacetic acid salt (0.50 g, 1.0 mmol) and dichloromethane (15 mL). Add (R)-2-thioacetyl-4-phenylbutyric acid (0.545 g, 1.0 mmol), N-methylmorpholine (0.22 mL, 2.0 mmol), and 1-ethyl-3-(3-(dimethylamino)propyl)carbodiimide hydrochloric acid salt (0.23 g, 1.2 mmol), and 1-hydroxybenztriazole hydrate (0.16 g, 1.2 mmol). After 18 hours, evaporate the reaction mixture *in vacuo*, dilute the concentrated reaction mixture with ethyl acetate, extract with an aqueous 5% sulfuric acid solution, a saturated sodium bicarbonate solution, and then brine. Extract each aqueous layer with ethyl acetate. Combine the organic layers, dry over Na₂SO₄, filter, and evaporate *in vacuo* to give a residue. Chromatograph the residue on silica gel eluting with 3/2 ethyl acetate/hexane to give the title compound (92%).

### 3.4 Synthesis of N,N'-diphenethyl-2-((S)-2-mercapto-4-phenylbutyrylamino)malonamide

Cool methanol (15 mL) in an ice bath and bubble with ammonia gas for about 15 minutes. Add a cooled (about 0°C) solution of N,N'-diphenethyl-2-((S)-2-thioacetyl-4-phenylbutyrylamino)malonamide (0.50 g, 0.92 mmol) in degassed methanol (15 mL). After 1 hour, evaporate *in vacuo* to give a residue. Chromatograph the residue on silica gel eluting with 3/2 hexane ethyl acetate to give the title compound (28%).

### EXAMPLE 4

### N,N'-Dibenzyl-2-((S)-2-mercapto-3-phenylpropionylamino)malonamide

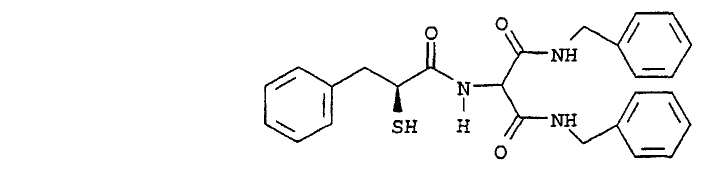

### 4.1 Synthesis of N,N'-dibenzyl-2-(t-butoxycarbonylamino)malonamide

Combine t-butoxycarbonylaminomalonic acid (0.438 g, 2.00 mmol), benzylamine (0.436 mL, 4.0 mmol), and dichloromethane (10 mL). Add N-methylmorpholine (0.55 mL, 5.00 mmol)), 1-ethyl-3-(3-(dimethylamino) propyl)carbodiimide hydrochloric acid salt (0.96 g, 5.0 mmol), and 1-hydroxybenztriazole hydrate (0.67 g, 5.0 mmol). After 18 hours, concentrate *in vacuo*, dilute the concentrated reaction mixture with ethyl acetate, extract with an aqueous 5% sulfuric acid solution, a saturated sodium bicarbonate solution, and then brine. Dry the organic layer over Na₂SO₄, filter, and evaporate *in vacuo* to give a residue. Chromatograph the residue on silica gel eluting with 3/2 ethyl acetate/hexane to give the title compound (52%).

### 4.2 Synthesis of N,N'-dibenzyl-2-aminomalonamide trifluoroacetic acid salt

Combine N,N'-dibenzyl-2-(t-butoxycarbonylamino)malonamide (0.415 g, 1.04 mmol) and dichloromethane (7 mL). Add trifluoroacetic acid (1.0 mL). After 2.5 hours, evaporate *in vacuo* dry under high vacuum to give the title compound.

### 4.3 Synthesis of N,N'-dibenzyl-2-((R)-2-bromo-3-phenylpropionylamino)malonamide

Combine N,N'-dibenzyl-2-aminomalonamide trifluoroacetic acid salt (0.41 g, 1.04 mmol), (R)-2-bromo-3-phenylpropionic acid (0.262 g, 1.15 mmol), N-methylmorpholine (0.23 mL, 2.08 mmol), 1-ethyl-3-(3-(dimethylamino) propyl)carbodiimide hydrochloric acid salt (0.22 g, 1.15 mmol), and 1-hydroxybenztriazole hydrate (0.155 g, 1.15 mmol) in dichloromethane (5 mL). After 18 hours, extract with an aqueous 5% sulfuric acid solution, a saturated sodium bicarbonate solution, and then brine. Dry the organic layer over Na₂SO₄, filter, and evaporate *in vacuo* to give a residue. Chromatograph the residue on silica gel eluting with 1/1 ethyl acetate/hexane to give the title compound.

### 4.4 Synthesis of N,N'-dibenzyl-2-((S)-2-thioacetyl-3-phenylpropionylamino)malonamide

Combine N,N'-dibenzyl-2-((R)-2-bromo-3-phenylpropionylamino)malonamide (0.49 g, 0.97 mmol) and thioacetic acid (0.172 mL, 2.42 mmol) in dimethylformamide (15 mL). Degas by repeated cycles of vacuum and filing with nitrogen. Add cesium carbonate (0.473 g, 1.45 mmol). After 18 hours, dilute the reaction mixture with water and extract twice with ethyl acetate. Combine the organic layers, extract with brine, dry over Na₂SO₄, filter, and evaporate *in vacuo* to give a residue. Chromatograph the residue on silica gel eluting with 3/2 ethyl acetate/hexane to give the title compound (46%).

### 4.5 Synthesis ofN.N'-dibenzyl-2-((S)-2-mercapto-3-phenylpropionylamino)malonamide

Cool methanol (12 mL) in an ice bath and bubble with ammonia gas for about 15 minutes. Add a cooled (about 0°C) solution of N,N'-dibenzyl-2-((S)-2-thioacetyl-3-phenylpropionylamino)malonamide (0.224 g, 0.445 mmol) in degassed methanol (12 mL). After 1 hour, evaporate *in vacuo* to give a residue. Chromatograph the residue on silica gel eluting with 3/2 ethyl acetate/hexane give the title compound.

### EXAMPLE 5

### N,N'-Di-(3-phenylpropyl)-2-((S)-2-mercapto-3-phenylpropionylamino)malonamide

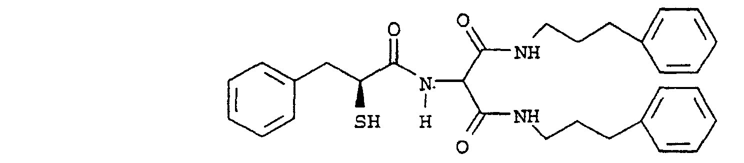

### 5.1 Synthesis ofN.N'-di-(3-phenylpropyl)-2-(t-butoxycarbonylamino)malonamide

Prepare by the method of Example 4.1 using 3-phenylpropylamine (0.54 mL, 4.0 mmol). Add Purify by chromatography on silica gel eluting with 2/1 ethyl acetate/hexane to give the title compound (27%).

### 5.2 Synthesis of N,N'-di-(3-phenylpropyl)-2-aminomalonamide trifluoroacetic acid salt

Prepare by the method of Example 4.2 using N,N'-di-(3-phenylpropyl)-2-(t-butoxycarbonylamino)malonamide (0.24 g, 0.53 mmol) to give, after evaporation *in vacuo* and drying under high vacuum, the title compound.

### 5.3 Synthesis of N,N'-di-(3-phenylpropyl)-2-((R)-2-bromo-3 phenylpropionylamino)malonamide

Prepare by the method of Example 4.3 using N,N'-di-(3-phenylpropyl)-2-aminomalonamide trifluoroacetic acid salt. Purify by chromatography on silica gel eluting with 1/1 ethyl acetate/hexane to give the title compound.

### 5.4 Synthesis of N,N'-di-(3-phenylpropyl)-2-((S)-2-thioacetyl-3-phenylpropionylamino)malonamide

Prepare by the method of Example 4.4 using N,N'-di-(3-phenylpropyl)-2-((R)-2-bromo-3-phenylpropionylamino)malonamide (0.124 g, 0.575 mmol). Purify by chromatography on silica gel eluting with 3/2 ethyl acetate/hexane to give the title compound.

### 5.5 Synthesis of N,N'-di-(3-phenylpropyl)-2-((S)-2-mercapto-3-phenylpropionylamino)malonamide

Prepare by the method of Example 4.5 using N,N'-di-(3-phenylpropyl)-2-((S)-2-thioacetyl-3-phenylpropionylamino)malonamide (0.156 g, 0.289 mmol). After 1 hour, evaporate *in vacuo* to give a residue. Purify by chromatography on silica gel eluting with 5% acetone/dichloromethane to give the title compound.

### EXAMPLE 6

### N,N'-Di-(4-methoxyphenethyl)-2-((S)-2-mercapto-3-phenylpropionylamino)malonamide;

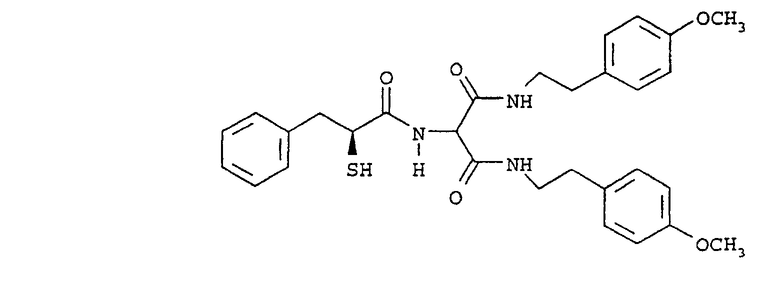

### 6.1 Synthesis of N,N'-di-(4-methoxyphenethyl)-2-(t-butoxycarbonylamino)malonamide

Prepare by the method of Example 4.1 using 4-methoxyphenethylamine (0.585 mL, 3.8 mmol). Add Purify by chromatography on silica gel eluting with 2/1 ethyl acetate/hexane to give the title compound (27%).

### 6.2 Synthesis of N,N'-di-(4-methoxyphenethyl)-2-aminomalonamide trifluoroacetic acid salt

Prepare by the method of Example 4.2 using N,N'-di-(4-methoxyphenethyl)-2-(t-butoxycarbonylamino)malonamide (0.20 g, 0.412 mmol) to give, after evaporation in *vacuo* and drying under high vacuum, the title compound.

### 6.3 Synthesis of N,N'-di-(4-methoxyphenethyl)-2-((R)-2-bromo-3-phenylpropionylamino)malonamide

Prepare by the method of Example 4.3 using N,N'-di-(4-methoxyphenethyl)-2-aminomalonamide trifluoroacetic acid salt. Purify by chromatography on silica gel eluting with 3/2 ethyl acetate/hexane to give the title compound.

### 6,4 Synthesis of N,N'-di-(4-methoxyphenethyl)-2-((S)-2-thioacetyl-3-phenylpropionylamino)malonamide

Prepare by the method of Example 4.4 using N,N'-di-(4-methoxyphenethyl)-2-((R)-2-bromo-3-phenylpropionylamino)malonamide (0.133 g, 0.223 mmol). Purify by chromatography on silica gel eluting with 3/2 ethyl acetate/hexane to give the title compound (54%).

### 6.5 Synthesis of N.N'-di-(4-methoxyphenethyl)-2-((S)-2-mercapto-3-phenylpropionylamino)malonamide

Prepare by the method of Example 4.5 using N,N'-di-(4-methoxyphenethyl)-2-((S)-2-thioacetyl-3-phenylpropionylamino)malonamide (0.071 g, 0.12 mmol). After 1 hour, evaporate *in vacuo* to give a residue. Chromatograph the residue on silica gel eluting with 1/1 ethyl acetate/hexane give the title compound.

### EXAMPLE 7

### N.N'-Dipentyl-2-(2-mercapto-3-phenylpropionylamino)malonamide

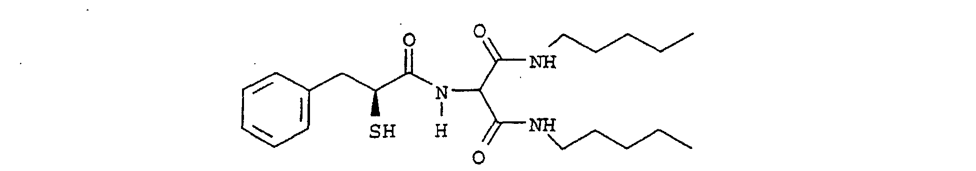

### 7.1 Synthesis of N,N'-dipentyl-2-(t-butoxycarbonylamino)malonamide

Prepare by the method of Example 4.1 using pentylamine (1.50 mL, 12.8 mmol). Purify by chromatography on silica gel eluting sequentially with 1/1 ethyl acetate/hexane, 3/1 ethyl acetate/hexane, and then 5/1 ethyl acetate/hexane to give the title compound (68%).

### 7.2 Synthesis of N,N'-dipentyl-2-aminomalonamide trifluoroacetic acid salt

Combine N,N'-dipentyl-2-(t-butoxycarbonylamino)malonamide (1.49 g, 4.17 mmol). After 1.5 hours, evaporate *in vacuo* and triturate with carbon tetrachloride to give the title compound.

### 7.3 Synthesis of N,N'-dipentyl-2-((R)-2-bromo-3-phenylpropionylamino)malonamide

Prepare by the method of Example 4.3 using N,N'-dipentyl-2-aminomalonamide trifluoroacetic acid salt. Purify by chromatography on silica gel eluting with 3/2 ethyl acetate/hexane to give the title compound.

### 7.4 Synthesis of N,N'-dipentyl-2-((S)-2-thioacetyl-3-phenylpropionylamino)malonamide

Prepare by the method of Example 4.4 using N,N'-dipentyl-2-((R)-2-bromo-3-phenylpropionylamino)malonamide (0.45 g, 0.961 mmol). Purify by chromatography on silica gel eluting with 3/2 ethyl acetate/hexane to give the title compound (62%).

### 7.5 Synthesis of N,N'-dipentyl-2-((S)-2-mercapto-3-phenylpropionylamino)malonamide

Prepare by the method of Example 4.5 using N,N'-dipentyl-2-((S)-2-thioacetyl-3-phenylpropionylamino)malonamide (0.277 g, 0.60 mmol). Purify by chromatograph on silica gel eluting sequentially with 5% acetone/dichloromethane and then 10% acetone/dichloromethane to give the title compound.

### EXAMPLE 8

### N,N'-Di-(2-(N-anilino)ethyl)-2-(2-mercapto-3-phenylpropionylamino)malonamide

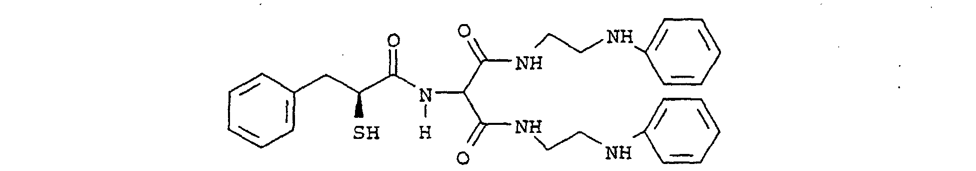

### 8.1 Synthesis of N,N'-di-(2-(N-anilino)ethyl)-2-(t-butoxycarbonylamino)malonamide

Combine t-butoxycarbonylaminomalonic acid (0.730 g, 2.00 mmol), 2-(N-anilino)ethylamine (0.654 mL, 5.00 mmol) N-methylmorpholine (0.55 mL, 5.0 mmol)), 1-ethyl-3-(3-(dimethylamino)propyl)carbodiimide hydrochloric acid salt (0.958 g, 5.0 mmol), and 1-hydroxybenztriazole hydrate (0.67 g, 5.00 mmol) in dichloromethane (10 mL) and tetrahydrofuran (1 mL). After 18 hours, concentrate *in vacuo*, dilute the concentrated reaction mixture with ethyl acetate and extract with a saturated aqueous sodium bicarbonate solution and then brine. Extract each of the aqueous layers with ethyl acetate. Combine the organic layers, dry over Na₂SO₄, filter, and evaporate *in vacuo* to give a residue. Combine the residue, methanol (10 mL), and celite and apply to a silica gel column. Chromatograph eluting with 3/2 ethyl acetate/hexane to give the title compound (60%).

### 8.2 Synthesis of N,N'-di-(2-(N-anilino)ethyl)-2-aminomalonamide hydrochloric acid salt

Combine N,N'-di-(2-(N-anilino)ethyl)-2-(t-butoxycarbonylamino)malonamide (0.55 g, 1.21 mmol) and tetrahydrofuran (7 mL) and diethyl ether (7 mL). Add a solution of hydrochloric acid in diethyl ether (10 mL, 1 M, 10 mmol) to give a solid, After 45 minutes, collect the solid by filtration to give the title compound.

### 8.3 Synthesis of N,N'-di-(2-(N-anilino)ethyl)-2-((R)-2-bromo-3-phenylpropionylamino)malonamide

Combine N,N'-di-(2-(N-anilino)ethyl)-2-aminomalonamide hydrochloric acid salt (0.474 g, 0.866 mmol) and dichloromethane (10 mL). Add (R)-2-bromo-3-phenylpropionic acid (0.26 g, 1.13 mmol). Add triethylamine (0.426 mL, 3.06 mmol). Add pyridine benzotriazol-1-yloxytris(dimethylamino)phosphonium hexafluorophosphate complex (0.478 g, 1.13 mmol). After 18 hours, evaporate *in vacuo* and dilute the concentrated reaction mixture with ethyl acetate, extract with an aqueous 5% sulfuric acid solution, a saturated sodium bicarbonate solution, and then brine. Extract each of the aqueous layers with ethyl acetate. Combine the organic layers and dry over Na₂SO₄, filter, and evaporate *in vacuo* to give a residue. Chromatograph the residue on silica gel eluting sequentially with 1/1 ethyl acetate/hexane and then 2/1 ethyl acetate/hexane to give the title compound (62%).

### 8.4 Synthesis of N,N'-di-(2-(N-anilino)ethyl)-2-((S)-2-thioacetyl-3-phenylpropionylamino)malonamide

Prepare by the method of Example 4.4 using N,N'-di-(2-(N-anilino)ethyl)-2-((R)-2-bromo-3-phenylpropionylamino)malonamide (0.311 g, 0.549 mmol) and thioacetic acid ( 0.08 mL, 0.010 mmol) in dimethylformamide (8 mL), and cesium carbonate (0.18 g, 0.549 mmol). Purify by chromatography on silica gel eluting sequentially with 1/1 ethyl acetate/hexane, 2/1 ethyl acetate/hexane, and then 3/1 ethyl acetate/hexane to give the title compound.

### 8.5 Synthesis of N,N'-di-(2-(N-anilino)ethyl)-2-((S)-2-mercapto-3-phenylpropionylamino)malonamide

Prepare by the method of Example 4.5 using methanol (10 mL) and N.N'-di-(2-(N-anilino)ethyl)-2-((S)-2-thioacetyl-3-phenylpropionylamino)malonamide (0.151 g, 0.269 mmol) in degassed methanol (10 mL) and tetrahydrofuran (7 mL). After 2.5 hour, evaporate *in vacuo* to give a residue. Chromatograph the residue on silica gel eluting sequentially with 3/2 ethyl acetate/hexane and then 2/1 ethyl acetate/hexane to give the title compound.

### PREPARATION 4

### 2-(S)-2-Thioacetyl-3-pheynylproionic acid

Combine 2-(R)-2-bromo-3-phenylpropionic acid (2.85 g, 12.4 mmol), thioacetic acid (1.4 mL, 19.5 mmol), and dimethylformamide (30 mL). Degas by repeated cycles of vacuum and filling with nitrogen gas. Add cesium carbonate (4.25 g, 12.4 mmol). After 18 hours, add with an aqueous 5% sulfuric acid solution, dilute with water, and extract four times with ethyl acetate. Extract the combined organic layers with brine, dry over Na₂SO₄, filter, and evaporate *in vacuo* to give a residue. Chromatograph the residue on silica gel eluting sequentially with 1/1/0.02 hexane/ethyl acetate/acetic acid and then 1/1/0.02 hexane/ethyl acetate/acetic acid to give the title compound (43%).

### EXAMPLE 9

### N,N'-Di-(pydrid-4-ylethyl)-2-(2-mercapto-3-phenylpropionylamino)malonamide

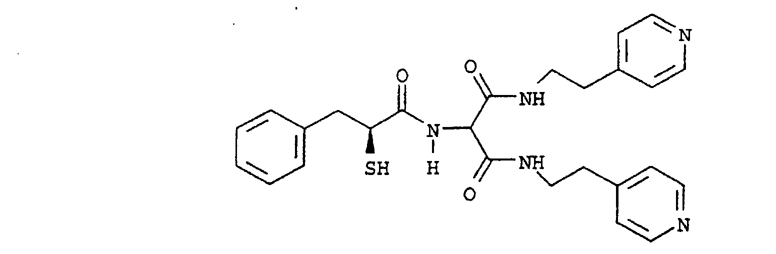

### 9.1 Synthesis of N,N'-di-(pydrid-4-ylethyl)-2-(t-butoxycarbonylamino)malonamide

Combine t-butoxycarbonylaminomalonic acid (0.438 g, 2.0 mmol), 2-(pyrid-4-yl)ethylamine (0.61 g, 5.0 mmol), N-methylmorpholine (0.55 mL, 5.0 mmol)), 1-ethyl-3-(3-(dimethylamino)propyl)carbodiimide hydrochloric acid salt (0.96 g, 5.0 mmol), and 1-hydroxybenztriazole hydrate (0.68 g, 5.0 mmol) in dichloromethane (10 mL) and tetrahydrofuran (1 mL). After 18 hours, concentrate *in vacuo*, dilute the concentrated reaction mixture with ethyl acetate, and extract with a saturated aqueous sodium bicarbonate solution and then brine. Extract each of the aqueous layers with ethyl acetate. Combine the organic layers, dry over Na₂SO₄, filter, and evaporate *in vacuo* to give a residue. Chromatograph the residue on silica gel eluting with 10% methanol/dichloromethane to give the title compound (80%).

### 9.2 Synthesis of N,N'-di-(pydrid-4-ylethyl)-2-aminomalonamide hydrochloric acid salt

Combine N,N'-di-(pydrid-4-ylethyl)-2-(t-butoxycarbonylamino)malonamide (0.686 g, 1.61 mmol) and diethyl ether (20 mL). Add a solution of hydrochloric acid in diethyl ether (10 mL, 1 M, 10 mmol). After 1 hour, evaporate *in vacuo* to give the title compound.

### 9.3 Synthesis of N,N'-di-(pydrid-4-ylethyl)-2-((R)-2-thioacetyl-3-phenylpropionylamino)malonamide

Combine N,N'-di-(pydrid-4-ylethyl)-2-aminomalonamide hydrochloric acid salt (0.669 g, 1.63 mmol) and dichloromethane (10 mL), tetrahydrofuran (5 mL), and dimethylformamide (3 mL). Add a solution of (S)-2-thioacetyl-3-phenylpropionic acid (0.43 g, 1.9 mmol) in dichloromethane (1.9 mL). Add N-methylmorpholine (0.357 mL, 4.90 mmol), 1-ethyl-3-(3-(dimethylamino)propyl)carbodiimide hydrochloric acid salt (0.343 g, 1.8 mmol), and 1-hydroxybenztriazole hydrate (0.242 g, 0.18 mmol). After 18 hours, dilute the reaction mixture with ethyl acetate, extract with a saturated aqueous sodium bicarbonate solution, and then brine to give a residue. Chromatograph the residue on silica gel eluting sequentially with 10% methanol/dichloromethane and then 15% methanol/dichloromethane to give the title compound (63%).

### 9.4 Synthesis of N,N'-di-(pydrid-4-ylethyl)-2-((S)-2-mercapto-3-phenylpropionylamino)malonamide

Prepare by the method of Example 4.5 using methanol (15 mL) and N,N'-di-(pydrid-4-ylethyl)-2-((S)-2-thioacetyl-3-phenylpropionylamino)malonamide (0.543 g, 1.02 mmol) in degassed methanol (15 mL). After 1.5 hours, evaporate *in vacuo* to give a residue. Chromatograph the residue on silica gel eluting with 1/10 methanol/dichloromethane to give the title compound.

### EXAMPLE 10

### N,N'-Diphenethyl-2-((S)-2-mercaptopropionylamino)malonamide

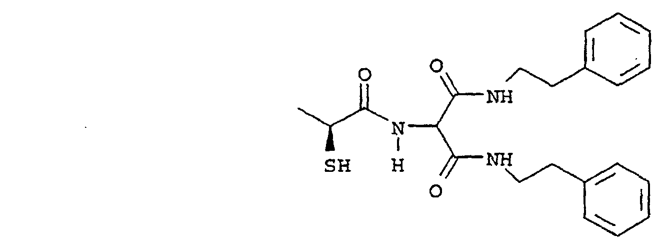

### 10.1 Synthesis of N,N'-diphenethyl-2-((R)-2-bromopropionylamino)malonamide

Prepare by the method of Example 4.3 using (R)-2-bromopropionic acid (0.109 mL, 1.2 mmol). Purify by chromatography on silica gel eluting with 3/2 ethyl acetate/hexane to give the title compound.

### 10.2 Synthesis of N,N'-diphenethyl-2-((S)-2-thioacetylpropionylamino)malonamide

Prepare by the method of Example 4.4 using N.N'-diphenethyl-2-((R)-2-bromopropionylamino)malonamide (0.175 g, 0.38 mmol). Purify by chromatography on silica gel eluting with 3/2 ethyl acetate/hexane to give the title compound (76%).

### 10.3 Synthesis of N,N'-diphenethyl-2-((S)-2-mercaptopropionylamino)malonamide

Prepare by the method of Example 1.5 using N,N'-diphenethyl-2-((R)-2-thioacetylpropionylamino)malonamide (0.131 g, 0.29 mmol). Purify by chromatography on silica gel eluting with 3/2 ethyl acetate/hexane to give the title compound (98%).

### EXAMPLE 11

### N,N'-Diphenethyl-2-(2-mercaptopropionylamino)malonamide

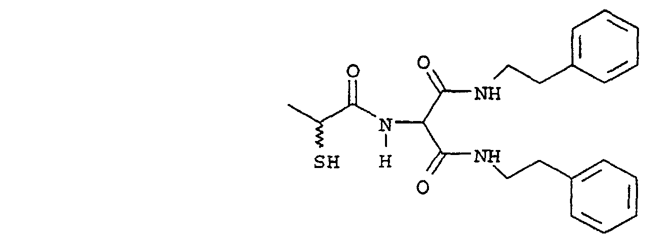

### 11.1 Synthesis of N,N'-diphenethyl-2-(2-bromopropionylamino)malonamide

Prepare by the method of Example 4.3 using 2-bromopropionic acid (0.108 mL, 1.2 mmol). Purify by chromatography on silica gel eluting with 3/2 ethyl acetate/hexane to give the title compound.

### 11.2 Synthesis of N,N'-diphenethyl-2-(2-thioacetylpropionylamino)malonamide

Prepare by the method of Example 4.4 using N,N'-diphenethyl-2-(2-bromopropionylamino)malonamide (0.152 g, 0.33 mmol). Purify by chromatography on silica gel eluting with 3/2 ethyl acetate/hexane to give the title compound (96%).

### 11.3 Synthesis of N,N'-diphenethyl-2-(2-mercaptopropionylamino)malonamide

Prepare by the method of Example 1.5 using N.N'-diphenethyl-2-(2-thioacetylpropionylamino)malonamide (0.131 g, 0.29 mmol). Purify by chromatography on silica gel eluting with 3/2 ethyl acetate/hexane to give the title compound (100%).

### EXAMPLE 12

### N,N'-Diphenethyl-2-(2-mercaptopentanoylamino)malonamide

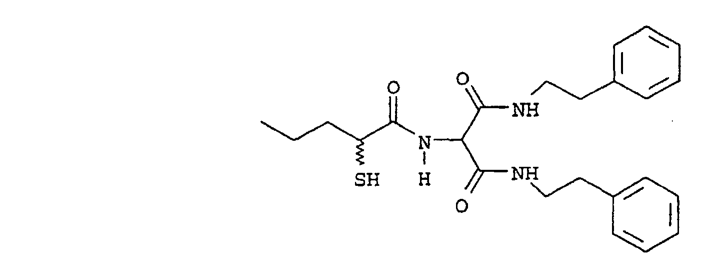

### 12.1 Synthesis of N,N'-diphenethyl-2-(2-bromopentanoylamino)malonamide

Prepare by the method of Example 4.3 using 2-bromopentanoic acid (0.16 mL, 1.2 mmol). Purify by chromatography on silica gel eluting with 1/1 ethyl acetate/hexane to give the title compound.

### 12.2 Synthesis of N,N'-diphenethyl-2-(2-thioacetylpentanoylamino)malonamide

Prepare by the method of Example 4.4 using N,N'-diphenethyl-2-(2-bromopentanoylamino)malonamide (0.162 g, 0.33 mmol). Purify by chromatography on silica gel eluting with 3/2 ethyl acetate/hexane to give the title compound (96%).

### 12.3 Synthesis of N.N'-diphenethyl-2-(2-mercaptopentanoylamino)malonamide

Prepare by the method of Example 1.5 using N,N'-diphenethyl-2-(2-thioacetylpentanoylamino)malonamide (0.131 g, 0.29 mmol). Purify by chromatography on silica gel eluting with sequentially with 2/3 ethyl acetate/hexane and then 1/1 ethyl acetate/hexane to give the title compound.

### PREPARATION 5

### 2-((S)-2-(p-methoxybenzylmercapto)-3-phenylpropionylamino)malonic acid

Combine (R)-2-bromo-3-phenylpropionic acid (3.44 g, 15 mmol), diethyl aminomalonate hydrochloride (4.23 g. 20 mmol), and N-methylmorpholine (5.5 mL, 50 mmol) in dichloromethane (50 mL). Add 1-ethyl-3-(3-(dimethylamino)propyl)carbodiimide hydrochloric acid salt (3.83 g, 20 mmol) and 1-hydroxybenztriazole hydrate (2.70 g, 20 mmol). Add tetrahydrofuran (10 mL). After 20 hours, concentrate *in vacuo* and partition the concentrated reaction mixture between an aqueous 5% sulfuric acid solution and methyl t-butyl ether. Separate the layers and extract the organic layer with a saturated aqueous sodium bicarbonate solution and then brine. Dry the organic layer over Na₂SO₄, filter, and evaporate *in vacuo* to give a residue. Chromatograph the residue on silica gel eluting sequentially with 1/4 ethyl acetate/hexane, 1/3 ethyl acetate/hexane, and then 2.5/1 ethyl acetate/hexane to give diethyl 2-((R)-2-bromo-3-phenylpropionylamino)malonate.
Combine diethyl 2-((R)-2-bromo-3-phenylpropionylamino)malonate (3.15 g, 8.16 mmol) and 4-methyoxybenzylmercaptan (2.6 mL, 19 mmol), and dimethylformamide (25 mL). Degas by repeatedly applying vacuum and filling the vessel with nitrogen. Add cesium carbonate (2.94 g, 9.9 mmol). After 16 hours, partition the reaction mixture between water and diethyl ether. Extract the organic layer with brine, dry over Na₂SO₄, filter, and evaporate *in vacuo* to give a residue. Chromatograph the residue on silica gel eluting sequentially with 3/1 hexane/ethyl acetate and then 2/1 hexane/ethyl acetate to give diethyl 2-((S)-2-(p-methoxybenzymercapto)-3-phenylpropionylamino)malonate.
Combine diethyl 2-((S)-2-(p-methoxybenzymercapto)-3-phenylpropionylamino) malonate (3.15 g, 6.85 mmol) and an aqueous 6 M sodium hydroxide solution (4.6 mL, 28 mmol) in ethanol (15 mL). After 24 hours, concentrate *in vacuo*, dilute the concentrated reaction mixture with water, adjust the pH to about 2.5 using an aqueous 6 M hydrochloric acid solution, and lyophilize to give the title compound and sodium chloride.

### EXAMPLE 13

### N,N'-Di-(4-chlorophenethyl)-2-(2-mercapto-3-phenylpropionylamino)malonamide

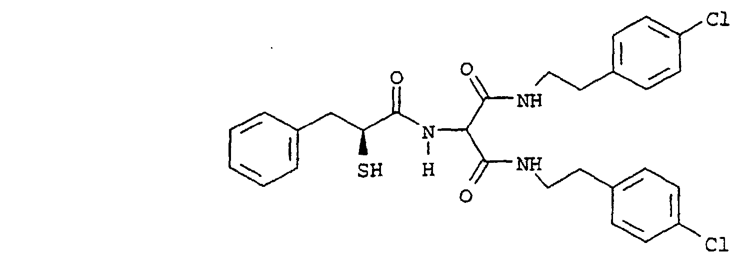

### 13.1 Synthesis of N,N'-di-(4-chlorophenethyl)-2-((S)-2-(p-methoxybenzylmercapto)-3-phenylpropionylamino)malonamide

Combine 2-((S)-2-(p-methoxybenzylmercapto)-3-phenylpropionylamino)malonic acid (prepared by the method of Preparation 5, 0.476 g, 63% acid/37% sodium chloride, 0.74 mmol), dichloromethane (9 mL), and tetrahydrofuran (1.5 mL). Add 4-chlorophenylethylamine (0.31 mL, 2.23 mmol), N-methylmorpholine (0.25 mL, 2.3 mmol), 1-ethyl-3-(3-(dimethylamino)propyl)carbodiimide hydrochloric acid salt (0.427 g, 2.23 mmol) and 1-hydroxybenztriazole hydrate (0.301 g, 2.23 mmol). After 3 days, concentrate *in vacuo* and partition the concentrated reaction mixture between an aqueous 5% sulfuric acid solution and ethyl acetate. Separate the layers and extract the organic layer with a saturated aqueous sodium bicarbonate solution and then brine. Dry the organic layer over Na₂SO₄, filter, and evaporate *in vacuo* to give a residue. Chromatograph the residue on silica gel eluting sequentially with 2/3 ethyl acetate/hexane, 1/1 ethyl acetate/hexane, and then 1/2 ethyl acetate/hexane to give the title compound.

### 13.2 Synthesis ofN.N'-di-(4-chlorophenethyl)-2-(2-mercapto-3-phenylpropionylamino)malonamide

Combine of N,N'-di-(4-chlorophenethyl)-2-((S)-2-(p-methoxybenzylmercapto)-3-phenylpropionylamino)malonamide (0.29 g, 0.43 mmol), mercury (II) acetate (0.171 g, 0.54 mmol), and anisole (0.47 mL) in dichloromethane (10 mL). Cool in an ice bath and degas by repeatedly applying vacuum and filling the vessel with nitrogen. Add trifluoroacetic acid (4 mL). After 3 hours, purge with hydrogen sulfide (gas) for about 10 minutes. Filter and evaporate *in vacuo* to give a residue. Triturate the residue with diethyl ether to give the title compound as a solid (80%).

### EXAMPLE 14

### N,N'-Di-(2-methoxyphenethyl)-2-((S)-2-mercapto-3-phenylpropionylamino)malonamide

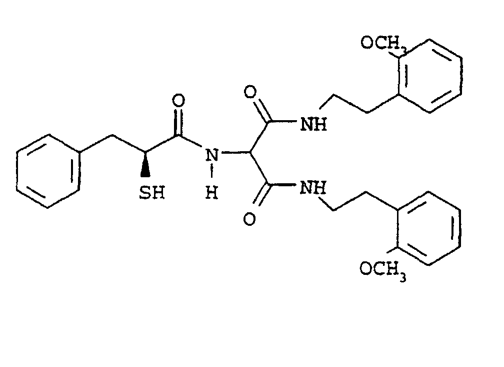

### 14.1 Synthesis of N,N'-di-(2-methoxyphenethyl)-2-((S)-2-(p-methoxybenzylmercapto)-3-phenylpropionylamino)malonamide

Prepare by the method of Example 13.1 using 2-methoxyphenylethylamine (0.31 mL, 2.23 mmol). Purify by chromatography on silica gel eluting sequentially with 2/3 ethyl acetate/hexane and then 1/2 ethyl acetate/hexane to give the title compound (50%).

### 14.2 Synthesis of N,N'-di-(2-methoxyphenethyl)-2-((S)-2-mercapto-3-phenylpropionylamino)malonamide

Prepare by the method of Example 13.2 using N,N'-di-(2-methoxyphenethyl)-2-((S)-2-(p-methoxybenzylmercapto)-3-phenylpropionylamino)malonamide (0.24 g, 0.37 mmol). Purify by chromatography on silica gel eluting with 2/3 ethyl acetate/hexane to give the title compound (91%).

### EXAMPLE 15

### N,N'-Di-(4-methylphenethyl)-2-((S)-2-mercapto-3-phenylpropionylamino)malonamide

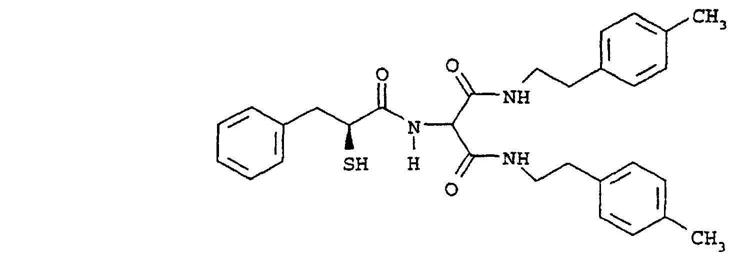

### 15.1 Synthesis of N,N'-di-(2-methylphenethyl)-2-((S)-2-(p-methoxybenzylmercapto)-3-phenylpropionylamino)malonamide

Combine 2-((S)-2-(p-methoxybenzylmercapto)-3-phenylpropionylamino)malonic acid (prepared by the method of Preparation 5, 0.476 g, 63% acid/37% sodium chloride, 0.74 mmol), and N-methylmorpholine (0.27 mL, 2.4 mmol), 2-methylphenylethylamine (0.25 mL, 1.7 mmol), dichloromethane (9 mL), and tetrahydrofuran (1 mL). Add, 1-ethyl-3-(3-(dimethylamino)propyl)carbodiimide hydrochloric acid salt (0.314 g, 1.64 mmol) and 1-hydroxybenztriazole hydrate (0.22 g, 1.64 mmol). After 1 day, concentrate *in vacuo* and partition the concentrated reaction mixture between an aqueous 5% sulfuric acid solution and ethyl acetate. Separate the layers and extract the organic layer with a saturated aqueous sodium bicarbonate solution and then brine. Dry the organic layer over Na₂SO₄, filter, and evaporate *in vacuo* to give a residue. Chromatograph the residue on silica gel eluting sequentially with 2/3 ethyl acetate/hexane and then 2/1 ethyl acetate/hexane to give the title compound.

### 15.2 Synthesis of N,N'-di-(2-methylphenethyl)-2-((S)-2-mercapto-3-phenylpropionylamino)malonamide

Combine of N,N'-di-(2-methylphenethyl)-2-((S)-2-(p-methoxybenzylmercapto)-3-phenylpropionylamino)malonamide (0.175 g, 0.274 mmol), mercury (II) acetate (0.108 g, 0.34 mmol), and anisole (0.3 mL) in dichloromethane (10 mL). Cool in an ice bath and degas by repeatedly applying vacuum and filling the vessel with nitrogen. Add trifluoroacetic acid (4 mL). After 3 hours, purge with hydrogen sulfide (gas) for about 10 minutes. Filter and evaporate *in vacuo* to give a residue. Combine the residue with carbon tetrachloride and evaporate *in vacuo* to remove most of the trifluoroacetic acid. Chromatograph the residue on silica gel eluting with 1/2 ethyl acetate/hexane and then 1/1 ethyl acetate/hexane to give the title compound.

### EXAMPLE 16

### N,N'-Di-(3-methoxyphenethyl)-2-(2-mercapto-3-phenylpropionylamino)malonamide

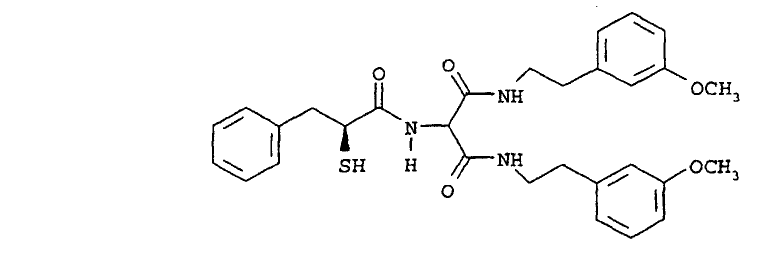

### 16.1 Synthesis of N,N'-di-(3-methoxyphenethyl)-2-((S)-2-(p-methoxybenzylmercapto)-3-phenylpropionylamino)malonamide

Prepare by the method of Example 15.1 using 3-methoxyphenylethylamine (0.24 mL, 1.7 mmol). Purify by chromatography on silica gel eluting sequentially with 2/3 ethyl acetate/hexane and then 2/1 ethyl acetate/hexane to give the title compound.

### 16.2 Synthesis of N,N'-di-(3-methoxyphenethyl)-2-((S)-2-mercapto-3-phenylpropionylamino)malonamide

Prepare by the method of Example 15.2 using N,N'-di-(3-methoxyphenethyl)-2-((S)-2-(p-methoxybenzylmercapto)-3-phenylpropionylamino)malonamide (0.181 g, 0.27 mmol). Purify by chromatography on silica gel eluting with 1/2 ethyl acetate/hexane and then 1/1 ethyl acetate/hexane to give the title compound.

### EXAMPLE 17

### N,N'-Di-(3,4-dimethoxyphenethyl)-2-((S)-2-mercapto-3-phenylpropionylamino)malonamide

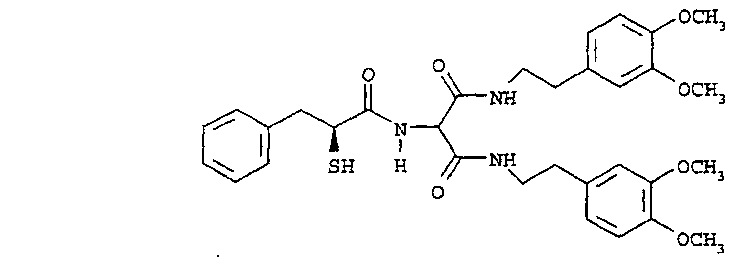

### 17.1 Synthesis of N,N'-di-(3,4-dimethoxyphenethyl)-2-((S)-2-(p-methoxybenzylmercapto)-3-phenylpropionylamino)malonamide

Combine 2-((S)-2-(p-methoxybenzylmercapto)-3-phenylpropionylamino)malonic acid (prepared by the method of Preparation 5. 0.476 g, 63% acid/37% sodium chloride, 0.74 mmol), and N-methylmorpholine (0.27 mL, 2.4 mmol), 3,4-dimethoxyphenylethylamine hydrochloride (0.50 g. 2.3 mmol), dichloromethane (9 mL), and tetrahydrofuran (1 mL). Cool in an ice bath. Add, 1-ethyl-3-(3-(dimethylamino)propyl)carbodiimide hydrochloric acid salt (0.314 g, 1.64 mmol) and l-hydroxybenztriazole hydrate (0.22 g, 1.64 mmol). After I day, concentrate *in vacuo* and partition the concentrated reaction mixture between an aqueous 5% sulfuric acid solution and ethyl acetate. Separate the layers and extract the organic layer with a saturated aqueous sodium bicarbonate solution and then brine. Dry the organic layer over Na₂SO₄, filter, and evaporate *in vacuo* to give a residue. Chromatograph the residue on silica gel eluting sequentially with 2/1 dichloromethane/ethyl acetate and then 1/1 dichloromethane/ethyl acetate to give the title compound.

### 17.2 Synthesis of N,N'-di-(3,4-dimethoxyphenethyl)-2-((S)-2-mercapto-3-phenylpropionylamino)malonamide

Combine of N,N'-di-(3,4-dimethoxyphenethyl)-2-((S)-2-(p-methoxybenzylmercapto)-3-phenylpropionylamino)malonamide (0.139 g, 0.19 mmol), mercury (II) acetate (0.076 g), and anisole (0.3 mL), and veratrole (0.24 mL) in dichloromethane (10 mL). Cool in an ice bath and degas by repeatedly applying vacuum and filling the vessel with nitrogen. Add trifluoroacetic acid (4 mL). After 3 hours, purge with hydrogen sulfide (gas) for about 10 minutes. Filter and evaporate *in vacuo* to give a residue. Chromatograph the residue on silica gel eluting with 3/1 dichloromethane/ethyl acetate/hexane and then 3/2 dichloromethane/ethyl acetate to give the title compound.

### EXAMPLE 18

### N.N'-Di-(3-chlorophenethyl)-2-(2-mercapto-3-phenylpropionylamino)malonamide

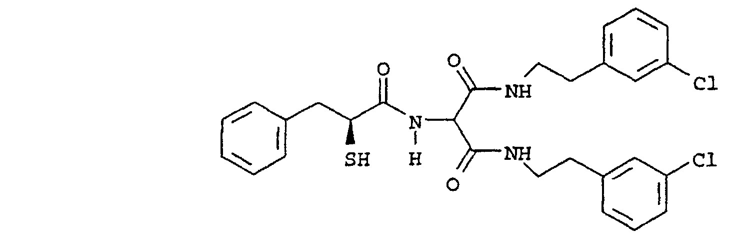

### 18.1 Synthesis of N,N'-di-(3-chlorophenethyl)-2-((S)-2-(p-methoxybenzylmercapto)-3-phenylpropionylamino)malonamide

Prepare by the method of Example 17.1 using 3-chlorophenylethylamine hydrochloride (0.50 g, 2.6 mmol). Purify by chromatography on silica gel eluting sequentially with 3/2 hexane/ethyl acetate and then 1/2 hexane/ethyl acetate to give the title compound.

### 18.2 Synthesis of N,N'-di-(3-chlorophenethyl)-2-(2-mercapto-3-phenylpropionylamino)malonamide

Prepare by the method of Example 15.2 using N,N'-di-(3-chlorophenethyl)-2-((S)-2-(p-methoxybenzylmercapto)-3-phenylpropionylamino)malonamide (0.126 g, 0.186 mmol). Purify by chromatography on silica gel eluting sequentially with 2/1 hexane/ethyl acetate and then 1/1 hexane/ethyl acetate to give the title compound.

### EXAMPLE 19

### N,N'-Di-(3,4-dichlorophenethyl)-2-(2-mercapto-3-phenylpropionylamino)malonamide

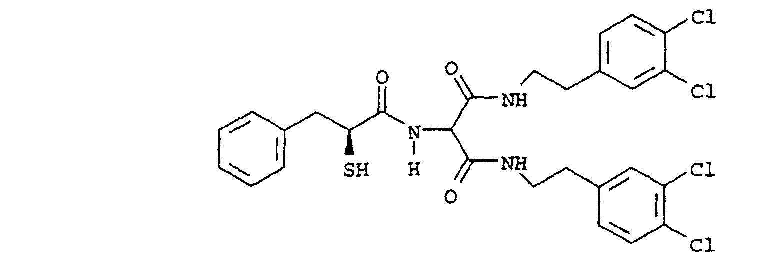

### 19.1 Synthesis of N,N'-di-(3,4-dichlorophenethyl)-2-((S)-2-(p-methoxybenzylmercapto)-3-phenylpropionylamino)malonamide

Prepare by the method of Example 17.1 using 3.4-dichlorophenylethylamine hydrochloride (0.50 g, 2.2 mmol). Purify by chromatography on silica gel eluting sequentially with 3/2 hexane/ethyl acetate and then 1/2 hexane/ethyl acetate to give the title compound.

### 19.2 Synthesis of N,N'-di-(3,4-dichlorophenethyl)-2-(2-mercapto-3-phenylpropionylamino)malonamide

Prepare by the method of Example 15.2 using N,N'-di-(3,4-dichlorophenethyl)-2-((S)-2-(p-methoxybenzylmercapto)-3-phenylpropionylamino)malonamide (0.166 g, 0.222 mmol). Coevaporation with carbon tetrachloride gives the title compound.

### PREPARATION 6

### Synthesis of (R)-2-bromo-6-phthalimidohexanoic acid

Combine (R)-2-N-carbobenzyloxy-6-aminohexanoic acid ((R)-Na-Cbz-lysine) (14.0 g, 50 mmol) and water (500 mL). Add sodium carbonate (5.65 g, 53 mmol) and N-carbethoxyphthalimide (13.15 g, 60 mmol). After 1.5 hours, acidify using aqueous 12M hydrochloric acid to give a solid. Collect the solid by filtration, rinse with water, and dry to give (R)-2-N-carbobenzyloxy-6-phthalamidohexanoic acid.
Combine (R)-2-N-carbobenzyloxy-6-phthalamidohexanoic acid obtained above, methanol (200 mL), 10% palladium-on-carbon (1 g) and treat with hydrogen at atmospheric pressure. After 18 hours, filter, add to the filtrate a solution of hydrochloric acid in methanol (50 mL, 1 M, 50 mmol), and evaporate *in vacuo* to give (R)-2-amino-6-phthalamidohexanoic acid hydrochloric acid salt.

### EXAMPLE 20

### N,N'-Diphenethyl-2-((S)-2-mercapto-6-phthalimidohexanoylamino)malonamide

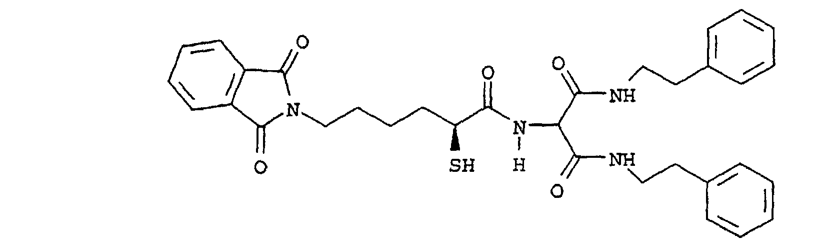

### 20.1 Synthesis of N,N'-diphenethyl-2-((S)-2-bromo-6-phthalimidohexanoylamino)malonamide

Prepare by the method of Example 3.3 using N,N'-diphenethyl-2-aminomalonamide trifluoroacetic acid salt (20 mmol) and (R)-2-bromo-6-phthalimidohexanoic acid (25 mmol) to give the title compound.

### 20.2 Synthesis of N.N'-diphenethyl-2-((S)-2-(p-methoxybenzylmercapto)-6-phthalimidohexanoylamino)malonamide

Combine N,N'-diphenethyl-2-((S)-2-bromo-6-phthalimidohexanoylamino)malonamide (10 mmol), 4-methyoxybenzylmercaptan (3.48 mL. 25 mmol), and tetrabutylammonium iodide (about 50 mg) in dimethylformamide (10 mL). Degas by repeatedly applying vacuum and filling the vessel with nitrogen. Add cesium carbonate (4.10 g, 12.5 mmol). After 15 hours, partition the reaction mixture between water and methyl t-butyl ether, saturate the aqueous layer with sodium chloride. Extract the aqueous layer with brine, dry over Na₂SO₄, filter, and evaporate *in vacuo* to give the title compound.

### 20.3 Synthesis of N,N'-diphenethyl-2-((S)-2-mercapto-6-phthalimidohexanoylamino)malonamide

Prepare by the method of Example 15.2 using N,N"-diphenethyl-2-((S)-2-(p-methoxybenzylmercapto)-6-phthalimidohexanoylamino)malonamide (5 mmol) to give the title compound.

The present invention provides a method of inhibiting matrix metalloproteinase (MMP) to a patient in need thereof comprising administering to the patient an effective matrix metalloproteinase inhibiting amount of a compound of formula (1).

As used herein, the term "patient" refers to warm-blooded animals or mammals, including guinea pigs, dogs, cats, rats, mice, hamsters, rabbits and primates, including humans. A patient is in need of treatment to inhibit MMP when it would be beneficial to the patient to reduce the physiological effect of active MMP. For example, a patient is in need of treatment to inhibit MMP when a patient is suffering from a disease state characterized by excessive tissue disruption or tissue degradation, such as, but not limited to, a neoplastic disease state or cancer; rheumatoid arthritis; osteoarthritis; osteoporosis; cardiovascular disorders, such as atherosclerosis; corneal ulceration; dental diseases, such as gingivitis or periodontal disease; and neurological disorders, such as multiple sclerosis; chronic inflammatory disorders, such as emphysema and especially smoking-induced emphysema.

The identification of those patients who are in need of treatment to inhibit MMP is well within the ability and knowledge of one skilled in the art. A clinician skilled in the art can readily identify, by the use of clinical tests, physical examination and medical/family history, those patients who are suffering from disease states characterized by excessive tissue disruption or tissue degradation.

An "effective matrix metalloproteinase inhibiting amount" of a compound of formula (1) is an amount which is effective, upon single or multiple dose administration to the patient, in providing relief of symptoms associated with MMP and is thus effective in inhibiting MMP-induced tissue disruption and/or MMP-induced tissue degradation. As used herein, "relief of symptoms" of MMP-mediated conditions refers to decrease in severity over that expected in the absence of treatment and does not necessarily indicate a total elimination or cure of the disease. Relief of symptoms is also intended to include prophylaxis.

An effective matrix metalloproteinase inhibiting dose can be readily determined by the use of conventional techniques and by observing results obtained under analogous circumstances. In determining the effective dose, a number of factors are considered including, but not limited to: the species of the patient; its size, age, and general health; the specific disease involved; the degree of involvement or the severity of the disease; the response of the individual patient; the particular compound administered; the mode of administration; the bioavailability characteristics of the preparation administered; the dose regimen selected; and the use of concomitant medication.

An effective matrix metalloproteinase inhibiting amount of a compound of formula (1) will generally vary from about 0.1 milligram per kilogram of body weight per day (mg/kg/day) to about 300 milligrams per kilogram of body weight per day (mg/kg/day). A daily dose of from about 1 mg/kg to about 100 mg/kg is preferred.

A neoplastic disease state refers to an abnormal state or condition characterized by rapidly proliferating cell growth or neoplasm. Neoplastic disease states for which treatment with a compound of formula (1) will be particularly useful include: Leukemias, such as, but not limited to, acute lymphoblastic, chronic lymphocytic. acute myeloblastic and chronic myelocytic; Carcinomas and adenocarcinomas, such as, but not limited to, those of the cervix, oesophagus, stomach, small intestines, colon, lungs (both small and large cell), breast and prostate; Sarcomas, such as, but not limited to, oesteroma, osteosarcoma, lipoma, liposarcoma, hemangioma and hemangiosarcoma; Melanomas, including amelanotic and melanotic; and mixed types of neoplasias such as, but not limited to carcinosarcoma, lymphoid tissue type, follicullar reticulum, cell sarcoma and Hodgkin's Disease. Neoplastic disease states for which treatment with a compound of formula (1) will be particularly preferred include carcinomas and adenocarcinomas, particularly of the breast, prostate and lung.

Atherosclerosis is a disease state characterized by the development and growth of atherosclerotic lesions or plaque. The identification of those patients who are in need of treatment for atherosclerosis is well within the ability and knowledge of one of ordinary skill in the art. For example, individuals who are either suffering from clinically significant atherosclerosis or who are at risk of developing clinically significant atherosclerosis are patients in need of treatment for atherosclerosis. A clinician of ordinary skill in the art can readily determine, by the use of clinical tests, physical examination and medical/family history, if an individual is a patient in need of treatment for atherosclerosis.

The term "chronic inflammatory disease" refers to diseases or conditions characterized by persistent inflammation in the absence of an identifiable irritant or microbial pathogen. Inflammatory diseases for which treatment with a compound of formula (1) will be particularly useful include: emphysema, chronic bronchitis, asthma, and chronic inflammation, and especially smoking-induced emphysema.

In effecting treatment of a patient, a compound of formula (1) can be administered in any form or mode which makes the compound bioavailable in effective amounts, including oral and parenteral routes. For example, the compound can be administered orally, subcutaneously, intramuscularly, intravenously, transdermally, topically, intranasally, rectally, inhalation, and other methods of administration intended to have the same effect. Oral and inhalation administration is generally preferred. One skilled in the art of preparing formulations can readily select the proper form and mode of administration depending upon the disease state to be treated, the stage of the disease, and other relevant circumstances. Remington's Pharmaceutical Sciences, 18th Edition, Mack Publishing Co. (1990).

A compound of formula (1) can be administered in the form of pharmaceutical compositions or medicaments which are made by combining a compound of formula (1) with pharmaceutically acceptable carriers or excipients, the proportion and nature of which are determined by the chosen route of administration, and standard pharmaceutical practice.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, gels, ointments, aerosol or other pharmaceutical composition forms intended to have the same use.

The pharmaceutical compositions may be administered orally, for example, with an inert diluent or with an edible carrier. They may be enclosed in gelatin capsules or compressed into tablets. For the purpose of oral therapeutic administration, a compound of formula (1) may be incorporated with excipients and used in the form of tablets, troches, capsules, elixirs, suspensions, syrups, wafers, chewing gums and other forms intended to have the same use. These preparations should contain at least 4% of a compound of formula (1), the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and other forms intended to have the same use may also contain one or more of the following adjuvants: binders such as microcrystalline cellulose, gum tragacanth or gelatin; excipients such as starch or lactose, disintegrating agents such as alginic acid, Primogel, corn starch and other disintegrating agents intended to have the same effect; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the compounds of the present invention may be incorporated into a solution or suspension. These preparations should contain at least 0.1% of a compound of the invention, but maybe varied to be between 0.1% and about 50% of the weight thereof. The amount of the active ingredient present in such compositions is such that a suitable dosage will be obtained. Preferred compositions and preparations are able to be determined by one skilled in the art.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

The compounds of the present invention may also be administered by inhalation, such as by aerosol or dry powder. Delivery may be by a liquefied or compressed gas or a suitable pump system which dispenses the compounds of the present invention or a formulation thereof. Formulations for administration by inhalation of compounds of formula (1) may be delivered in single phase, bi-phasic, or tri-phasic systems. A variety of systems are available for the administration by aerosol of the compounds of formula (1). Dry powder formulations are prepared by either pelletizing or milling the compound of formula (1) to a suitable particle size or by admixing the pelletized or milled compound of formula (1) with a suitable carrier material, such as lactose and other suitable carrier materials intended to have the same effect. Delivery by inhalation includes the necessary container, activators, valves, subcontainers, and other forms of delivery by inhalation intended to have the same effect. Preferred aerosol and dry powder formulations for administration by inhalation can be determined by one skilled in the art.

The MMP inhibitors of the present invention can be evaluated by the procedures that follow.

### EXAMPLE A

### Source and Activation of proMMP-1

ProMMP-1 (EC 3.4.24.7; interstitial collagenase) was purified from culture medium of human rheumatoid synovial fibroblasts stimulated with macrophage-conditioned medium according to Okada, Y. et al., J. Biol. Chem. 261, 14245-14255 (1986). The active MMP-1 was obtained by treatment of proMMP-1 with trypsin (5 µg/mL) at 37°C for 30 minutes, followed by addition of soybean trypsin inhibitor (50 µg/mL).

### Determination of Inhibition Constant (Kᵢ) for MMP-1

The activated MMP-1 is assayed using a fluorogenic substrate, Mca-Pro-Leu-Gly-Leu-Dpa-Ala-Arg-NH₂. Knight, C.G. et al., FEBS Lett. 296, 263-266(1992), at 37°C in 2.0 mL of assay buffer containing 50 mM Tris, pH 7.6, 0.2 M sodium chloride, 50 mM calcium chloride, and 0.02% Brij-35. The increase in fluorescence due to cleavage of Gly-Leu peptide bond by MMP-3 was monitored with Perkin-Elmer LS50B Fluorimeter (λₑₓ 328 nm, λₑₘ 393 nm, excitation slit 2.5, emission slit 10). Substrate and inhibitor stock solutions were made in DMF. For determination of Kᵢ values for MMP-1 inhibitors, a series of intermediate inhibitor solutions were prepared in DMF and 1 or 2 µL of the diluted inhibitor solution was mixed with 1 µL of 2 mM substrate solution in DMF in a quartz cuvette containing 2 mL of assay buffer. The enzyme (10 µL of 0.2 µM MMP-3 dilution in assay buffer) was added at the last to start the reaction. For routine measurement of a Kᵢ value for a reversible, competitive inhibitor, the initial rates in the presence of at least four inhibitor concentrations (two concentrations above Kᵢ and two concentrations below Kᵢ) were measured using [S] = 1 µM (<< Km) and [MMP-1] = 0.8 nM. Under these conditions, the measured K_{i, app} is close to true Kᵢ.

### Calculation of Kᵢ Values

The Kᵢ for a competitive inhibitor is calculated using: v₀/vᵢ = (1 + [I]/K_{i, app}) and Kᵢ = K_{i, app}/(1 + [S]/Kₘ), where v₀ is the initial rate in the absence of inhibitor, vᵢ is the initial rate in the presence of inhibitor at the concentration of [I], [S] is the substrate concentration, and Kₘ is the Michaelis constant. If slow binding is observed (i.e. if the approach to the binding equilibrium is slow), the final steady-state rate rather than the initial rate is taken as vᵢ.

### EXAMPLE B

### Source and Activation of proMMP-2

Recombinant MMP-2 was purified from the fermentation broth of yeast *Pichia pastoris* that carries the integrated MMP-2 gene into its chromosome. In brief, the full-length cDNA for MMP-2 was obtained by reverse transcription of RNA from human melanoma A375M cell line by the reverse transcriptase polymerase chain reaction (RT-PCR) using sequence specific oligonucleotides. The nucleotide sequence was confirmed by Taq cycle sequencing. The cDNA was ligated into the *Pichia pastoris* expression vector pHIL-D2 in such a way that the expression of pro-MMP-2 is under the control of the methanol inducible alcohol oxidase promoter. The expression construct was digested with either Sail or Nsil and used to transform the *Pichia pastoris* strains KM71 and SMD1168. A large-scale culture of a selected clone designated 24S was performed in a high cell density fermentor and the recombinant MMP-2 was purified from the culture supernatant by gelatin-sepharose 4B (Pharmacia). The enzyme is sufficiently pure at this stage for routine measurement of inhibition. If desired, however, the enzyme may be further purified by AcA 44 gel filtration (Spectra).

### Determination of Inhibition Constant (Kᵢ) for MMP-2

The active MMP-2 was obtained by activation of proMMP-2 at 37°C for 1 h with 4-aminophenylmercuric acetate which was then removed by a Sephadex G-50 spin column. The enzyme is assayed using a fluorogenic substrate, Mca-Pro-Leu-Gly-Leu-Dpa-Ala-Arg-NH₂, at 37°C in 2.0 mL of assay buffer containing 50 mM Tris, pH 7.6, 0.2 M sodium chloride, 50 mM calcium chloride, 0,02% Brij-35, and 50 µM β-mercaptoethanol. The increase in fluorescence is monitored (λₑₓ 328 nm, λₑₘ 393 nm). Substrate and inhibitor stock solutions are made in DMF. The enzyme is added at the last to start the reaction. For routine measurement of a Kᵢ value for a reversible, competitive inhibitor, the initial rates in the presence of at least four inhibitor concentrations (two inhibitor concentrations above Kᵢ and two below Kᵢ) are measured using [S] = 1 µM (<<Km) and [MMP-2] = 0.4 nM. Under these conditions, the measured Kᵢ, app is close to true Kᵢ.

### EXAMPLE C

### Source and Activation of proMMP-3

ProMMP-3 (EC 3.4.24.17; Stromelysin-1) was purified from culture medium of human rheumatoid synovial fibroblasts stimulated with macrophage-conditioned medium according to Okada, Y. et al., J. Biol. Chem. 261, 14245-14255 (1986). The active MMP-3 was obtained by treatment of proMMP-3 with trypsin (5 µg/mL) at 37°C for 30 minutes, followed by addition of soybean trypsin inhibitor (50 µg/mL). Aliquots of the activated MMP-3 were stored at -20°C.

### Determination of Inhibition Constant (Kᵢ) for MMP-3

The activated MMP-3 is assayed using a fluorogenic substrate. Mca-Pro-Leu-Gly-Leu-Dpa-Ala-Arg-NH₂, Knight, C.G. et al., FEBS Lett. 296, 263-266 (1992), at 37°C in an assay buffer containing 50 mM Tris, pH 7.6, 0.2 M sodium chloride, 50 mM calcium chloride, and 0.02% Brij-35. The increase in fluorescence due to cleavage of Gly-Leu peptide bond by MMP-3 was monitored with Perkin-Elmer LS50B Fluorimeter (λₑₓ 328 nm, λₑₘ 393 nm, excitation slit 2.5, emission slit 10). Substrate and inhibitor stock solutions were made in DMF and 0.1% HCl-DMF, respectively. For determination of K; values for MMP-3 inhibitors, a series of intermediate inhibitor solutions were prepared in 0.1% HCI-DMF and 1 or 2 µL of the diluted inhibitor solution was mixed with 1 µL of 2 mM substrate solution in DMF in a quartz cuvette containing 2 mL of assay buffer. The enzyme (10 µL of 0.2 µM MMP-3 dilution in assay buffer) was added at the last to start the reaction. For routine measurement of a Kᵢ value for a reversible, competitive inhibitor, the initial rates in the presence of at least four inhibitor concentrations (two concentrations above Kᵢ and two concentrations below Kᵢ) were measured using [S] = 1 µM (<< Kₘ) and [MMP-3] = 1 nM. Under these conditions, the measured K_{i, app} is close to true Kᵢ.

### Calculation of Kᵢ Values

The Kᵢ for a competitive inhibitor is calculated using: v₀/vᵢ = (1 + [I]/K_{i, app}) and Kᵢ = K_{i, app}/(1 + [S]/Kₘ), where v₀ is the initial rate in the absence of inhibitor, vᵢ is the initial rate in the presence of inhibitor at the concentration of [I], [S] is the substrate concentration, and Kₘ is the Michaelis constant. If slow binding is observed (i.e. if the approach to the binding equilibrium is slow), the final steady-state rate rather than the initial rate is taken as vᵢ.

### EXAMPLE D

### Source of MMP-12 (macrophage metalloelastase)

MMP-12 (EC 3.4.24.65) was cloned, expressed and purified according to Shapiro, S.D. et al., J Biol. Chem. 268, 23824-23829 (1993). Autoactivation resulted in the fully processed active form of the enzyme. Aliquots of MMP-12 were stored at -70C.

### Determination of the inhibition constant (Kᵢ) for MMP-12.

The potency of inhibitors of MMP-12 was measured using either quartz cuvettes or microtiter plates. The activity of MMP-12 was measured using a fluorogenic substrate, Mca-Pro-Leu-Gly-Leu-Dpa-Ala-Arg-NH2, Knight, C.G. et al., FEBS Lett. 296,263-266 (1992), at 25C in an assay buffer containing 50 mM Tris, pH 7.6, 0.2 M sodium chloride, 50 mM calcium chloride, and 0.02% Brij-35. The increase in fluorescence due to cleavage of Gly-Leu peptide bond by MMP-12 was monitored with a Perkin-Elmer LS50B Fluorimeter (λex 328 nm, λem 393 nm, excitation slit 2.5, emission slit 10) for the cuvette assay and with a Molecular Devices Fmax fluorescence plate reader (λex 320 nm, λλem 405 nm) for the microtiter plate assay. Substrate and inhibitor stock solutions were made in N,N,dimethylformamide (DMF) and 0.1% HCl-DMF, respectively.

Ki values were determined using the cuvette method by preparing a series of intermediate inhibitors solutions in 0.1% HCI-DMF and mixing the inhibitor with substrate (final concentration 2 µM) in a quartz cuvette containing 2 ml of assay buffer. MMP-12 was added to start the reaction at a concentration of 2 nM and progress curves were generated. For routine measurement of a Ki value for a reversible competitive inhibitor, the initial rates in the presence of at least four inhibitor concentrations ( two concentrations above and two concentrations below the Ki) were measured [S] = 2 µM (<<Km) and [MMP-12] = 2 nM. Under these conditions, the measured Ki,app is close to the true Ki.

Ki values were determined using the microtiter plate method in a manner similar to that described for the cuvette method with some modifications. Four different inhibitor concentrations (50 µl in assay buffer)of each compound were added to separate wells of a microtiter plate and substrate was added (100 µl) to get a final concentration of 4 mM. MMP-12 was added to a final concentration of 2 nM (50 µl) to start the reaction. Cleavage of substrate was recorded every 30 seconds for 30 minutes and progress curves were generated.

### Calculation of Ki values

The Ki for a competitive inhibitor was calculated using: Vo/Vi = (1+[I]/Ki,app) and Ki = Ki,app/(1+[S]/Km), where Vo is the initial rate in the absence of inhibitor, Vi is the initial rate in the presence of inhibitor at the concentration of [I], [S] is the substrate concentration, and Km is the Michaelis constant. If slow binding is observed (i.e. if the approach to the binding equilibium is slow), the final steady-state rate rather than the initial rate is taken as Vi.

## Claims

1. A compound of the formula 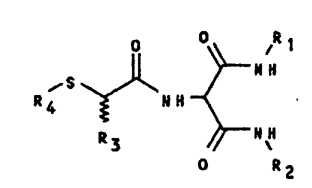 wherein
R₁ and R₂ are each independently selected from the group consisting of hydrogen, C₁- C₁₀ alkyl, -(CH₂)ₐ-Ar₁, and -(CH₂)_{b}-Ar₂;
wherein
a is an integer from 1 to 6;
b is an integer from 2 to 6;
Ar₁ is a radical selected from the group consisting of 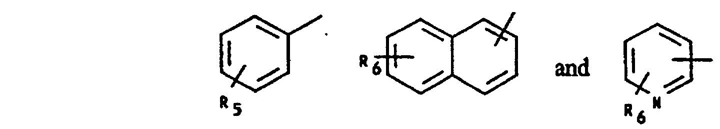 wherein
R₅ is 1 or 2 substituents independently selected from the group consisting of hydrogen, halogen, C₁₋C₄ alkyl, hydroxy, and C₁₋C₄ alkoxy;
R₆ is selected from the group consisting of hydrogen, halogen, C₁₋C₄ alkyl, and C₁₋C₄ alkoxy;
Ar₂ is the radical 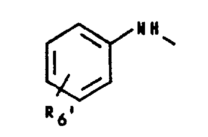 wherein
R_{6'} is selected from the group consisting of hydrogen, halogen, C₁-C₄ alkyl, and C₁-C₄ alkoxy;
R₃ is selected from the group consisting of C₁-C₆ alkyl, -(CH₂)ₘ-W, -(CH₂)ₚ-Ar₃, -(CH₂)ₖ-CO₂R₉, -(CH₂)ₘ-NR_{8'}SO₂-Y₁, and -(CH₂)ₘ-Z-Q
wherein
m is an integer from 2 to 8;
p is an integer from 0-10;
k is an integer from 1 to 9;
W is phthalimido;
Ar₃ is selected from the group consisting of 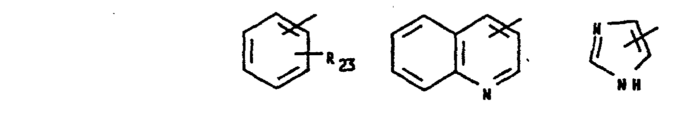 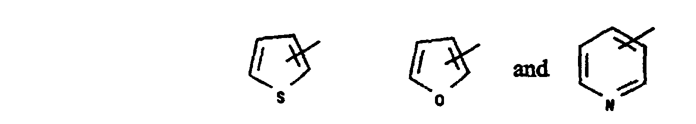 wherein
R₂₃ is from 1 to 2 substituents independently selected from the group consisting of hydrogen, halogen, C₁-C₄ alkyl, and C₁-C₄ alkoxy;
R₈' is hydrogen or C₁-C₆ alkyl;
R₉ is hydrogen or C₁-C₆ alkyl;
Y₁ is selected from the group consisting of hydrogen, -(CH₂)ⱼ-Ar₄, and -N(R₂₄)₂
wherein
j is 0 or 1;
R₂₄ each time selected is independently hydrogen or C₁-C₆ alkyl or are taken together with the nitrogen to which they are attached to form N-morpholino, N-piperidino, N-pyrrolidino, or N-isoindolyl;
Ar₄ is the radical 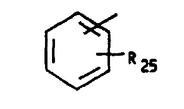 wherein
R₂₅ is from 1 to 3 substituents independently selected from the group consisting of hydrogen, halogen, C₁-C₄ alkyl, and C₁-C₄ alkoxy;
Z is selected from the group consisting of -O-, -NR₈-, -C(O)NR₈", -NR₈C(O)-, -NR₈C(O)NH-, -NR₈C(O)O-, and -OC(O)NH-;
wherein
R₈ is hydrogen or C₁-C₆ alkyl;
Q is selected from the group consisting of hydrogen, -(CH₂)ₙ-Y₂, and -(CH₂)ₓY₃;
wherein
n is an integer from 0 to 4;
Y₂ is selected from the group consisting of hydrogen, -(CH₂)ₕ-Ar₅ and -(CH₂)ₜ-C(O)OR₂₇
wherein
Ar₅ is selected from the group consisting of 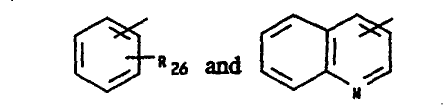 wherein
R₂₆ is from 1 to 3 substituents independently selected from the group consisting of hydrogen, halogen, C₁-C₄ alkyl, and C₁-C₄ alkoxy;
h is an integer from 0 to 6;
t is an integer from 1 to 6;
R₂₇ is hydrogen or C₁-C₆ alkyl;
x is an integer from 2 to 4;
Y₃ is selected from the group consisting of -N(R₂₈)₂, N-morpholino, N-piperidino, N-pyrrolidino, and N-isoindolyl;
wherein
R₂₈ each time taken is independently hydrogen or C₁-C₆ alkyl;
R₄ is selected from the group consisting of hydrogen, -C(O)R₁₀, -C(O)-(CH₂)_{q}-K and -S-G
wherein
R₁₀ is selected from the group consisting of hydrogen, C₁₋C₄ alkyl, phenyl, and benzyl;
q is 0, 1, or 2;
K is selected from the group consisting of 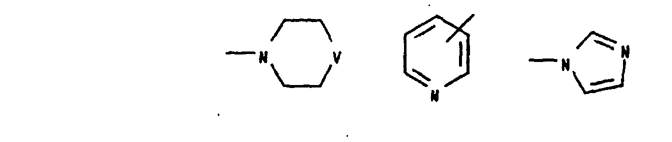 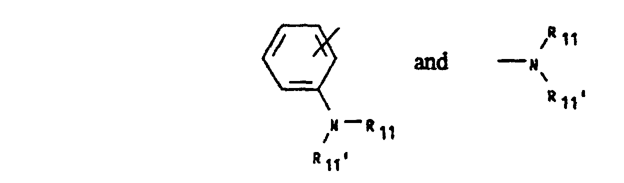 wherein
V is selected from the group consisting of a bond, -CH₂-, -O-, -S(O)ᵣ-, -NR-, and -NC(O)R'-;
wherein
r is 0, 1, or 2;
R is selected from the group consisting of hydrogen, C₁₋C₄ alkyl, and benzyl;
R' is selected from the group consisting of hydrogen, -CF₃, C₁-C₁₀ allyl, phenyl, and benzyl;
R₁₁ is selected from the group consisting of hydrogen, C₁₋C₄ alkyl, and benzyl;
R₁₁' is selected from the group consisting of hydrogen, C₁₋C₄ alkyl, and benzyl;
G is selected from the group consisting of 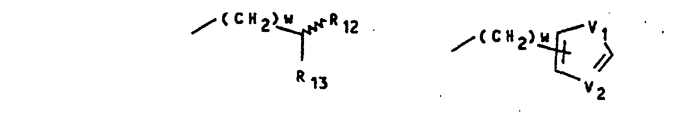 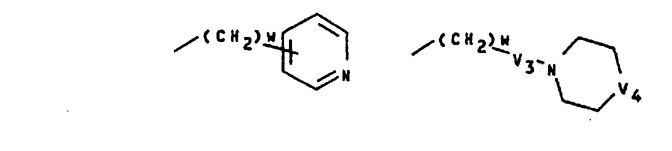 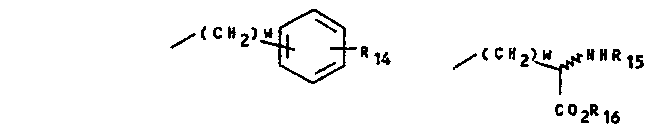 and 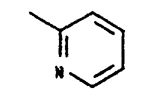 wherein
w is an integer from 1 to 3;
R₁₂ is selected from the group consisting of hydrogen, C₁-C₆ alkyl, -CH₂CH₂S(O)ₑCH₃, and benzyl;
wherein e is 0,1, or 2;
R₁₃ is selected from the group consisting of hydrogen, hydroxy, amino, C₁-C₆ alkyl, N-methylamino, N,N-dimethylamino, -CO₂R₁₇, and -OC(O)R₁₈;
wherein
R₁₇ is hydrogen, -CH₂O-C(O)C(CH₃)₃, C₁₋C₄ alkyl, benzyl, or diphenylmethyl;
R₁₈ is hydrogen, C₁-C₆ alkyl or phenyl;
R₁₄ is 1 or 2 substituents independently selected from the group consisting of hydrogen, C₁₋C₄ alkyl, C₁₋C₄ alkoxy, or halogen;
V₁ is selected from the group consisting of -O-, -S-, and -NH-;
V₂ is selected from the group consisting of -N- and -CH-;
V₃ is selected from the group consisting of a bond and -C(O)-;
V₄ is selected from the group consisting of -O-, -S-, -NR₁₉-, and -NC(O)R₂₀-;
wherein
R₁₉ is hydrogen, C₁₋C₄ alkyl, or benzyl;
R₂₀ is hydrogen, -CF₃, C₁-C₁₀ alkyl, or benzyl;
R₁₅ is selected from the group consisting of hydrogen, C₁-C₆ alkyl and benzyl;
R₁₆ is selected from the group consisting of hydrogen and C₁-C₄ alkyl;
and stereoisomers, pharmaceutically acceptable salt, and hydrate thereof.

2. A compound according to Claim 1 wherein R₁ and R₂ are a C₁-C₆ alkyl.

3. A compound according to Claim 1 wherein R₁ and R₂ -(CH₂)ₐ-Ar₁ wherein a and Art are as defined in Claim 1.

4. A compound according to Claim 3 wherein a is 1 or 2.

5. A compound according to Claim 3 wherein Ar₁ is phenyl or substituted phenyl.

6. A compound according to Claim 1 wherein R₄ is hydrogen.

7. A compound according to Claim 1 wherein R₄ is -S-G.

8. A compound according to Claim 1 wherein R₄ -C(O)R₁₀.

9. A comopund according to Claim 8 wherein R₁₀ is C₁₋C₄ alkyl.

10. A compound according to Claim 1 wherein the compound is N,N'-Diphenethyl-2-((S)-2-mercapto-3-methylbutyrylamino)malonamide.

11. A compound according to Claim 1 wherein the compound is N,N'-Diphenethyl-2-((S)-2-mercapto-3-phenylpropionylamino)malonamide.

12. A compound according to Claim 1 wherein the compound is N,N'-Diphenethyl-2-((S)-2-mercapto-4-phenylbutyrylamino)malonamide.

13. A compound according to Claim 1 wherein the compound is N,N'-Dibenzyl-2-((S)-2-mercapto-3-phenylpropionylamino)malonamide.

14. A compound according to Claim 1 wherein the compound is N,N'-Di-(3-phenylpropyl)-2-((S)-2-mercapto-3-phenylpropionylamino)malonamide.

15. A compound according to Claim 1 wherein the compound is N,N'-Di-(4-methoxyphenethyl)-2-((S)-2-mercapto-3-phenylpropionylamino)malonamide.

16. A compound according to Claim 1 wherein the compound is N,N'-Dipentyl-2-(2-mercapto-3-phenylpropionylamino)malonamide.

17. A compound according to Claim 1 wherein the compound is N,N'-Di-(2-(N-anilino)ethyl)-2-(2-mercapto-3-phenylpropionylmino)malonamide.

18. A compound according to Claim 1 wherein the compound is N,N'-Di-(pydrid-4-ylethyl)-2-(2-mercapto-3-phenylpropionylamino)malonamide.

19. A compound according to Claim 1 wherein the compound is N,N'-Diphenethyl-2-((S)-2-mercaptopropionylamino)malonamide.

20. A compound according to Claim 1 wherein the compound is N,N'-Diphenethyl-2-(2-mercaptopropionylamino)malonamide.

21. A compound according to Claim 1 wherein the compound is N,N'-Diphenethyl-2-(2-mercaptopentanoylamino)malonamide.

22. A compound according to Claim 1 wherein the compound is N,N'-Di-(4-chlorophenethyl)-2-(2-mercapto-3-phenylpropionylamino)malonamide.

23. A compound according to Claim 1 wherein the compound is N,N'-Di-(2-methoxyphenethyl)-2-((S)-2-mercapto-3-phenylpropionylamino)malonamide.

24. A compound according to Claim 1 wherein the compound is N,N'-Di-(4-methylphenethyl)-2-((S)-2-mercapto-3-phenylpropionylamino)malonamide.

25. A compound according to Claim 1 wherein the compound is N,N'-Di-(3-methoxyphenethyl)-2-(2-mercapto-3-phenylpropionylamino)malonamide.

26. A compound according to Claim 1 wherein the compound is N,N'-Di-(3,4-dimethoxyphenethyl)-2-((S)-2-mercapto-3-phenylpropionylamino)malonamide.

27. A compound according to Claim 1 wherein the compound is N,N'-Di-(3-chlorophenethyl)-2-(2-mercapto-3-phenylpropionylamino)malonamide.

28. A compound according to Claim 1 wherein the compound is N,N'-Di-(3,4-dichlorophenethyl)-2-(2-mercapto-3-phenylpropionylamino)malonamide.

29. A pharmaceutical composition comprising an effective matrix metalloproteinase inhibiting amount of a compound of Claim 1.

30. Use of an effective amount of matrix metalloproteinase inhibiting compound of claim 1 or a pharmaceutically acceptable salt thereof for the preparation of a pharmaceutical composition useful in the treatment of a patient in need thereof.

31. Use of an effective amount of matrix metalloproteinase inhibiting compound of claim 1 or a pharmaceutically acceptable salt thereof for the preparation of a pharmaceutical composition useful in the treatment of a neoplastic disease state.

32. Use of an effective amount of matrix metalloproteinase inhibiting compound of claim 1 or a pharmaceutically acceptable salt thereof for the preparation of a pharmaceutical composition useful in the treatment of rheumatoid arthritis.

33. Use of an effective amount of matrix metalloproteinase inhibiting compound of claim 1 or a pharmaceutically acceptable salt thereof for the preparation of a pharmaceutical composition useful in the treatment of osteoporosis.

34. Use of an effective amount of matrix metalloproteinase inhibiting compound of claim 1 or a pharmaceutically acceptable salt thereof for the preparation of a pharmaceutical composition useful in the treatment of chronic inflammatory disorders.

35. The use according to claim 34 wherein the chronic inflammatory disorder is emphysema.

36. A pharmaceutical composition according to claim 29 useful in the treatment of a patient in need thereof.

37. A pharmaceutical composition according to claim 29 useful in the treatment of a neoplastic disease state.

38. A pharmaceutical composition according to claim 29 useful in the treatment of rheumatoid arthritis.

39. A pharmaceutical composition according to claim 29 useful in the treatment of osteoarthritis.

40. A pharmaceutical composition according to claim 29 useful in the treatment of chronic inflammatory disorders.

41. A pharmaceutical composition according to claim 40 wherein the chronic inflammatory disorder is emphysema.

## Patentansprüche

1. Verbindung der Formel 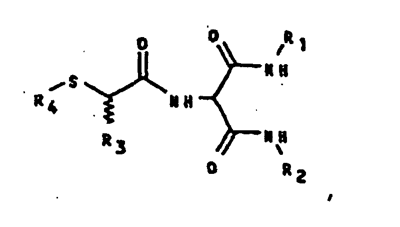 worin
R₁ und R₂ jeweils unabhängig voneinander von der Gruppe bestehend aus Wasserstoff, C₁-C₁₀-Alkyl, -(CH₂)ₐ-Ar₁ und -(CH₂)_{b}-Ar₂ ausgewählt sind;
worin
a eine ganze Zahl von 1 bis 6 darstellt;
b eine ganze Zahl von 2 bis 6 bedeutet;
Ar₁ ein Rest ist, welcher von der Gruppe bestehend aus 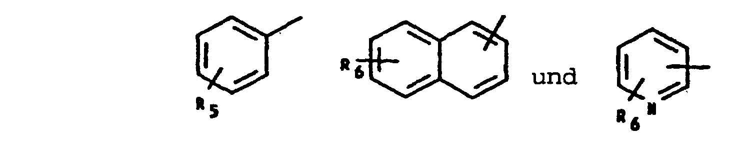 ausgewählt ist,
worin
R₅ ein oder zwei Substituenten darstellt, die unabhängig voneinander von der Gruppe bestehend aus Wasserstoff, Halogen, C₁-C₄-Alkyl, Hydroxy und C₁-C₄-Alkoxy ausgewählt sind;
R₆ von der Gruppe bestehend aus Wasserstoff, Halogen, C₁-C₄-Alkyl und C₁-C₄-Alkoxy ausgewählt ist;
Ar2 ein Rest 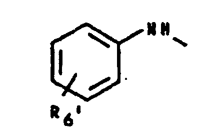 ist,
worin
R_{6'} von der Gruppe bestehend aus Wasserstoff,
Halogen, C₁-C₄-Alkyl und C₁-C₄-Alkoxy ausgewählt ist;
R₃ von der Gruppe bestehend aus C₁-C₆-Alkyl, -(CH₂)ₘ-W, - (CH₂)ₚ-Ar₃, -(CH₂)ₖ-CO₂R₉, -(CH₂)ₘ-NR₈,SO₂-Y₁ und -(CH₂)ₘ-Z-Q ausgewählt ist,
worin
m eine ganze Zahl von 2 bis 8 ist;
p eine ganze Zahl von 0 bis 10 ist;
k eine ganze Zahl von 1 bis 9 ist;
W für Phthalimido steht;
Ar₃ von der Gruppe bestehend aus 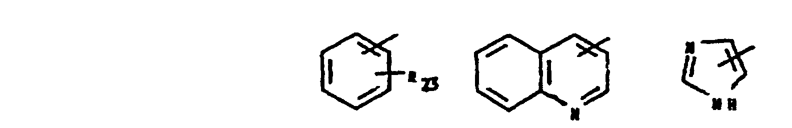 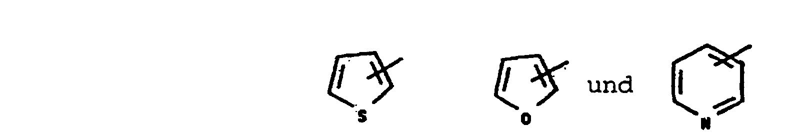 ausgewählt ist,
worin
R₂₃ 1 bis 2 Substituenten darstellt, die unabhängig voneinander von der Gruppe bestehend aus Wasserstoff, Halogen, C₁-C₄-Alkyl und C₁-C₄-Alkoxy ausgewählt sind;
R_{8'} Wasserstoff oder C₁-C₆-Alkyl darstellt;
R₉ Wasserstoff oder C₁-C₆-Alkyl bedeutet;
Y₁ von der Gruppe bestehend aus Wasserstoff, -(CH₂)ⱼ-Ar₄ und -N(R₂₄)₂ ausgewählt ist;
worin
j 0 oder 1 bedeutet;
R₂₄ jeweils unabhängig voneinander unter Wasserstoff oder C₁-C₆-Alkyl ausgewählt ist oder zusammengenommen mit dem Stickstoff, an welches sie gebunden sind, N-Morpholino, N-Piperidino, N-Pyrrolidino oder N-Isoindolyl ausbilden; Ar₄ der Rest 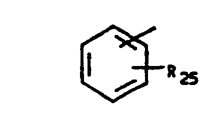 ist,
worin
R₂₅ 1 bis 3 Substituenten darstellt, welche unabhängig voneinander von der Gruppe bestehend aus Wasserstoff, Halogen, C₁-C₄-Alkyl und C₁-C₄-Alkoxy ausgewählt sind;
Z von der Gruppe bestehend aus -O-, -NR₈-, -C(O)NR₈-, -NR₈C(O)-, -NR₈C(O)NH-, -NR₈C(O)O- und -OC(O)NHausgewählt ist;
worin
R₈ Wasserstoff oder C₁-C₆-Alkyl darstellt;
Q von der Gruppe bestehend aus Wasserstoff, -(CH₂)ₙ-Y₂ und -(CH₂)ₓY₃ ausgewählt ist;
worin
n eine ganze Zahl von 0 bis 4 darstellt;
Y₂ von der Gruppe bestehend aus Wasserstoff, - (CH₂)ₕ -Ar₅ und -(CH₂)ₜ-C(O)OR₂₇ ausgewählt ist,
worin
Ar₅ von der Gruppe bestehend aus 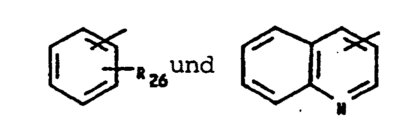 ausgewählt ist,
worin
R₂₆ 1 bis 3 Substituenten darstellt, welche unabhängig voneinander von der Gruppe bestehend aus Wasserstoff, Halogen, C₁-C₄-Alkyl und C₁-C₄-Alkoxy ausgewählt sind;
h eine ganze Zahl von 0 bis 6 ist;
t eine ganze Zahl von 1 bis 6 ist;
R₂₇ Wasserstoff oder C₁-C₆-Alkyl ist;
x eine ganze Zahl von 2 bis 4 bedeutet;
Y₃ von der Gruppe bestehend aus -N(R₂₈)₂, N-Morpholino, N-Piperidino, N-Pyrrolidino und N-Isoindolyl ausgewählt ist;
worin
R₂₈ jeweils unabhängig Wasserstoff oder C₁-C₆-Alkyl ist;
R₄ von der Gruppe bestehend aus Wasserstoff, -C(O)R₁₀, -C(O)-(CH₂)_{q}-K und S-G ausgewählt ist,
worin
R₁₀ von der Gruppe bestehend aus Wasserstoff, C₁-C₄-Alkyl, Phenyl und Benzyl ausgewählt ist;
q 0, 1 oder 2 ist;
K von der Gruppe bestehend aus 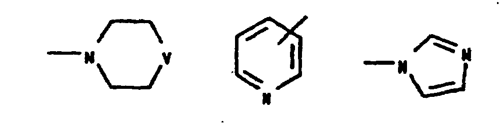 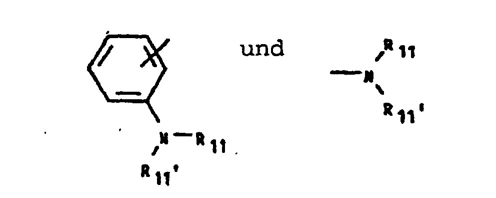 ausgewählt ist,
worin
V von der Gruppe bestehend aus einer Bindung, -CH₂-, -O-, -S(O)ᵣ-, -NR- und -NC(O)R'- ausgewählt ist;
worin
r 0, 1 oder 2 darstellt;
R von der Gruppe bestehend aus Wasserstoff, C₁-C₄-Alkyl und Benzyl ausgewählt ist;
R' von der Gruppe bestehend aus Wasserstoff, -CF₃, C₁-C₁₀-Alkyl, Phenyl und Benzyl ausgewählt ist;
R₁₁ von der Gruppe bestehend aus Wasserstoff, C₁-C₄-Alkyl und Benzyl ausgewählt ist;
R_{11'} von der Gruppe bestehend aus Wasserstoff, C₁-C₄-Alkyl und Benzyl ausgewählt ist;
G von der Gruppe bestehend aus 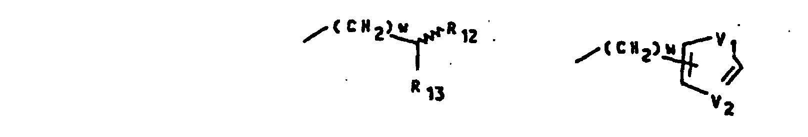 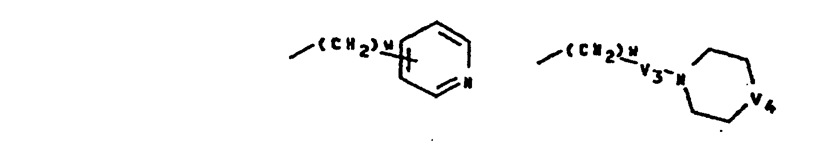 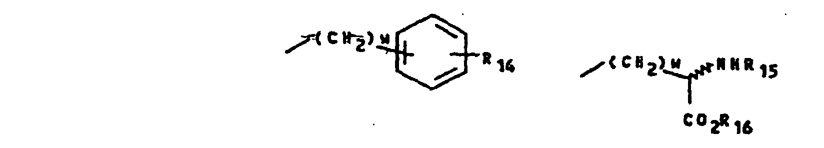 und 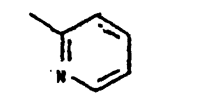 ausgewählt ist,
worin
w eine ganze Zahl von 1 bis 3 darstellt;
R₁₂ von der Gruppe bestehend aus Wasserstoff, C₁-C₆-Alkyl, -CH₂CH₂S(O)ₑCH₃ und Benzyl ausgewählt ist;
worin e 0, 1 oder 2 beträgt;
R₁₃ von der Gruppe bestehend aus Wasserstoff, Hydroxy, Amino, C₁-C₆-Alkyl, N-Methylamino, N,N-Dimethylamino, -CO₂R₁₇ und -OC(O)R₁₈ ausgewählt ist;
worin
R₁₇ Wasserstoff, -CH₂O-C(O)C(CH₃)₃, C₁-C₄-Alkyl, Benzyl oder Diphenylmethyl ist;
R₁₈ Wasserstoff, C₁-C₆-Alkyl oder Phenyl ist;
R₁₄ 1 oder 2 Substituenten darstellt, die unabhängig voneinander von der Gruppe bestehend aus Wasserstoff, C₁-C₄-Alkyl, C₁-C₄-Alkoxy oder Halogen ausgewählt sind;
V₁ von der Gruppe bestehend aus -O-, -S- und -NHausgewählt ist;
V₂ von der Gruppe bestehend aus -N- und -CH- ausgewählt ist;
V₃ von der Gruppe bestehend aus einer Bindung und -C(O)- ausgewählt ist;
V₄ von der Gruppe bestehend aus -O-, -S-, -NR₁₉- und -NC(O)R₂₀- ausgewählt ist;
worin
R₁₉ Wasserstoff, C₁-C₄-Alkyl oder Benzyl ist;
R₂₀ Wasserstoff, -CF₃, C₁-C₁₀-Alkyl oder Benzyl ist;
R₁₅ von der Gruppe bestehend aus Wasserstoff, C₁-C₆-Alkyl und Benzyl ausgewählt ist;
R₁₆ von der Gruppe bestehend aus Wasserstoff und C₁-C₄-Alkyl ausgewählt ist;
und Stereoisomere, pharmazeutisch annehmbare Salze und Hydrate hievon.

2. Verbindung nach Anspruch 1, worin R₁ und R₂ C₁-C₆-Alkyl sind.

3. Verbindung nach Anspruch 1, worin R₁ und R₂ -(CH₂)ₐ-Ar₁ sind, worin a und Ar₁ wie in Anspruch 1 definiert sind.

4. Verbindung nach Anspruch 3, worin a 1 oder 2 beträgt.

5. Verbindung nach Anspruch 3, worin Ar₁ Phenyl oder substituiertes Phenyl ist.

6. Verbindung nach Anspruch 1, worin R₄ Wasserstoff ist.

7. Verbindung nach Anspruch 1, worin R₄ -S-G bedeutet.

8. Verbindung nach Anspruch 1, worin R₄ -C(O)R₁₀ darstellt.

9. Verbindung nach Anspruch 8, worin R₁₀ C₁-C₄-Alkyl ist.

10. Verbindung nach Anspruch 1, wobei die Verbindung N,N'-Diphenethyl-2- ((S)-2-mercapto-3-methylbutyrylamino)malonamid ist.

11. Verbindung nach Anspruch 1, wobei die Verbindung N,N'-Diphenethyl-2-((S)-2-mercapto-3-phenylpropionylamino)-malonamid ist.

12. Verbindung nach Anspruch 1, wobei die Verbindung N,N'-Diphenethyl-2-((S)-2-mercapto-4-phenylbutyrylamino)malonamid ist.

13. Verbindung nach Anspruch 1, wobei die Verbindung N,N'-Dibenzyl-2-((S)-2-mercapto-3-phenylpropionylamino)malonamid ist.

14. Verbindung nach Anspruch 1, wobei die Verbindung N,N'-Di-(3-phenylpropyl)-2-((S)-2-mercapto-3-phenylpropionylamino) malonamid ist.

15. Verbindung nach Anspruch 1, wobei die Verbindung N,N'-Di-(4-methoxyphenethyl)-2-((S)-2-mercapto-3-phenylpropionylamino) malonamid ist.

16. Verbindung nach Anspruch 1, wobei die Verbindung N,N'-Dipentyl-2-(2-mercapto-3-phenylpropionylamino)malonamid ist.

17. Verbindung nach Anspruch 1, wobei die Verbindung N,N'-Di-(2-(N-anilino)ethyl)-2-(2-mercapto-3-phenylpropionylamino)malonamid ist.

18. Verbindung nach Anspruch 1, wobei die Verbindung N,N'-Di-(pydrid-4-ylethyl)-2-(2-mercapto-3-phenylpropionylamino)-malonamid ist.

19. Verbindung nach Anspruch 1, wobei die Verbindung N,N'-Diphenethyl-2-((S)-2-mercaptopropionylamino)malonamid ist.

20. Verbindung nach Anspruch 1, wobei die Verbindung N,N'-Diphenethyl-2-(2-mercaptopropionylamino)malonamid ist.

21. Verbindung nach Anspruch 1, wobei die Verbindung N,N'-Diphenethyl-2-(2-mercaptopentanoylamino)malonamid ist.

22. Verbindung nach Anspruch 1, wobei die Verbindung N,N'-Di-(4-chlorphenethyl)-2-(2-mercapto-3-phenylpropionylamino)-malonamid ist.

23. Verbindung nach Anspruch 1, wobei die Verbindung N,N'-Di-(2-methoxyphenethyl)-2-((S)-2-mercapto-3-phenylpropionylamino)malonamid ist.

24. Verbindung nach Anspruch 1, wobei die Verbindung N,N'-Di-(4-methylphenethyl)-2-((S)-2-mercapto-3-phenylpropionylamino)malonamid ist.

25. Verbindung nach Anspruch 1, wobei die Verbindung N,N'-Di-(3-methoxyphenethyl)-2-(2-mercapto-3-phenylpropionylamino)malonamid ist.

26. Verbindung nach Anspruch 1, wobei die Verbindung N,N'-Di-(3,4-dimethoxyphenethyl)-2-((S)-2-mercapto-3-phenylprionylamino)malonamid ist.

27. Verbindung nach Anspruch 1, wobei die Verbindung N,N'-Di-(3-chlorphenethyl)-2-(2-mercapto-3-phenylpropionylamino)-malonamid ist.

28. Verbindung nach Anspruch 1, wobei die Verbindung N,N'-Di-(3,4-dichlorphenethyl)-2-(2-mercapto-3-phenylpropionylamino)malonamid ist.

29. Pharmazeutische Zusammensetzung, umfassend eine wirksame Matrixmetalloproteinase-inhibierende Menge einer Verbindung nach Anspruch 1.

30. Verwendung einer wirksamen Menge einer Matrixmetalloproteinase-inhibierenden Verbindung nach Anspruch 1 oder eines pharmazeutisch annehmbaren Salzes hievon zur Herstellung einer pharmazeutischen Zusammensetzung, welche zur Behandlung eines Patienten, welcher einer solchen Bedarf, nützlich ist.

31. Verwendung einer wirksamen Menge einer Matrixmetalloproteinase-inhibierenden Verbindung nach Anspruch 1 oder eines pharmazeutisch annehmbaren Salzes hievon zur Herstellung einer pharmazeutischen Zusammensetzung, welche zur Behandlung eines neoplastischen Krankheitszustandes nützlich ist.

32. Verwendung einer wirksamen Menge einer Matrixmetalloproteinase-inhibierenden Verbindung nach Anspruch 1 oder eines pharmazeutisch annehmbaren Salzes hievon zur Herstellung einer pharmazeutischen Zusammensetzung, welche zur Behandlung von rheumatoider Arthritis nützlich ist.

33. Verwendung einer wirksamen Menge einer Matrixmetalloproteinase-inhibierenden Verbindung nach Anspruch 1 oder eines pharmazeutisch annehmbaren Salzes hievon zur Herstellung einer pharmazeutischen Zusammensetzung, welche zur Behandlung von Osteoporose nützlich ist.

34. Verwendung einer wirksamen Menge einer Matrixmetalloproteinase-inhibierenden Verbindung nach Anspruch 1 oder eines pharmazeutisch annehmbaren Salzes hievon zur Herstellung einer pharmazeutischen Zusammensetzung, welche zur Behandlung von chronischen Entzündungskrankheiten nützlich ist.

35. Verwendung nach Anspruch 24, wobei die chronische Entzündungskrankheit Emphysem ist.

36. Pharmazeutische Zusammensetzung nach Anspruch 29, welche zur Behandlung eines Patienten nützlich ist, welcher derselben bedarf.

37. Pharmazeutische Zusammensetzung nach Anspruch 29, welche zur Behandlung eines neoplastischen Krankheitszustandes nützlich ist.

38. Pharmazeutische Zusammensetzung nach Anspruch 29, welche zur Behandlung von rheumatoider Arthritis nützlich ist.

39. Pharmazeutische Zusammensetzung nach Anspruch 29, welche zur Behandlung von Osteoarthritis nützlich ist.

40. Pharmazeutische Zusammensetzung nach Anspruch 29, welche zur Behandlung von chronischen Entzündungskrankheiten nützlich ist.

41. Pharmazeutische Zusammensetzung nach Anspruch 40, wobei die chronische Entzündungskrankheit Emphysem ist.

## Revendications

1. Composé de formule 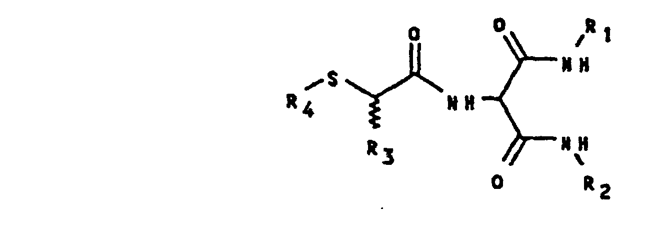 dans laquelle
R₁ et R₂ sont choisis chacun indépendamment dans le groupe constitué par l'hydrogène et les restes alkyle en C₁-C₁₀, -(CH₂)ₐ-Ar₁ et -(CH₂)_{b}Ar₂;
dans lesquels
a est un entier de 1 à 6;
b est un entier de 2 à 6;
Ar₁ est un radical choisi dans le groupe constitué par 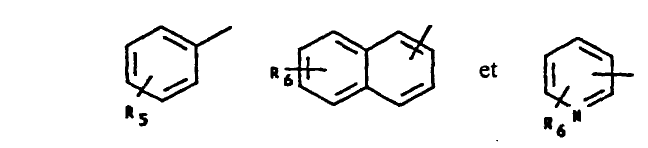 où
R₅ représente un ou deux substituants choisis indépendamment dans le groupe constitué par l'hydrogène et les restes halogéno, alkyle en C₁-C₄, hydroxy et alcoxy en C₁-C₄;
R₆ est choisi dans le groupe constitué par l'hydrogène et les restes halogéno, alkyle en C₁-C₄ et alcoxy en C₁-C₄;
Ar₂ est un radical 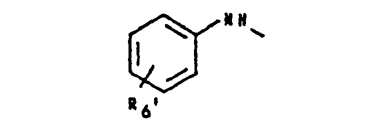 dans lequel R_{6'} est choisi dans le groupe constitué par l'hydrogène et les restes halogéno, alkyle en C₁-C₄ et alcoxy en C₁-C₄; R₃ est choisi dans le groupe constitué par les restes alkyle en C₁-C₆, -(CH₂)ₘ-W, -(CH₂)ₚ-Ar₃, -(CH₂)ₖ-CO₂R₉, -(CH₂)ₘ-NR_{8'}SO₂-Y₁ et -(CH₂)ₘ-Z-Q, dans lesquels
m est un entier de 2 à 8;
p est un entier de 0 à 10;
k est un entier de 1 à 9;
W est un reste phtalimido;
Ar₃ est choisi dans le groupe constitué par 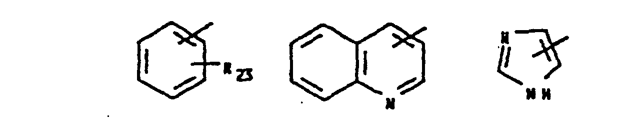 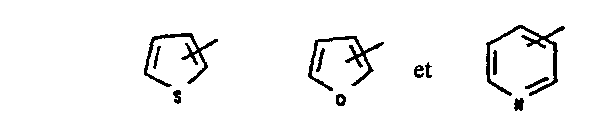 où
R₂₃ représente 1 à 2 substituants choisis indépendamment dans le groupe constitué par l'hydrogène et les restes halogéno, alkyle en C₁-C₄ et alcoxy en C₁-C₄;
R_{8'} est l'hydrogène ou un reste alkyle en C₁-C₆;
R₉ est l'hydrogène ou un reste alkyle en C₁-C₆;
Y₁ est choisi dans le groupe constitué par l'hydrogène et les restes -(CH₂)ⱼ-Ar₄ et -N(R₂₄)₂ dans lesquels
j est 0 ou 1;
R₂₄ est choisi à chaque fois indépendamment parmi l'hydrogène et les restes alkyle en C₁-C₆, ou les R₂₄ sont pris ensemble avec l'azote auquel ils sont liés pour former un reste N-morpholino, N-pipéridino, N-pyrrolidino ou N-isoindolyle;
Ar₄ est le radical 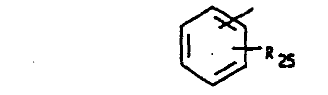 dans lequel R₂₅ représente 1 à 3 substituants choisis indépendamment dans le groupe constitué par l'hydrogène et les restes halogéno, alkyle en C₁-C₄ et alcoxy en C₁-C₄;
Z est choisi dans le groupe constitué par -O-, -NR₈-, -C(O)NR₈-, -NR₈C(O)-, -NR₈C(O)NH-, -NR₈C(O)O- et -OC(O)NH-; où
R₈ est l'hydrogène ou un reste alkyle en C₁-C₆;
Q est choisi dans le groupe constitué par l'hydrogène et les restes -(CH₂)ₙ-Y₂ et -(CH₂)ₓ-Y₃; dans lesquels
n est un entier de 0 à 4;
Y₂ est choisi dans le groupe constitué par l'hydrogène et les restes -(CH₂)ₕ-Ar₅ et -(CH₂)ₜ-C(O)OR₂₇, dans lesquels
Ar₅ est choisi dans le groupe constitué par 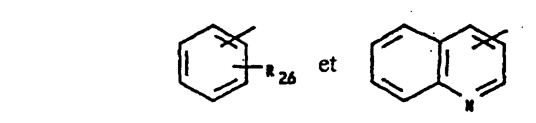 où
R₂₆ représente 1 à 3 substituants choisis dans le groupe constitué par l'hydrogène et les restes halogéno, alkyle en C₁-C₄ et alcoxy en C₁-C₄;
h est un entier de 0 à 6;
t est un entier de 1 à 6;
R₂₇ est l'hydrogène ou un reste alkyle en C₁-C₆;
x est un entier de 2 à 4;
Y₃ est choisi dans le groupe constitué par -N(R₂₈)₂, N-morpholino, N-pipéridino, N-pyrrolidino et N-isoindolyle, où
les R²⁸ sont chacun indépendamment un atome d'hydrogène ou un reste alkyle en C₁-C₆;
R₄ est choisi dans le groupe constitué par l'hydrogène et les restes -C(O)R₁₀, -C(O)-(CH₂)_{q}-K et -S-G, dans lesquels
R₁₀ est choisi dans le groupe constitué par l'hydrogène et les restes alkyle en C₁-C₄, phényle et benzyle;
q est 0, 1 ou 2;
K est choisi dans le groupe constitué par 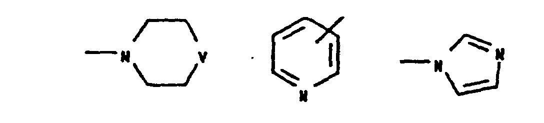 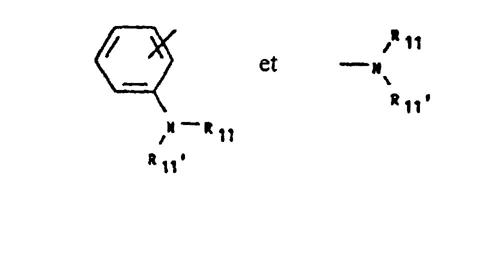 où
V est choisi dans le groupe constitué par une liaison, -CH₂-, -O-, -S(O)ᵣ-, -NR- et -NC(O)R'-; où
r est 0, 1 ou 2;
R est choisi dans le groupe constitué par l'hydrogène et les restes alkyle en C₁-C₄ et benzyle;
R' est choisi dans le groupe constitué par l'hydrogène et les restes -CF₃, alkyle en C₁-C₁₀, phényle et benzyle;
R₁₁ est choisi dans le groupe constitué par l'hydrogène et les restes alkyle en C₁-C₄ et benzyle;
R_{11'} est choisi dans le groupe constitué par l'hydrogène et les restes alkyle en C₁-C₄ et benzyle;
G est choisi dans le groupe constitué par 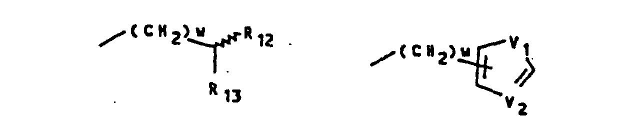 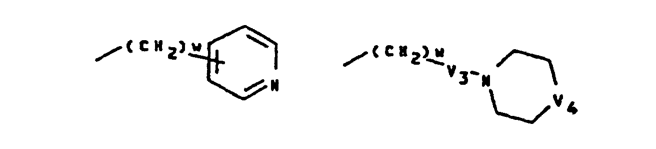 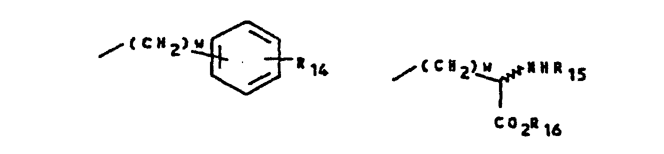 et 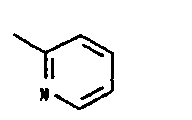 où
w est un entier de 1 à 3;
R₁₂ est choisi dans le groupe constitué par l'hydrogène et les restes alkyle en C₁-C₆, -CH₂CH₂S(O)ₑCH₃ et benzyle;
où e est 0, 1 ou 2;
R₁₃ est choisi dans le groupe constitué par l'hydrogène et les restes hydroxy, amino, alkyle en C₁-C₆, N-méthylamino, N,N-diméthylamino, -CO₂R₁₇ et -OC(O)R₁₈, dans lesquels
R₁₇ est l'hydrogène ou un reste -CH₂O-C(O)C(CH₃)₃, alkyle en C₁-C₄, benzyle ou diphénylméthyle;
R₁₈ est l'hydrogène ou un reste alkyle en C₁-C₆ ou phényle;
R₁₄ représente un ou deux substituants choisis indépendamment dans le groupe constitué par l'hydrogène et les restes alkyle en C₁-C₄, alcoxy en C₁-C₄ et halogéno;
V₁ est choisi dans le groupe constitué par -O-, -S- et -NH-;
V₂ est choisi dans le groupe constitué par -N- et -CH-;
V₃ est choisi dans le groupe constitué par une liaison et -C(O)-;
V₄ est choisi dans le groupe constitué par -O-, -S-, -NR₁₉- et -NC(O)R₂₀-;
où
R₁₉ est l'hydrogène ou un reste alkyle en C₁-C₄ ou benzyle;
R₂₀ est l'hydrogène ou un reste -CF₃, alkyle en C₁-C₁₀ ou benzyle;
R₁₅ est choisi dans le groupe constitué par l'hydrogène et les restes alkyle en C₁-C₆ et benzyle;
R₁₆ est choisi dans le groupe constitué par l'hydrogène et les restes alkyle en C₁-C₄;
et ses stéréoisomères, ses sels pharmaceutiquement acceptables et ses hydrates.

2. Composé selon la revendication 1, dans lequel R₁ et R₂ représentent un reste alkyle en C₁-C₆.

3. Composé selon la revendication 1, dans lequel R₁ et R₂ représentent un reste -(CH₂)ₐ-Ar₁ dans lequel a et Ar₁ ont la définition donnée dans la revendication 1.

4. Composé selon la revendication 3, dans lequel a est 1 ou 2.

5. Composé selon la revendication 3, dans lequel Ar₁ est un reste phényle ou phényle substitué.

6. Composé selon la revendication 1, dans lequel R₄ représente l'hydrogène.

7. Composé selon la revendication 1, dans lequel R₄ est -S-G.

8. Composé selon la revendication 1, dans lequel R₄ est -C(O)R₁₀.

9. Composé selon la revendication 8, dans lequel R₁₀ est un reste alkyle en C₁-C₄.

10. Composé selon la revendication 1, le composé étant le N,N'-diphénéthyl-2-((S)-2-mercapto-3-méthylbutyrylamino)malonamide.

11. Composé selon la revendication 1, le composé étant le N,N'-diphénéthyl-2-((S)-2-mercapto-3-phénylpropionylamino)malonamide.

12. Composé selon la revendication 1, le composé étant le N,N'-diphénéthyl-2-((S)-2-mercapto-4-phénylbutyrylamino)malonamide.

13. Composé selon la revendication 1, le composé étant le N,N'-dibenzyl-2-((S)-2-mercapto-3-phénylpropionylamino)malonamide.

14. Composé selon la revendication 1, le composé étant le N,N'-di(3-phénylpropyl)-2-((S)-2-mercapto-3-phénylpropionylamino)malonamide.

15. Composé selon la revendication 1, le composé étant le N,N'-di(4-méthoxyphénéthyl)-2-((S)-2-mercapto-3-phénylpropionylamino)malonamide.

16. Composé selon la revendication 1, le composé étant le N,N'-dipentyl-2-(2-mercapto-3-phénylpropionylamino)malonamide.

17. Composé selon la revendication 1, le composé étant le N,N'-di(2-(N-anilino)éthyl)-2-(2-mercapto-3-phénylpropionylamino)malonamide.

18. Composé selon la revendication 1, le composé étant le N,N'-di(pyrid-4-yléthyl)-2-(2-mercapto-3-phénylpropionylamino)malonamide.

19. Composé selon la revendication 1, le composé étant le N,N'-diphénéthyl-2-((S)-2-mercaptopropionylamino)malonamide.

20. Composé selon la revendication 1, le composé étant le N,N'-diphénéthyl-2-(2-mercaptopropionylamino)malonamide.

21. Composé selon la revendication 1, le composé étant le N,N'-diphénéthyl-2-(2-mercaptopentanoylamino)malonamide.

22. Composé selon la revendication 1, le composé étant le N,N'-di(4-chlorophénéthyl)-2-(2-mercapto-3-phénylpropionylamino)malonamide.

23. Composé selon la revendication 1, le composé étant le N,N'-di(2-méthoxyphénéthyl)-2-((S)-2-mercapto-3-phénylpropionylamino)malonamide.

24. Composé selon la revendication 1, le composé étant le N,N'-di(4-méthylphénéthyl)-2-((S)-2-mercapto-3-phénylpropionylamino)malonamide.

25. Composé selon la revendication 1, le composé étant le N,N'-di(3-méthoxyphénéthyl)-2-(2-mercapto-3-phénylpropionylamino)malonamide.

26. Composé selon la revendication 1, le composé étant le N,N'-di(3,4-diméthoxyphénéthyl)-2-((S)-2-mercapto-3-phénylpropionylamino)malonamide.

27. Composé selon la revendication 1, le composé étant le N,N'-di(3-chlorophénéthyl)-2-(2-mercapto-3-phénylpropionylamino)malonamide.

28. Composé selon la revendication 1, le composé étant le N,N'-di(3,4-dichlorophénéthyl)-2-(2-mercapto-3-phénylpropionylamino)malonamide.

29. Composition pharmaceutique comprenant une quantité efficace inhibitrice des métalloprotéinases matricielles d'un composé de la revendication 1.

30. Utilisation d'une quantité efficace du composé inhibiteur des métalloprotéinases matricielles de la revendication 1 ou d'un de ses sels pharmaceutiquement acceptables pour la préparation d'une composition pharmaceutique utile dans le traitement d'un patient qui en a besoin.

31. Utilisation d'une quantité efficace du composé inhibiteur des métalloprotéinases matricielles de la revendication 1 ou d'un de ses sels pharmaceutiquement acceptables pour la préparation d'une composition pharmaceutique utile dans le traitement d'une maladie néoplasique.

32. Utilisation d'une quantité efficace du composé inhibiteur des métalloprotéinases matricielles de la revendication 1 ou d'un de ses sels pharmaceutiquement acceptables pour la préparation d'une composition pharmaceutique utile dans le traitement de la polyarthrite rhumatoïde.

33. Utilisation d'une quantité efficace du composé inhibiteur des métalloprotéinases matricielles de la revendication 1 ou d'un de ses sels pharmaceutiquement acceptables pour la préparation d'une composition pharmaceutique utile dans le traitement de l'ostéoporose.

34. Utilisation d'une quantité efficace du composé inhibiteur des métalloprotéinases matricielles de la revendication 1 ou d'un de ses sels pharmaceutiquement acceptables pour la préparation d'une composition pharmaceutique utile dans le traitement de maladies inflammatoires chroniques.

35. Utilisation selon la revendication 34, dans laquelle la maladie inflammatoire chronique est l'emphysème.

36. Composition pharmaceutique selon la revendication 29, utile dans le traitement d'un patient qui en a besoin.

37. Composition pharmaceutique selon la revendication 29, utile dans le traitement d'une maladie néoplasique.

38. Composition pharmaceutique selon la revendication 29, utile dans le traitement de la polyarthrite rhumatoïde.

39. Composition pharmaceutique selon la revendication 29, utile dans le traitement de l'arthrose.

40. Composition pharmaceutique selon la revendication 29, utile dans le traitement de maladies inflammatoires chroniques.

41. Composition pharmaceutique selon la revendication 40, dans laquelle la maladie inflammatoire chronique est l'emphysème.
